(19) Europäisches Patentamt
European Patent Office
Office européen des brevets

(11) EP 3 406 265 B1

(12) **EUROPEAN PATENT SPECIFICATION**

(45) Date of publication and mention of the grant of the patent:
**29.07.2020 Bulletin 2020/31**

(51) Int Cl.:
***A61K 47/69*** *(2017.01)* ***A61K 47/59*** *(2017.01)*
***A61P 35/00*** *(2006.01)*

(21) Application number: **18382351.7**

(22) Date of filing: **22.05.2018**

(54) **COMPLEXES OF VIRAL-BASED THERAPEUTIC AGENTS AND MODIFIED POLY(BETA-AMINO ESTERS)**

KOMPLEXE VON VIRENBASIERTEN THERAPEUTISCHEN WIRKSTOFFEN UND MODIFIZIERTEN POLY(BETA-AMINOESTERN)

COMPLEXES D'AGENTS THÉRAPEUTIQUES À BASE VIRALE ET DE POLY(BÊTA-AMINO ESTERS) MODIFIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.05.2017 GB 201708203**

(43) Date of publication of application:
**28.11.2018 Bulletin 2018/48**

(73) Proprietors:
- **Sagetis Biotech, SL**
  **08017 Barcelona (ES)**
- **Institut Químic de Sarrià CETS Fundació Privada**
  **08017 Barcelona (ES)**
- **Institut d'Investigacions Biomèdiques August Pi i Sunyer**
  **08036 Barcelona (ES)**

(72) Inventors:
- **CASCANTE CIRERA, Anna**
  **08017 Barcelona (ES)**
- **BRUGRADA VILA, Pau**
  **08017 Barcelona (ES)**
- **BORRÓS GÓMEZ, Salvador**
  **08017 Barcelona (ES)**
- **FILLAT FONTS, Cristina**
  **08036 Barcelona (ES)**

(74) Representative: **Cabinet Beau de Loménie**
**158, rue de l'Université**
**75340 Paris Cedex 07 (FR)**

(56) References cited:
**WO-A1-2010/067041** **WO-A1-2014/093966**
**WO-A1-2014/136100** **WO-A2-2005/012407**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The invention relates to the use of modified poly(beta-amino ester)s (PBAEs) as vectors for the delivery of virus-based therapeutic agents in therapy. The invention also relates to complexes of modified poly(beta-amino ester)s and virus-based therapeutic agents, and to specific methods of treatment using these adducts.

BACKGROUND OF THE INVENTION

The lack of safe and efficient vectors to deliver virus-based therapeutic agents *in vivo* remains the principal handicap for the success of systemic gene therapy. The main hurdles are the high sero-prevalence of antibodies against viral vectors, and the natural liver tropism and liver-mediated clearance of viral vectors, which significantly reduces the available circulating dose of these agents after administration, for instance intravenous administration. It would be desirable to by-pass the immune system in a way which promotes a sero-prevalent population in patients. It would also be desirable to engineer viral tropism to enhance therapeutic utility (for instance, tumour targeting), to improve therapeutic efficiency and to reduce or eliminate undesirable side-effects.

WO-2014/136100-A describes modified poly(β-amino ester)s (PBAEs) as polynucleotide delivery vectors but makes no mention of the delivery of virus-based therapeutic agents.

Rojas et al., Journal of Controlled Release 237 (2016) 78-88 describes the use of albumin binding as a protection mechanism for the human adenovirus serotype 5 against neutralizing antibodies (NAbs).

DESCRIPTION OF THE INVENTION

The present invention addresses the aforementioned problems and provides the use of end-modified PBAEs in the delivery of virus-based therapeutic agents *in vivo.* The invention also provides complexes of the end-modified polymers with virus-based therapeutic agents, methods of preparing the complexes, drug delivery devices (e.g., microparticles, nanoparticles) including these polymers, and methods of using the complexes.

The end-modified PBAE polymers have biodegradable groups. The polyester nature of these systems provides an attractive biocompatible profile owing to their high biodegradability and reduced toxicity. These polymers have applications as viral delivery vectors in the treatment of many diseases such as cancer, monogenetic diseases, vascular disease and infectious diseases. Another application of these viral delivery vectors can be *in vitro* research as a tool to investigate gene function or regulation within a cellular and physiological context.

In a first aspect, the invention provides a complex of a virus-based therapeutic agent with a polymer of **Formula I**:

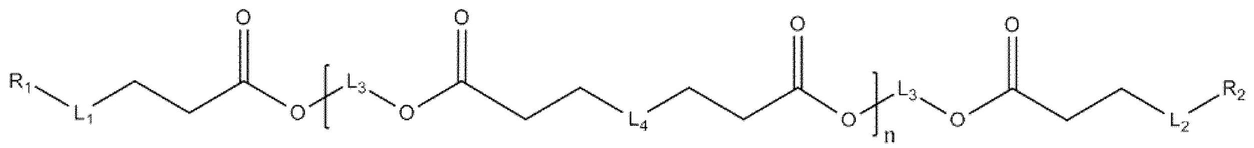

**Formula I**

wherein

$L_1$ and $L_2$ are independently selected from the group consisting of:

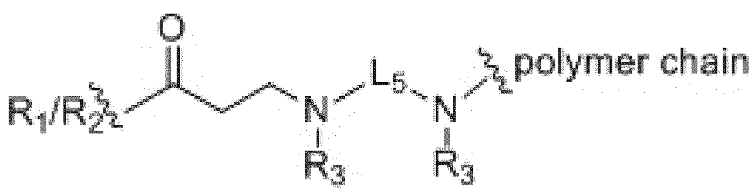

,

O, S, $NR_x$ and a bond; wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl; $L_3$ is independently selected from the group consisting of alkylene, alkenylene, heteroalkylene, heteroalkenylene, arylene or heteroarylene; or at least one occurrence of $L_3$ is

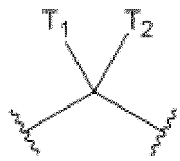,

wherein $T_1$ is

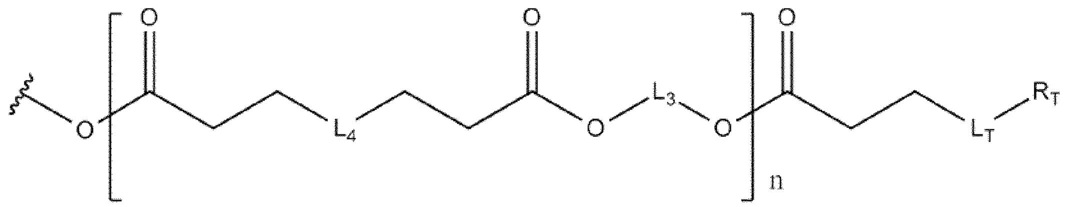

and $T_2$ is selected from H, alkyl or

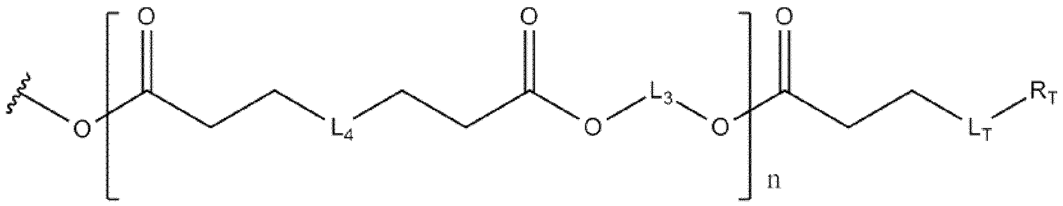;

wherein $L_T$ is independently selected from the group consisting of:

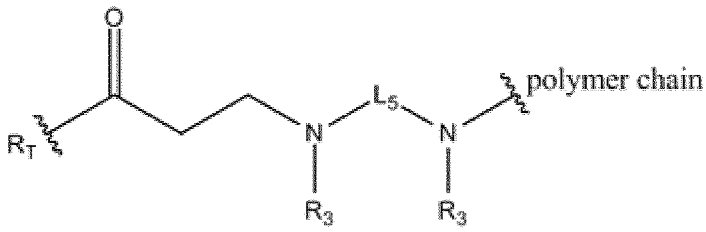,

O, S, $NR_x$ and a bond,
wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl, and the remaining $L_3$ groups are independently selected at each occurrence from the group consisting of alkylene, alkenylene, heteroalkylene, heteroalkenylene, arylene or heteroarylene;
$L_4$ is independently selected from the group consisting of

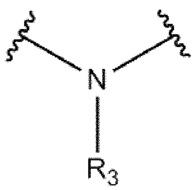 and 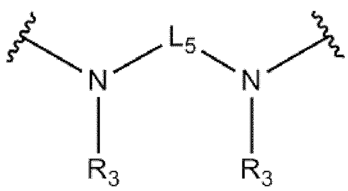;

$L_5$ is independently selected from the group consisting of alkylene, alkenylene, heteroalkylene, heteroalkenylene, arylene or heteroarylene;
$R_1$ and $R_2$ and $R_T$ (if present) are independently selected from an oligopeptide and $R_y$;
wherein at least one of $R_1$ and $R_2$ and $R_T$ (if present) is an oligopeptide;

and wherein $R_y$ is selected from the group consisting of hydrogen, halogen, alkyl, cycloalkyl, alkenyl, cycloalkenyl, heteroalkyl, heterocycloalkyl, acyl, aryl or heteroaryl;

each $R_3$ is independently selected from the group consisting of hydrogen, halogen, alkyl, cycloalkyl, alkenyl, cycloalkenyl, heteroalkyl, heterocycloalkyl, acyl, aryl, heteroaryl, and polyalkylene glycols, wherein said polyalkylene glycol is either bound directly to the nitrogen atom to which $R_3$ is attached or bound to the nitrogen atom to which $R_3$ is attached via a linker moiety, wherein said linker moiety is an alkylene, cycloalkylene, alkenylene, cycloalkenylene, heteroalkylene, heterocycloalkylene, arylene or heteroarylene group; and

n is an integer from 5 to 1,000;

or a pharmaceutically acceptable salt thereof.

According to the first aspect above, in some embodiments at least one $R_3$ group is a polyalkylene glycol, preferably a polyethylene glycol. In some embodiments the polyalkylene glycol (for example, polyethylene glycol) is bound directly to the nitrogen atom to which $R_3$ is attached. In some embodiments the polyalkylene glycol (for example, polyethylene glycol) is bound to the nitrogen atom to which $R_3$ is attached via a linker moiety. In preferred embodiments the linker moiety is an alkylene, alkenylene, or heteroalkylene group, more preferably the linker moiety is an alkylene group. In some embodiments the linker moiety is from 3 to 20 carbon and/or heteroatoms in length, preferably from 4 to 15 carbon and/or heteroatoms in length, more preferably from 5 to 10 carbon and/or heteroatoms in length.

In some preferred embodiments of the first aspect of the invention at least one $R_3$ group is a polyalkylene glycol and the polyalkylene glycol (for example, polyethylene glycol) bound directly to the nitrogen atom of an $L_4$ group. In some embodiments at least one $R_3$ group is a polyalkylene glycol and the polyalkylene glycol (for example, polyethylene glycol) bound to the nitrogen atom of an $L_4$ group via a linker moiety. In preferred embodiments the linker moiety is an alkylene, alkenylene or heteroalkylene group, more preferably the linker moiety is an alkylene group. In some embodiments the linker moiety is from 3 to 20 carbon and/or heteroatoms in length, preferably from 4 to 15 carbon and/or heteroatoms in length, more preferably from 5 to 10 carbon and/or heteroatoms in length.

In some embodiments of the first aspect, $L_3$ is independently selected from the group consisting of alkylene, alkenylene, heteroalkylene, heteroalkenylene, arylene or heteroarylene.

In some embodiments of the first aspect, at least one occurrence of $L_3$ is

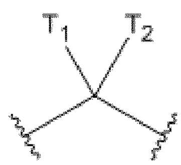

,

wherein $T_1$ is

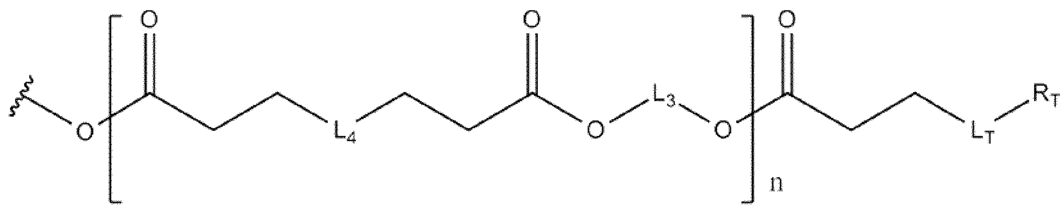

and $T_2$ is selected from H, alkyl or

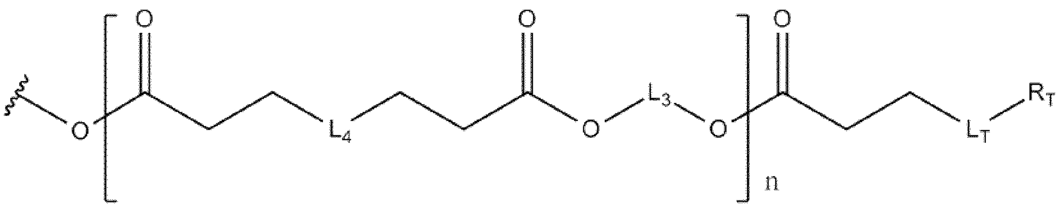

;

wherein $L_T$ is independently selected from the group consisting of:

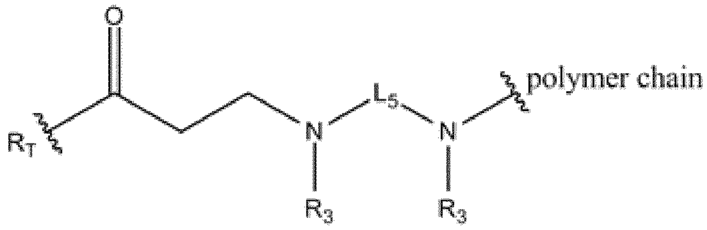

,

O, S, $NR_x$ and a bond; wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl, and the remaining $L_3$ groups are independently selected at each occurrence from the group consisting of alkylene, alkenylene, heteroalkylene, heteroalkenylene, arylene or heteroarylene.

In a second aspect, the invention provides a polymer of **Formula I**, wherein
$L_1$ and $L_2$ are independently selected from the group consisting of:

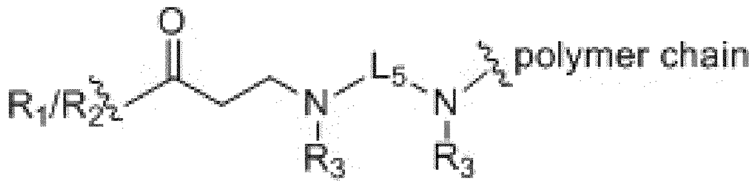

,

O, S, $NR_x$ and a bond; wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl;
$L_3$ is independently selected from the group consisting of alkylene, alkenylene, heteroalkylene, heteroalkenylene, arylene or heteroarylene; or at least one occurrence of $L_3$ is

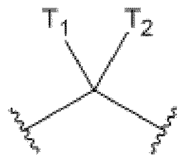

,

wherein $T_1$ is

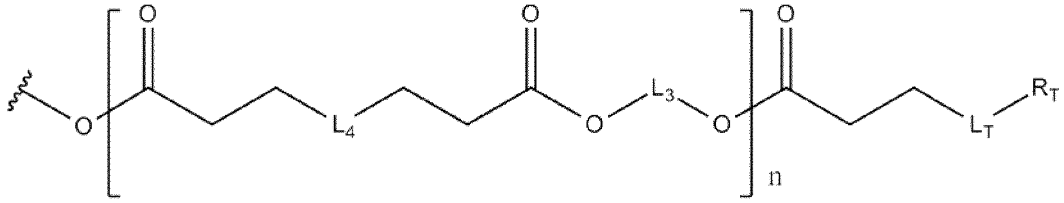

and
$T_2$ is selected from H, alkyl or

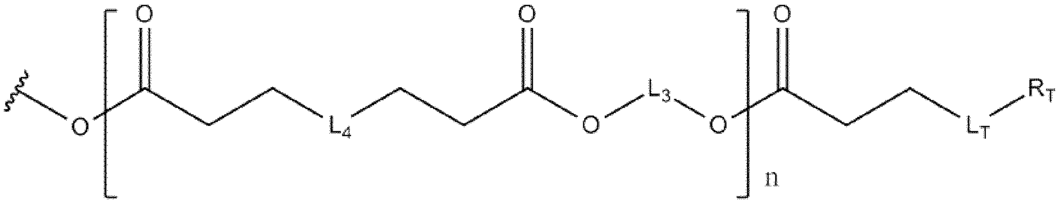

;

wherein $L_T$ is independently selected from the group consisting of:

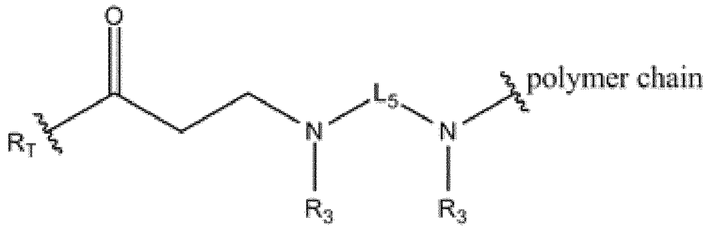

,

O, S, $NR_x$ and a bond, wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl, and the remaining $L_3$ groups are independently selected at each occurrence from the group consisting of alkylene, alkenylene, heteroalkylene, heteroalkenylene, arylene or heteroarylene;

$L_4$ is independently selected from the group consisting of

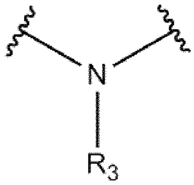

and

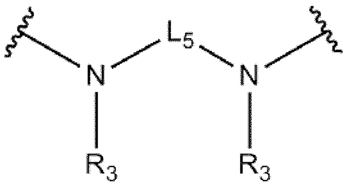

;

$L_5$ is independently selected from the group consisting of alkylene, alkenylene, heteroalkylene, heteroalkenylene, arylene or heteroarylene;

$R_1$ and $R_2$ and $R_T$ (if present) are independently selected from an oligopeptide and $R_y$;

wherein at least one of $R_1$ and $R_2$ and $R_T$ (if present) is an oligopeptide;

and wherein $R_y$ is selected from the group consisting of hydrogen, halogen, alkyl, cycloalkyl, alkenyl, cycloalkenyl, heteroalkyl, heterocycloalkyl, acyl, aryl or heteroaryl;

each $R_3$ is independently selected from the group consisting of hydrogen, halogen, alkyl, cycloalkyl, alkenyl, cycloalkenyl, heteroalkyl, heterocycloalkyl, acyl, aryl, heteroaryl, and polyalkylene glycols wherein said polyalkylene glycol is either bound directly to the nitrogen atom to which $R_3$ is attached or bound to the nitrogen atom to which $R_3$ is attached via a linker moiety, wherein said linker moiety is an alkylene, cycloalkylene, alkenylene, cycloalkenylene, heteroalkylene, heterocycloalkylene, arylene or heteroarylene group;

wherein at least one $R_3$ group is a polyalkylene glycol; and

n is an integer from 5 to 1,000;

or a pharmaceutically acceptable salt thereof.

According to the second aspect above wherein at least one $R_3$ group is a polyalkylene glycol, the polyalkylene glycol is preferably a polyethylene glycol. In some embodiments of the second aspect the polyalkylene glycol (for example, polyethylene glycol) is bound directly to the nitrogen atom to which $R_3$ is attached. In some embodiments the polyalkylene glycol (for example, polyethylene glycol) is bound to the nitrogen atom to which $R_3$ is attached via a linker moiety. In preferred embodiments the linker moiety is an alkylene, alkenylene, or heteroalkylene group, more preferably the linker moiety is an alkylene group. In some embodiments the linker moiety is from 3 to 20 carbon and/or heteroatoms in length, preferably from 4 to 15 carbon and/or heteroatoms in length, more preferably from 5 to 10 carbon and/or heteroatoms in length.

In some preferred embodiments of the second aspect of the invention the at least one $R_3$ group which is a polyalkylene glycol (for example, polyethylene glycol) is bound directly to the nitrogen atom of an $L_4$ group. In some embodiments the at least one $R_3$ group which is a polyalkylene glycol (for example, polyethylene glycol) is bound to the nitrogen atom of an $L_4$ group via a linker moiety. In preferred embodiments the linker moiety is an alkylene, alkenylene or heteroalkylene group, more preferably the linker moiety is an alkylene group. In some embodiments the linker moiety is from 3 to 20 carbon and/or heteroatoms in length, preferably from 4 to 15 carbon and/or heteroatoms in length, more preferably from 5 to 10 carbon and/or heteroatoms in length.

In some embodiments of the second aspect, $L_3$ is independently selected from the group consisting of alkylene, alkenylene, heteroalkylene, heteroalkenylene, arylene or heteroarylene.

In some embodiments of the second aspect, at least one occurrence of $L_3$ is

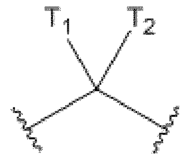

,

wherein $T_1$ is

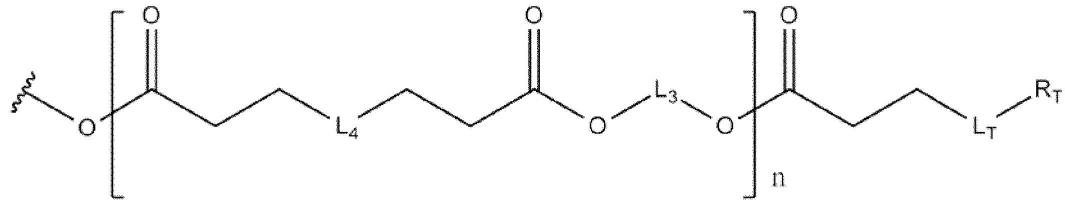

and $T_2$ is selected from H, alkyl or

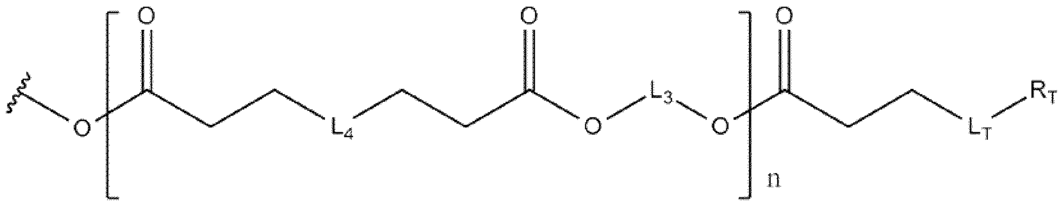

;

wherein $L_T$ is independently selected from the group consisting of:

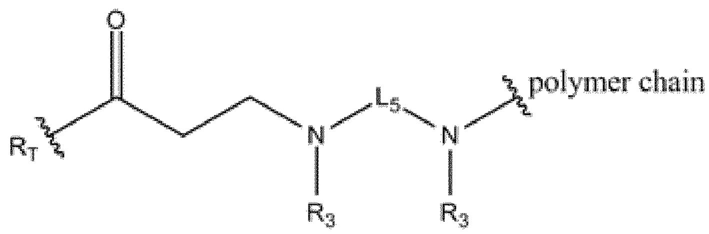

,

O, S, $NR_x$ and a bond; wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl, and the remaining $L_3$ groups are independently selected at each occurrence from the group consisting of alkylene, alkenylene, heteroalkylene, heteroalkenylene, arylene or heteroarylene.

Thus, the complexes of the present invention comprise PBAEs end-modified with at least one oligopeptide. In some embodiments of the invention, the complexes of the present invention comprise PBAEs substituted with at least one polyalkylene glycol group (preferably a polyethylene glycol group) either directly or through a linker, and end-modified with at least one oligopeptide.

The polymers of **Formula I** may be prepared by the reaction of diacrylate monomers of **Formula II** with substituted amines of formula $L_4H_2$ to form an acrylate terminated intermediate, **Formula III**.

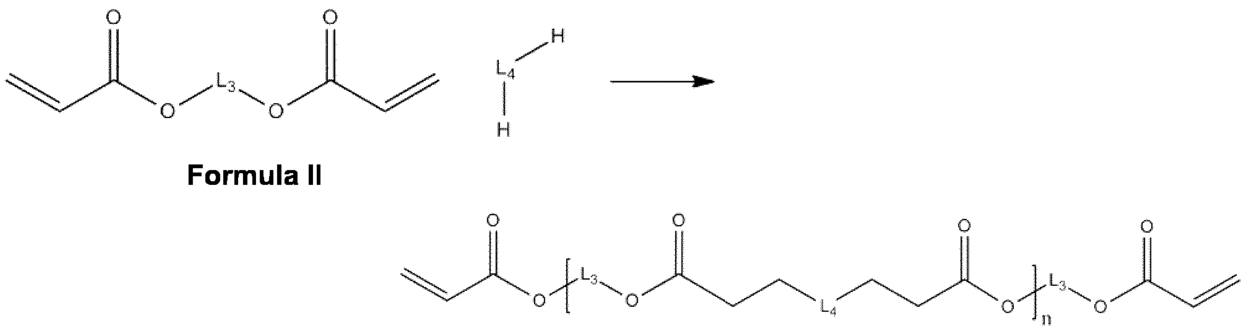

Formula II

Formula III

Groups $R_1L_1$ and $R_2L_2$ may then be added by reaction with a terminal acrylate group to form a polymer of **Formula I**.

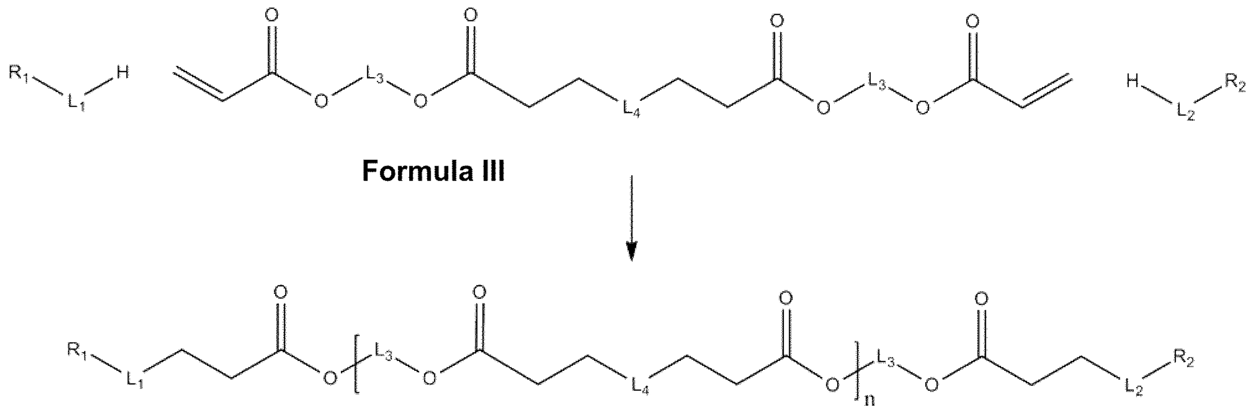

Formula III

Formula I

The polymers of **Formula I** wherein at least one $R_3$ group is a polyalkylene glycol moiety may be prepared in an analogous way by the reaction of diacrylate monomers of **Formula II** with substituted amines of formula $L_4H_2$ where the amines are substituted with a polyalkylene glycol moiety optionally bound to the nitrogen of the amine through a linker moiety as defined above.

Each $L_1$ and $L_2$ is selected to facilitate coupling of the end-modifying groups $R_1$ and $R_2$ to the PBAE polymer. Each $L_1$ and $L_2$ may be a bond, for example where the end-modifying group is an oligopeptide that comprises a terminal cysteine residue.

$L_T$ is selected to facilitate coupling of the end-modifying group $R_T$ to the PBAE polymer. $L_T$ may be a bond, for example where the end-modifying group is an oligopeptide that comprises a terminal cysteine residue.

$R_x$ may be independently selected from the group consisting of hydrogen, alkyl, cycloalkyl, alkenyl, cycloalkenyl, heteroalkyl and heterocycloalkyl, for example, from the group consisting of hydrogen, alkyl and cycloalkyl.

In compounds disclosed herein where a repeating unit is depicted (by square brackets), each group (e.g. $L_3$, $L_4$) within the square brackets is independently selected from the provided definitions for each single repeating unit. In other words, the repeating units within a particular polymer need not be identical.

### *Oligopeptides*

According to the present invention, an "oligopeptide" comprises a string of at least three amino acids linked together by peptide bonds. Such peptides preferably contain only natural amino acids, although non-natural amino acids (i.e., compounds that do not occur in nature but that can be incorporated into a polypeptide chain) and/or amino acid analogues as are known in the art may alternatively be employed. Also, one or more of the amino acids in such peptides may be modified, for example, by the addition of a chemical entity such as a carbohydrate group, a phosphate group, a farnesyl group, an isofarnesyl group, a fatty acid group, or a linker for conjugation, functionalization, or other modification, etc. The oligopeptides in the polymers defined herein typically comprise from 3 to 20 amino acid residues, more preferably

from 3 to 10 amino acid residues, more preferably from 3 to 6 amino acid residues. Alternatively, the oligopeptides in the polymers defined herein may comprise from 4 to 20 amino acid residues, more preferably from 4 to 10 amino acid residues, more preferably from 4 to 6 amino acid residues.

In the polymers of **Formula I**, the or each oligopeptide preferably has a net positive charge at pH7. The or each oligopeptide may comprise naturally occurring amino acids that are positively charged at pH7, that is, lysine, arginine and histidine. For example, the or each oligopeptide may be selected from the group consisting of polylysine, polyarginine or polyhistidine, each of which may be terminated with cysteine.

In a preferred embodiment, the or each oligopeptide is a compound of **Formula IV**:

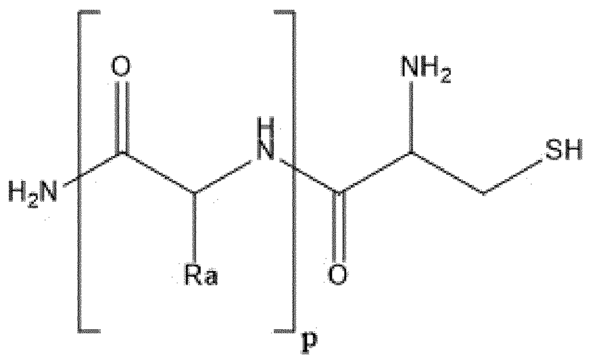

**Formula IV**

wherein p is an integer from 2 to 19, typically from 3 to 9 or from 3 to 5, and wherein $R_a$ is selected at each occurrence from the group consisting of $H_2NC(=NH)-NH(CH_2)_3-$, $H_2N(CH_2)_4-$ or (1*H*-imidazol-4-yl)-$CH_2-$.

Where the or each oligopeptide is a compound of **Formula IV**, the $L_1$ and/or $L_2$ (and/or $L_T$, when present) linking the or each oligopeptide to the polymer is a bond and the terminal cysteine residue provides a means of coupling the or each oligopeptide to the acrylate terminated intermediate, **Formula III**. The thiol functionality provides faster, more efficient and more easily controlled addition to the double bond. By contrast, where the or each oligopeptide is terminated in an amine functionality for coupling, an excess of this compound is required in the coupling step.

In the polymers of **Formula I**, the or each oligopeptide may have a net negative charge at pH7. The or each oligopeptide may comprise naturally occurring amino acids that are negatively charged at pH7, that is, aspartic acid and glutamic acid. For example, the or each oligopeptide may be selected from the group consisting of polyaspartic acid and polyglutamic acid, each of which may be terminated with cysteine. In this embodiment, the or each oligopeptide may be a compound of **Formula IV** wherein p is an integer from 2 to 19, typically from 3 to 9 or from 3 to 5, and wherein $R_a$ is $HO_2C(CH_2)_2-$ or $HO_2C-CH_2-$. In this case, the $L_1$ and/or $L_2$ linking the or each oligopeptide to the polymer is a bond as the terminal cysteine residue provides a means of coupling the or each oligopeptide to the acrylate terminated intermediate, **Formula IV**.

Alternatively, the or each oligopeptide may comprise a mixture of naturally occurring amino acids that are negatively charged at pH7 and naturally occurring amino acids that are positively charged at pH7.

In the polymers of **Formula I**, the or each oligopeptide may be hydrophobic. The or each oligopeptide may comprise naturally occurring amino acids that are hydrophobic such as valine, leucine, isoleucine, methionine, tryptophan, phenylalanine, cysteine, tyrosine and alanine; in particular, the or each oligopeptide may comprise valine, leucine, isoleucine, methionine, tryptophan and phenylalanine.

In the polymers of **Formula I**, the or each oligopeptide may be hydrophilic. The or each oligopeptide may comprise naturally occurring amino acids that are hydrophilic such as serine, threonine, cysteine, asparagine and glutamine, and may further comprise naturally occurring amino acids that are charged at pH7.

***Substituents***

In the polymers of **Formula I**, either both $R_1$ and $R_2$ are oligopeptides or one of $R_1$ and $R_2$ is an oligopeptide and one of $R_1$ and $R_2$ is $R_y$.

Where one of $R_1$ and $R_2$ is $R_y$, then $R_y$ is preferably selected from the group consisting of hydrogen, $-(CH_2)_mNH_2$, $-(CH_2)_mNHMe$, $-(CH_2)_mOH$, $-(CH_2)_mCH_3$, $-(CH_2)_2(OCH_2CH_2)_mNH_2$,$-(CH_2)_2(OCH_2CH_2)_mOH$ and $-(CH_2)_2(OCH_2CH_2)_mCH_3$ wherein m is an integer from 1 to 20, for example from 1 to 5. Preferably, $R_y$ is selected from the group consisting of $-(CH_2)_mNH_2$, $-(CH_2)_mNHMe$ and$-(CH_2)_2(OCH_2CH_2)_mNH_2$. Preferably, when $L_1$ is NH or $NR_x$, and one of $R_1$ and $R_2$ is $R_y$, then $R_y$ is different to $R_3$.

The polymers may be asymmetric. For example, in the polymers of the invention, one of $R_1$ and $R_2$ may be an oligopeptide and the other may be $R_y$. Alternatively, $R_1$ and $R_2$ may each be a different oligopeptide. In polymers where $R_T$ is present, at least one selected from $R_1$, $R_2$ and the one or two occurrences of $R_T$ may be an oligopeptide and the remaining groups selected from $R_1$, $R_2$ and the one or two occurrences of $R_T$ may be $R_y$. Alternatively, $R_1$, $R_2$ and the one or two occurrences of $R_T$ may each be a different oligopeptide.

For example, in the polymers of the invention one of $R_1$ and $R_2$ may be CysArgArgArg and the other may be derived from $H_2N(CH_2)_3CH(CH_3)CH_2NH_2$.

$L_3$ and $L_5$ may be independently selected from alkylene, alkenylene, heteroalkylene or heteroalkenylene and including polyethylene glycol linkers. Said alkylene, alkenylene, heteroalkylene or heteroalkenylene moieties may be of 1-20 carbon atoms, preferably of 1-12 carbon atoms, more preferably of 1-6 carbon atoms. Said polyethylene glycol linkers may be of 3 to 25 atoms in length, preferably of 3 to 18 atoms in length.

In a preferred embodiment, $L_3$ and $L_5$ are independently selected from alkylene moieties, preferably of 1-12 carbon atoms, more preferably of 1-6 carbon atoms, more preferably of 3-5 carbon atoms, and in a preferred embodiment of 4 carbon atoms.

In a particularly preferred embodiment, $L_3$ is selected from $-CH_2-$, $-(CH_2)_2-$,$-(CH_2)_3-$, $-(CH_2)_4-$, $-(CH_2)_5-$ and $-(CH_2)_6-$.

In a further embodiment, one or more carbon atoms in $L_3$ and/or $L_5$ (particularly as defined in the aforementioned preferred embodiments) may be replaced with -S-S-. In this embodiment, $L_3$ is preferably selected from $-(CH_2)_z-S-S-(CH_2)_z-$ wherein the value of each z is independently selected from 1 to 4 and preferably from 2 to 3 and preferably 2, preferably wherein the value of each z is the same. The inclusion of at least one disulfide bond in the main polymer chain can facilitate unpacking of the virus-based therapeutic agents inside the target cells.

Preferably, $L_4$ is independently selected from the group consisting of $-N(R_3)-$.

Preferably, each $R_3$ is independently selected from the group consisting of hydrogen, $-(CH_2)_pNH_2$,$-(CH_2)_pNHMe$, $-(CH_2)_pOH$, $-(CH_2)_pCH_3$, $-(CH_2)_2(OCH_2CH_2)_qNH_2$, $-(CH_2)_2(OCH_2CH_2)_qOH$,$-(CH_2)_2(OCH_2CH_2)_qCH_3$, and polyalkylene glycols, wherein p is an integer from 1 to 20 (preferably 1 to 5), and q is an integer from 1 to 10, for example from 1 to 5, and wherein said polyalkylene glycol is either bound directly to the nitrogen atom to which $R_3$ is attached or bound to the nitrogen atom to which $R_3$ is attached via a linker moiety, wherein said linker moiety is an alkylene, cycloalkylene, alkenylene, cycloalkenylene, heteroalkylene, heterocycloalkylene, arylene or heteroarylene group. In some embodiments of the invention at least one $R_3$ group is a polyalkylene glycol, preferably a polyethylene glycol. In some embodiments the linker moiety joining the at least one $R_3$ group which is a polyalkylene glycol to the nitrogen atom to which $R_3$ is bound is an alkylene, alkenylene or heteroalkylene group, preferably an alkylene group. In some embodiments the linker moiety is from 3 to 20 carbon and/or heteroatoms in length, preferably from 4 to 15 carbon and/or heteroatoms in length, more preferably from 5 to 10 carbon and/or heteroatoms in length

In **Formula I** or **III** above, n is preferably from 10 to 700, more preferably from 20 to 500. The molecular weight of the polymer of **Formula I** or **Formula III** is preferably from 500 to 150,000 g/mol, more preferably from 700 to 100,000 g/mol, more preferably from 2,000 to 50,000 g/mol, more preferably from 5,000 to 40,000 g/mol. In embodiments where at least one $R_3$ group is a polyalkylene glycol (e.g. polyethylene glycol) the molecular weight of the polymer of **Formula I or Formula III** is preferably from 2,500 to 150,000 g/mol, more preferably from 2,700 to 100,000 g/mol, more preferably from 4,000 to 50,000 g/mol, more preferably from 7,000 to 40,000 g/mol.

### *Compounds of the invention*

Certain compounds of the present invention may exist in particular geometric or stereoisomeric forms. The present invention contemplates all such compounds, including cis- and trans-isomers, *R*- and *S*-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

Isomeric mixtures containing any of a variety of isomer ratios may be utilized in accordance with the present invention. For example, where only two isomers are combined, mixtures containing 50:50, 60:40, 70:30, 80:20, 90: 10, 95:5, 96:4, 97:3, 98:2 or 99:1 isomer ratios are all contemplated by the present invention. Those of ordinary skill in the art will readily appreciate that analogous ratios are contemplated for more complex isomer mixtures.

### *Chemical Groups*

The term "halogen" (or "halo") includes fluorine, chlorine, bromine and iodine.

The term "alkyl" includes monovalent, straight or branched, saturated, acyclic hydrocarbyl groups. Alkyl is suitably $C_{1-10}$alkyl, or $C_{1-6}$alkyl, or $C_{1-4}$alkyl, such as methyl, ethyl, n-propyl, i-propyl or t-butyl groups. Alkyl may be substituted.

The term "cycloalkyl" includes monovalent, saturated, cyclic hydrocarbyl groups. Cycloalkyl is suitably $C_{3-10}$cycloalkyl, or $C_{3-6}$cycloalkyl such as cyclopentyl and cyclohexyl. Cycloalkyl may be substituted.

The term "alkoxy" means alkyl-O-.

The term "alkylamino" means alkyl-NH-.

The term "alkylthio" means alkyl-$S(O)_t$-, wherein t is defined below.

The term "alkenyl" includes monovalent, straight or branched, unsaturated, acyclic hydrocarbyl groups having at least one carbon-carbon double bond and, suitably, no carbon-carbon triple bonds. Alkenyl is suitably $C_{2-10}$alkenyl, or $C_{2-6}$alkenyl, or $C_{2-4}$alkenyl. Alkenyl may be substituted.

The term "cycloalkenyl" includes monovalent, partially unsaturated, cyclic hydrocarbyl groups having at least one carbon-carbon double bond and, suitably, no carbon-carbon triple bonds. Cycloalkenyl is suitably $C_{3-10}$cycloalkenyl, or $C_{5-10}$cycloalkenyl, *e.g.* cyclohexenyl or benzocyclohexyl. Cycloalkenyl may be substituted.

The term "alkynyl" includes monovalent, straight or branched, unsaturated, acyclic hydrocarbyl groups having at least one carbon-carbon triple bond and, suitably, no carbon-carbon double bonds. Alkynyl is suitably $C_{2-10}$alkynyl, or $C_{2-6}$alkynyl, or $C_{2-4}$alkynyl. Alkynyl may be substituted.

The term "alkylene" includes divalent, straight or branched, saturated, acyclic hydrocarbyl groups. Alkylene is suitably $C_{1-10}$alkylene, or $C_{1-6}$alkylene, or $C_{1-4}$alkylene, such as methylene, ethylene, n-propylene, i-propylene or t-butylene groups. Alkylene may be substituted.

The term "alkenylene" includes divalent, straight or branched, unsaturated, acyclic hydrocarbyl groups having at least one carbon-carbon double bond and, suitably, no carbon-carbon triple bonds. Alkenylene is suitably $C_{2-10}$alkenylene, or $C_{2-6}$alkenylene, or $C_{2-4}$alkenylene. Alkenylene may be substituted.

The term "heteroalkyl" includes alkyl groups, for example, $C_{1-65}$alkyl groups, $C_{1-17}$alkyl groups or $C_{1-10}$alkyl groups, in which up to twenty carbon atoms, or up to ten carbon atoms, or up to two carbon atoms, or one carbon atom, are each replaced independently by O, $S(O)_t$ or N, provided at least one of the alkyl carbon atoms remains. The heteroalkyl group may be C-linked or hetero-linked, i.e. it may be linked to the remainder of the molecule through a carbon atom or through O, $S(O)_t$ or N, wherein t is defined below. Heteroalkyl may be substituted.

The term "heterocycloalkyl" includes cycloalkyl groups in which up to ten carbon atoms, or up to two carbon atoms, or one carbon atom, are each replaced independently by O, $S(O)_t$ or N, provided at least one of the cycloalkyl carbon atoms remains. Examples of heterocycloalkyl groups include oxiranyl, thiaranyl, aziridinyl, oxetanyl, thiatanyl, azetidinyl, tetrahydrofuranyl, tetrahydrothiophenyl, pyrrolidinyl, tetrahydropyranyl, tetrahydrothiopyranyl, piperidinyl, 1,4-dioxanyl, 1,4-oxathianyl, morpholinyl, 1,4-dithianyl, piperazinyl, 1,4-azathianyl, oxepanyl, thiepanyl, azepanyl, 1,4-dioxepanyl, 1,4-oxathiepanyl, 1,4-oxaazepanyl, 1,4-dithiepanyl, 1,4-thieazepanyl and 1,4-diazepanyl. The heterocycloalkyl group may be C-linked or N-linked, i.e. it may be linked to the remainder of the molecule through a carbon atom or through a nitrogen atom. Heterocycloalkyl may be substituted.

The term "heteroalkenyl" includes alkenyl groups, for example, $C_{1-65}$alkenyl groups, $C_{1-17}$alkenyl groups or $C_{1-10}$alkenyl groups, in which up to twenty carbon atoms, or up to ten carbon atoms, or up to two carbon atoms, or one carbon atom, are each replaced independently by O, $S(O)_t$ or N, provided at least one of the alkenyl carbon atoms remains. The heteroalkenyl group may be C-linked or hetero-linked, *i.e.* it may be linked to the remainder of the molecule through a carbon atom or through O, $S(O)_t$ or N. Heteralkenyl may be substituted.

The term "heterocycloalkenyl" includes cycloalkenyl groups in which up to three carbon atoms, or up to two carbon atoms, or one carbon atom, are each replaced independently by O, $S(O)_t$ or N, provided at least one of the cycloalkenyl carbon atoms remains. Examples of heterocycloalkenyl groups include 3,4-dihydro-2H-pyranyl, 5-6-dihydro-2H-pyranyl, 2H-pyranyl, 1,2,3,4-tetrahydropyridinyl and 1,2,5,6-tetrahydropyridinyl. The heterocycloalkenyl group may be C-linked or N-linked, *i.e.* it may be linked to the remainder of the molecule through a carbon atom or through a nitrogen atom. Heterocycloalkenyl may be substituted.

The term "heteroalkynyl" includes alkynyl groups, for example, $C_{1-65}$alkynyl groups, $C_{1-17}$alkynyl groups or $C_{1-10}$alkynyl groups, in which up to twenty carbon atoms, or in which up to ten carbon atoms, or up to two carbon atoms, or one carbon atom, are each replaced independently by O, $S(O)_t$ or N, provided at least one of the alkynyl carbon atoms remains. The heteroalkynyl group may be C-linked or hetero-linked, *i.e.* it may be linked to the remainder of the molecule through a carbon atom or through O, $S(O)_t$ or N. Heteroalkynyl may be substituted.

The term "heteroalkylene" includes alkylene groups, for example, $C_{1-65}$alkylene groups, $C_{1-17}$alkylene groups or $C_{1-10}$alkylene groups, in which up to twenty carbon atoms, or in which up to ten carbon atoms, or up to two carbon atoms, or one carbon atom, are each replaced independently by O, $S(O)_t$ or N, provided at least one of the alkylene carbon atoms remains. Heteroalkynylene may be substituted.

The term "heteroalkenylene" includes alkenylene groups, for example, $C_{1-65}$alkenylene groups, $C_{1-17}$alkenylene groups or $C_{1-10}$alkenylene groups, in which up to twenty carbon atoms, or in which up to ten carbon atoms, or up to two carbon atoms, or one carbon atom, are each replaced independently by O, $S(O)_t$ or N, provided at least one of the alkenylene carbon atoms remains. Heteroalkenylene may be substituted.

The term "aryl" includes monovalent, aromatic, cyclic hydrocarbyl groups, such as phenyl or naphthyl (*e.g.* 1-naphthyl or 2-naphthyl). In general, the aryl groups may be monocyclic or polycyclic fused ring aromatic groups. Preferred aryl are $C_6$-$C_{14}$aryl. Aryl may be substituted.

Other examples of aryl groups are monovalent derivatives of aceanthrylene, acenaphthylene, acephenanthrylene, anthracene, azulene, chrysene, coronene, fluoranthene, fluorene, *as*-indacene, *s*-indacene, indene, naphthalene, ovalene, perylene, phenalene, phenanthrene, picene, pleiadene, pyrene, pyranthrene and rubicene.

The term "arylalkyl" means alkyl substituted with an aryl group, *e.g.* benzyl.

The term "heteroaryl" includes aryl groups in which one or more carbon atoms are each replaced by heteroatoms independently selected from O, S, N and $NR^N$, where $R^N$ is defined below (and in one embodiment is H or alkyl (*e.g.* $C_{1-6}$alkyl)). Heteroaryl may be substituted.

In general, the heteroaryl groups may be monocyclic or polycyclic (*e.g.* bicyclic) fused ring heteroaromatic groups. Typically, heteroaryl groups contain 5-14 ring members (preferably 5-10 members) wherein 1, 2, 3 or 4 ring members are independently selected from O, S, N and $NR^N$. A heteroaryl group is suitably a 5, 6, 9 or 10 membered, *e.g.* 5-membered monocyclic, 6-membered monocyclic, 9-membered fused-ring bicyclic or 10-membered fused-ring bicyclic.

Monocyclic heteroaromatic groups include heteroaromatic groups containing 5-6 ring members wherein 1, 2, 3 or 4 ring members are independently selected from O, S, N or $NR^N$.

5-Membered monocyclic heteroaryl groups may contain 1 ring member which is an -$NR^N$- group, an -O- atom or an -S- atom and, optionally, 1-3 ring members (*e.g.* 1 or 2 ring members) which are =N- atoms (where the remainder of the 5 ring members are carbon atoms).

Examples of 5-membered monocyclic heteroaryl groups are pyrrolyl, furanyl, thiophenyl, pyrazolyl, imidazolyl, isoxazolyl, oxazolyl, isothiazolyl, thiazolyl, 1,2,3 triazolyl, 1,2,4 triazolyl, 1,2,3 oxadiazolyl, 1,2,4 oxadiazolyl, 1,2,5 oxadiazolyl, 1,3,4 oxadiazolyl, 1,3,4 thiadiazolyl, pyridyl, pyrimidinyl, pyridazinyl, pyrazinyl, 1,3,5 triazinyl, 1,2,4 triazinyl, 1,2,3 triazinyl and tetrazolyl.

Examples of 6-membered monocyclic heteroaryl groups are pyridinyl, pyridazinyl, pyrimidinyl and pyrazinyl.

6-Membered monocyclic heteroaryl groups may contain 1 or 2 ring members which are =N- atoms (where the remainder of the 6 ring members are carbon atoms).

Bicyclic heteroaromatic groups include fused-ring heteroaromatic groups containing 9-14 ring members wherein 1, 2, 3, 4 or more ring members are independently selected from O, S, N or $NR^N$.

9-Membered bicyclic heteroaryl groups may contain 1 ring member which is an -$NR^N$- group, an-O- atom or an -S- atom and, optionally, 1-3 ring members (*e.g.* 1 or 2 ring members) which are =N- atoms (where the remainder of the 9 ring members are carbon atoms).

Examples of 9-membered fused-ring bicyclic heteroaryl groups are benzofuranyl, benzothiophenyl, indolyl, benzimidazolyl, indazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,2-a]pyridinyl, pyrrolo[1,2-b]pyridazinyl and imidazo[1,2-c]pyrimidinyl.

10-Membered bicyclic heteroaryl groups may contain 1-3 ring members which are =N- atoms (where the remainder of the 10 ring members are carbon atoms).

Examples of 10-membered fused-ring bicyclic heteroaryl groups are quinolinyl, isoquinolinyl, cinnolinyl, quinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "heteroarylalkyl" means alkyl substituted with a heteroaryl group.

Examples of acyl groups include alkyl-C(=O)-, cycloalkyl-C(=O)-, alkenyl-C(=O)-, cycloalkenyl-C(=O)-, heteroalkyl-C(=O)-, heterocycloalkyl-C(=O)-, aryl-C(=O)- or heteroaryl-C(=O)-, in particular, alkyl-C(=O)- and aryl-C(=O)-.

Unless indicated explicitly otherwise, where combinations of groups are referred to herein as one moiety, e.g. arylalkyl, the last mentioned group contains the atom by which the moiety is attached to the rest of the molecule.

Where reference is made to a carbon atom of an alkyl group or other group being replaced by O, $S(O)_t$ or N, what is intended is that:

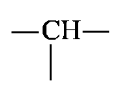

is replaced by

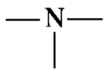

-CH= is replaced by -N=;

≡C-H is replaced by ≡N; or

$-CH_2-$ is replaced by -O-, $-S(O)_t-$ or $-NR^N-$.

By way of clarification, in relation to the above mentioned heteroatom containing groups (such as heteroalkyl *etc.*), where a numerical of carbon atoms is given, for instance $C_{3-6}$heteroalkyl, what is intended is a group based on $C_{3-6}$alkyl in which one of more of the 3-6 chain carbon atoms is replaced by O, $S(O)_t$ or N. Accordingly, a $C_{3-6}$heteroalkyl group, for example, will contain less than 3-6 chain carbon atoms.

Where mentioned above, $R^N$ is H, alkyl, cycloalkyl, aryl, heteroaryl, -C(O)-alkyl, -C(O)-aryl, -C(O)-heteroaryl, $-S(O)_t$-alkyl, $-S(O)_t$-aryl or $-S(O)_t$-heteroaryl. $R^N$ may, in particular, be H, alkyl (*e.g.* $C_{1-6}$alkyl) or cycloalkyl (*e.g.* $C_{3-6}$cycloalkyl).

Where mentioned above, t is independently 0, 1 or 2, for example 2. Typically, t is 0.

Where a group has at least 2 positions which may be substituted, the group may be substituted by both ends of an alkylene or heteroalkylene chain to form a cyclic moiety.

Optionally substituted groups (*e.g.* alkyl, cycloalkyl, alkoxy, alkenyl, cycloalkenyl, alkynyl, alkylene, alkenylene, heteroalkyl, heterocycloalkyl, heteroalkenyl, heterocycloalkenyl, heteroalkynyl, heteroalkylene, heteroalkenylene, aryl, arylalkyl, arylheteroalkyl, heteroaryl, heteroarylalkyl or heteroarylheteroalkyl groups *etc.*) may be substituted or unsubstituted, or may be unsubstituted. Typically, substitution involves the notional replacement of a hydrogen atom with a substituent group, or two hydrogen atoms in the case of substitution by =O.

Where substituted, there will generally be 1 to 3 substituents, or 1 or 2 substituents, or 1 substituent.

The optional substituent(s) is/are independently halogen, trihalomethyl, trihaloethyl,-OH, $-NH_2$, $-NO_2$, -CN, $-N^+(C_{1-6}alkyl)_2O^-$, $-CO_2H$, $-CO_2C_{1-6}$alkyl, $-SO_3H$, $-SOC_{1-6}$alkyl, $-SO_2C_{1-6}$alkyl, $-SO_3C_{1-6}$alkyl, $-OC(=O)OC_{1-6}$alkyl, -C(=O)H, $-C(=O)C_{1-6}$alkyl, $-OC(=O)C_{1-6}$alky), =O, $-NH(C_{1-6}alkyl)$, $-N(C_{1-6}alkyl)_2$, $-C(=O)NH_2$, $-C(=O)N(C_{1-6}alkyl)_2$, $-N(C_{1-6}alkyl)C(=O)O(C_{1-6}alkyl)$, $-N(C_{1-6}alkyl)C(=O)N(C_{1-6}alkyl)_2$, $-OC(=O)N(C_{1-6}alkyl)_2$, $-N(C_{1-6}alkyl)C(=O)C_{1-6}$alkyl, $-C(=S)N(C_{1-6}alkyl)_2$, $-N(C_{1-6}alkyl)C(=S)C_{1-6}$alkyl, $-SO_2N(C_{1-6}alkyl)_2$, $-N(C_{1-6}alkyl)SO_2C_{1-6}$alkyl, $-N(C_{1-6}alkyl)C(=S)N(C_{1-6}alkyl)_2$, $-N(C_{1-6}alkyl)SO_2N(C_{1-6}alkyl)_2$, $-C_{1-6}$alkyl, $-C_{1-6}$heteroalkyl, $-C_{3-6}$cycloalkyl, $-C_{3-6}$heterocycloalkyl, $-C_{2-6}$alkenyl, $-C_{2-6}$heteroalk enyl, $-C_{3-6}$cycloalkenyl, $-C_{3-6}$heterocycloalkenyl, $-C_{2-6}$alkynyl, $-C_{2-6}$heteroalkynyl, $-Z^u-C_{1-6}$alkyl,$-Z^u-$ $C_{3-6}$cycloalkyl, $-Z^u-C_{2-6}$alkenyl, $-Z^u-C_{3-6}$cycloalkenyl or $-Z^u-C_{2-6}$alkynyl, wherein $Z^u$ is independently O, S, NH or $N(C_{1-6}alkyl)$.

In another embodiment, the optional substituent(s) is/are independently halogen, trihalomethyl, trihaloethyl, $-NO_2$, -CN, $-N^+(C_{1-6}alkyl)_2O^-$, $-CO_2H$, $-SO_3H$, $-SOC_{1-6}$alkyl, $-SO_2C_{1-6}$alkyl, -C(=O)H, $-C(=O)C_{1-6}$alkyl, =O, $-N(C_{1-6}alkyl)_2$, $-C(=O)NH_2$, $-C_{1-6}$alkyl, $-C_{3-6}$cycloalkyl, $-C_{3-6}$heterocycloalkyl, $-Z^uC_{1-6}$alkyl or-$z^u$-$C_{3-6}$cycloalkyl, wherein $Z^u$ is defined above.

In another embodiment, the optional substituent(s) is/are independently halogen, trihalomethyl, $-NO_2$, -CN, $-CO_2H$, $-C(=O)C_{1-6}$alkyl, =O, $-N(C_{1-6}alkyl)_2$, $-C(=O)NH_2$, $-C_{1-6}$alkyl, $-C_{3-6}$cycloalkyl, $-C_{3-6}$heterocycloalkyl, $-Z^uC_{1-6}$alkyl or-$Z^u$-$C_{3-6}$cycloalkyl, wherein $Z^u$ is defined above.

In another embodiment, the optional substituent(s) is/are independently halogen, $-NO_2$, -CN, $-CO_2H$, =O, $-N(C_{1-6}alkyl)_2$, $-C_{1-6}$alkyl, $-C_{3-6}$cycloalkyl or $-C_{3-6}$heterocycloalkyl.

In another embodiment, the optional substituent(s) is/are independently halogen, -OH, $NH_2$, $NH(C_{1-6}alkyl)$, $-N(C_{1-6}alkyl)_2$, $-C_{1-6}$alkyl, $-C_{3-6}$cycloalkyl or $-C_{3-6}$heterocycloalkyl.

The term "polyalkylene glycol" (PAG) refers to compounds having the general formula $H-[O-C_yH_{2y}]_x-OH$, such as $H-[O-CH_2-CH_2]_x-OH$ (polyethylene glycol or PEG) and $H-[O-CH(CH_3)-CH_2]_x-OH$ (polypropylene glycol). When found in a compound of the invention the PAG is bound by the bond between a carbon atom and one of the terminal hydroxyl groups e.g. in the case of PEG the substituent would be $H-[O-CH_2-CH_2]_x-$. The polyalkylene glycols used in the compounds of the invention, unless otherwise defined, may have a molecular weight of from 500 to 20,000 g/mol, preferably from 1,000 to 10,000 g/mol, more preferably from 2,000 to 5,000 g/mol, more preferably from 2,000 to 3,500 g/mol.

As used herein, the term "polymer of **Formula I**" includes pharmaceutically acceptable derivatives thereof and polymorphs, isomers and isotopically labelled variants thereof.

The term "pharmaceutically acceptable derivative" includes any pharmaceutically acceptable salt, solvate, hydrate or prodrug of a polymer of **Formula I**. The pharmaceutically acceptable derivatives suitably refers to pharmaceutically acceptable salts, solvates or hydrates of a polymer of **Formula I**.

The term "pharmaceutically acceptable salt" includes a salt prepared from pharmaceutically acceptable non-toxic acids or bases including inorganic or organic acids and bases.

Polymers of **Formula I** which contain basic, e.g. amino, groups are capable of forming pharmaceutically acceptable salts with acids. Pharmaceutically acceptable acid addition salts of the polymers of **Formula I** may include, but are not limited to, those of inorganic acids such as hydrohalic acids (*e.g.* hydrochloric, hydrobromic and hydroiodic acid), sulfuric acid, nitric acid and phosphoric acids. Pharmaceutically acceptable acid addition salts of the polymers of **Formula I** may include, but are not limited to, those of organic acids such as aliphatic, aromatic, carboxylic and sulfonic classes of organic acids, examples of which include: aliphatic monocarboxylic acids such as formic acid, acetic acid, propionic acid or butyric acid; aliphatic hydroxy acids such as lactic acid, citric acid, tartaric acid or malic acid; dicarboxylic acids such as maleic acid or succinic acid; aromatic carboxylic acids such as benzoic acid, p-chlorobenzoic acid, phenylacetic acid, diphenylacetic acid or triphenylacetic acid; aromatic hydroxyl acids such as o-hydroxybenzoic acid, p-hydroxybenzoic acid, 1-hydroxynaphthalene-2-carboxylic acid or 3-hydroxynaphthalene-2-carboxylic acid; and sulfonic acids such as methanesulfonic acid, ethanesulfonic acid or benzenesulfonic acid. Other pharmaceutically acceptable acid addition salts of the polymers of **Formula I** include, but are not limited to, those of glycolic acid, glucuronic acid, furoic acid, glutamic acid, anthranilic acid, salicylic acid, mandelic acid, embonic (pamoic) acid, pantothenic acid, stearic acid, sulfanilic acid, algenic acid and galacturonic acid. Wherein the polymer of **Formula I** comprises a plurality of basic groups, multiple centres may be protonated to provide multiple salts, e.g. di- or tri-salts of compounds of **Formula I**. For example, a hydrohalic acid salt of a polymer of **Formula I** as described herein may be a monohydrohalide, dihydrohalide or trihydrohalide, *etc.* The salts include, but are not limited to those resulting from addition of any of the acids disclosed above. In one embodiment of the polymer of **Formula I**, two basic groups form acid addition salts. In a further embodiment, the two addition salt counterions are the same species, e.g. dihydrochloride, dihydrosulphide etc. Typically, the pharmaceutically acceptable salt is a hydrochloride salt, such as a dihydrochloride salt.

Polymers of **Formula I** which contain acidic, *e.g.* carboxyl, groups are capable of forming pharmaceutically acceptable salts with bases. Pharmaceutically acceptable basic salts of the polymers of **Formula I** may include, but are not limited to, metal salts such as alkali metal or alkaline earth metal salts (*e.g.* sodium, potassium, magnesium or calcium salts) and zinc or aluminium salts. Pharmaceutically acceptable basic salts of the polymers of **Formula I** may include, but are not limited to, salts formed with ammonia or pharmaceutically acceptable organic amines or heterocyclic bases such as ethanolamines (*e.g.* diethanolamine), benzylamines, N-methyl-glucamine, amino acids (*e.g.* lysine) or pyridine.

Hemisalts of acids and bases may also be formed, *e.g.* hemisulphate salts.

Pharmaceutically acceptable salts of polymers of **Formula I** may be prepared by methods well-known in the art.

For a review of pharmaceutically acceptable salts, see Stahl and Wermuth, Handbook of Pharmaceutical Salts: Properties, Selection and Use (Wiley-VCH, Weinheim, Germany, 2002).

The polymers of **Formula I** may exist in both unsolvated and solvated forms. The term "solvate" includes molecular complexes comprising the polymer and one or more pharmaceutically acceptable solvent molecules such as water or $C_{1-6}$ alcohols, *e.g.* ethanol. The term "hydrate" means a "solvate" where the solvent is water.

The polymers may exist in solid states from amorphous through to crystalline forms. All such solid forms are included within the invention.

The polymers may exist in one or more geometrical, optical, enantiomeric, diastereomeric and tautomeric forms, including but not limited to *cis*- and *trans*-forms, *E*- and *Z*-forms, *R*-, *S*- and *meso*-forms, keto- and enol-forms. All such isomeric forms are included within the invention. The isomeric forms may be in isomerically pure or enriched form, as well as in mixtures of isomers (*e.g.* racemic or diastereomeric mixtures).

The invention includes pharmaceutically acceptable isotopically-labelled polymers of **Formula I** wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as $^{2}H$ and $^{3}H$, carbon, such as $^{11}C$, $^{13}C$ and $^{14}C$, chlorine, such as $^{36}Cl$, fluorine, such as $^{18}F$, iodine, such as $^{123}I$ and $^{125}I$, nitrogen, such as $^{13}N$ and $^{15}N$, oxygen, such as $^{15}O$, $^{17}O$ and $^{18}O$, phosphorus, such as $^{32}P$, and sulphur, such as $^{35}S$. Certain isotopically-labelled polymers of **Formula I**, for example, those incorporating a radioactive isotope, are useful in drug and/or substrate tissue distribution studies. The radioactive isotopes $^{3}H$ and $^{14}C$ are particularly useful for this purpose in view of their ease of incorporation and ready means of detection.

Substitution with positron emitting isotopes, such as $^{11}C$, $^{18}F$, $^{15}O$ and $^{13}N$, can be useful in Positron Emission Topography (PET) studies for examining substrate receptor occupancy.

Isotopically-labelled polymers of **Formula I** can generally be prepared by conventional techniques known to those skilled in the art or by processes analogous to those described herein using an appropriate isotopically-labelled reagent in place of the non-labelled reagent previously employed.

It will be appreciated that the polymers, as described herein, may be substituted with any number of substituents or functional moieties. The terms substituted, whether preceded by the term "optionally" or not, and substituent, as used herein, refer to the ability, as appreciated by one skilled in this art, to change one functional group for another functional

group provided that the valency of all atoms is maintained. When more than one position in any given structure may be substituted with more than one substituent selected from a specified group, the substituent may be either the same or different at every position. The substituents may also be further substituted (e.g., an aryl group substituent may have another substituent off it, such as another aryl group, which is further substituted with fluorine at one or more positions).

The term thiohydroxyl or thiol, as used herein, refers to a group of the formula -SH.

***Virus-based therapeutic agents***

The virus-based therapeutic agent may be any viral vector suitable for use in therapy. In some embodiments, the virus-based therapeutic agent is suitable for use in systemic viral gene therapy. In some embodiments, the virus-based therapeutic agent is an oncolytic viral vector. In some embodiments, the virus-based therapeutic agent is a vaccine.

The virus-based therapeutic agent may be an adenoviral vector, an adeno-associated viral (AAV) vector, or a retroviral vector such as a lentiviral vector. In some embodiments, the virus-based therapeutic agent is an adenoviral vector or an adeno-associated viral vector (AAV). In some embodiments the virus-based therapeutic agent is an adenoviral vector. In some embodiments the virus-based therapeutic agent is an adeno-associated viral vector. In some embodiments the virus-based therapeutic agent is a retroviral vector, such as a lentiviral vector.

In some embodiments the virus is any viral vector suitable for use in therapy other than a retroviral vector. In some embodiments the virus is any viral vector suitable for use in therapy other than a lentiviral vector. In some embodiments the virus is any viral vector suitable for use in therapy other than the oncolytic adenovirus AdNuPARmE1A or AduPARmE1A.

In some embodiments the virus-based therapeutic agent is selected from a herpex simplex viral vector, a vaccinia viral vector, a vesicular stomatitis viral vector, a reoviral vector and a Semliki forest viral vector. In some embodiments the virus-based therapeutic agent is a herpex simplex viral vector. In some embodiments the virus-based therapeutic agent is a vaccinia viral vector.

Preferably, the virus-based therapeutic agent is an adenoviral; vector. In some embodiments the virus-based therapeutic agent is an oncolytic adenoviral vector, including AdNuPARmE1A and AduPARmE1A. In some embodiments the virus-based therapeutic agent is AdNuPARmE1A. In some embodiments the virus-based therapeutic agent is AduPARmE1A.

In some embodiments the virus-based therapeutic agent is enveloped, for example a lentiviral vector. In some embodiments the virus-based therapeutic agent is non-enveloped, for example an adenoviral vector.

In one embodiment, the surface of the virus-based therapeutic agent comprises binding sites suitable for binding to a polymer of **Formula I** wherein the or each oligopeptide has a net positive charge at pH 7. In general the surface of the virus-based therapeutic agent is negatively charged and interacts with positively charged polymers of **Formula I**.

In alternative embodiment, the surface of the virus-based therapeutic agent comprises binding sites suitable for binding to a polymer of **Formula I** wherein the or each oligopeptide has a net negative charge at pH 7.

In the complexes of the present invention, the virus-based therapeutic agent is preferably non-covalently linked to the polymer of **Formula I**, for example, by hydrogen bonding, electrostatic interaction or physical encapsulation, and typically the interaction is electrostatic. Preferably, the virus-based therapeutic agent and polymer of **Formula I** are linked by one or more interactions selected from dipole-dipole interactions, ion-dipole interactions, ion-induced dipole interactions and/or hydrogen-bonding. The virus-based therapeutic agent(s) are suitably encapsulated within the nanoparticles.

In some embodiments, the surface of the virus-based therapeutic agent is negatively charged and the polymers of the invention feature highly positive-charged end termini that interact easily with the viral surface, a slightly positive-charged polymer backbone that helps to stabilize the interaction between the polymer and the viral particle and a hydrophobic side chain that enables the interaction of the polymer with lipid components of the viral envelope

The complexes of the invention unexpectedly provide one or more, and preferably all, of the following properties:

(i) Masking capacity of the virus-based therapeutic agent against neutralizing antibodies;
(ii) Lower activation of the adaptive immune response by the virus-based therapeutic agent;
(iii) Increase in blood circulation time of the virus-based therapeutic agent;
(iv) Decrease in liver tropism of the virus-based therapeutic agent; and
(v) Increased tumor tropism of the virus-based therapeutic agent.

The combined effect of these properties is that the complexes of the invention may allow dosages of the virus-based therapeutic agent which are much higher (for example, possibly at least about 10 to 20 times greater) than the existing conventional regimes to be used.

In a third aspect, the present invention provides a composition comprising a virus-based therapeutic agent coated with polymeric material comprising or consisting of polymer(s) of **Formula I** as defined hereinabove in any one

of the previous aspects of the invention.

***Coated virus-based therapeutic agents***

The present invention provides virus-based therapeutic agents as described herein which are coated with a polymeric material comprising or consisting of polymer(s) of **Formula I** as defined herein according to any of the aspects of the invention.

In some embodiments, the present invention provides a virus-based therapeutic agent as described herein which is coated with a combination of two or more different polymers of **Formula I**, wherein at least one of the polymers is a PAG-ylated or PEG-ylated polymer of **Formula I** and at least one of the polymers does not contain a PAG or PEG moiety.

In some embodiments the present invention provides the adenovirus AdNuPARmE1A (see examples 9 and 10 below) which is coated with a polymeric material comprising or consisting of polymer(s) of **Formula I** as defined herein according to any of the aspects of the invention. In some embodiments, the polymeric material is a combination of two different polymers of **Formula I** as defined herein or described herein above.

In some embodiments the present invention provides a virus-based therapeutic agent coated with a combination of (i) R3C-C6-CR3 (see example 3A below) and (ii) R3C-C6-CR3-PEG (see example 5A below), preferably where polymers (i) and (ii) are present in a ratio of 65:35.

In some embodiments the present invention provides the adenovirus AdNuPARmE1A (see examples 9 and 10 below) coated with a combination of (i) R3C-C6-CR3 (see example 3A below) and (ii) R3C-C6-CR3-PEG (see example 5A below) where polymers (i) and (ii) are present in a ratio of 65:35. This coated virus-based therapeutic agent is referred to as SAG-101.

In some embodiments the invention provides virus-based therapeutic agents as described herein which are coated with a polymeric material comprising or consisting of one or more polymer(s) of **Formula I** as defined herein wherein the coated virus-based therapeutic agent is other than SAG-101.

In some embodiments the invention provides virus-based therapeutic agents as described herein which are coated with a polymeric material comprising or consisting of one or more polymer(s) of **Formula I** as defined herein wherein the virus-based therapeutic agent is a retroviral vector (such as a lentiviral vector), adenoviral vector, adeno-associated viral vector, herpex simplex viral vector, a vaccinia viral vector, a vesicular stomatitis viral vector, a reoviral vector, or a Semliki forest viral vector.

In some embodiments the invention provides virus-based therapeutic agents as described herein which are coated with a polymeric material comprising or consisting of one or more polymer(s) of **Formula I** as defined herein wherein the virus-based therapeutic agent is any viral vector suitable for use in therapy other than a retroviral vector, for example, wherein the virus-based therapeutic agent is an adenoviral vector, adeno-associated viral vector, herpex simplex viral vector, a vaccinia viral vector, a vesicular stomatitis viral vector, a reoviral vector, or a Semliki forest viral vector, for example, wherein the virus-based therapeutic agent is an adenoviral vector or adeno-associated viral vector.

In some embodiments the invention provides virus-based therapeutic agents as described herein which are coated with a polymeric material comprising or consisting of one or more polymer(s) of **Formula I** as defined herein wherein the virus-based therapeutic agent is any viral vector suitable for use in therapy other than a lentiviral vector, for example, wherein the virus-based therapeutic agent is a retroviral vector (other than a lentiviral vector), adenoviral vector, adeno-associated viral vector, herpex simplex viral vector, a vaccinia viral vector, a vesicular stomatitis viral vector, a reoviral vector, or a Semliki forest viral vector, for example, wherein the virus-based therapeutic agent is an adenoviral vector, adeno-associated viral vector, or a retroviral vector other than a lentiviral vector.

In some embodiments the invention provides virus-based therapeutic agents as described herein which are coated with a polymeric material comprising or consisting of one or more polymer(s) of **Formula I** as defined herein wherein the virus-based therapeutic agent is any viral vector suitable for use in therapy other than an adenoviral vector, for example, wherein the virus-based therapeutic agent is a retroviral vector, adeno-associated viral vector, herpex simplex viral vector, a vaccinia viral vector, a vesicular stomatitis viral vector, a reoviral vector, or a Semliki forest viral vector, for example, wherein the virus-based therapeutic agent is an adeno-associated viral vector, a retroviral vector, or a lentiviral vector.

In some embodiments the invention provides virus-based therapeutic agents as described herein which are coated with a polymeric material comprising or consisting of one or more polymer(s) of **Formula I** as defined herein wherein the virus-based therapeutic agent is any viral vector suitable for use in therapy other than the adenovirus AdNuPARmE1A.

In some embodiments the invention provides virus-based therapeutic agents as described herein which are coated with a polymeric material comprising or consisting of one or more polymer(s) of **Formula I** as defined herein wherein the virus-based therapeutic agent is a herpex simplex viral vector, a vaccinia viral vector, a vesicular stomatitis viral vector, a reoviral vector, or a Semliki forest viral vector.

***Nanoparticles***

The composition may comprise nanoparticles and/or microparticles containing the virus-based therapeutic agent coated with the polymeric material of **Formula I**. The composition may comprise two or more different polymers as defined in **Formula I**. For example, the composition may comprise polymers of **Formula I** wherein $R_1$ and $R_2$ are both CysLysLysLys and polymers of **Formula I** wherein $R_1$ and $R_2$ are both CysHisHisHis. The composition may comprise a first polymer of **Formula I** wherein $R_1$ and $R_2$ are both CysArgArgArg and no polyalkylene glycol moiety is present, in combination with a second polymer of **Formula I** wherein $R_1$ and $R_2$ are both CysArgArgArg and a polyalkylene glycol moiety is present e.g. R3C-C6-CR3-PEG or R3C-C6-CR3-linkPEG.

The invention is discussed principally hereinbelow with regard to nanoparticles. It will be understood that the discussion applies equally to microparticles.

The nanoparticles may comprise a virus-based therapeutic agent and polymers of **Formula I** wherein the or each oligopeptide has a net positive charge at pH 7. The positively charged oligopeptides interact with a negatively charged virus-based therapeutic agent during the process of nanoparticle formation and facilitate encapsulation of the virus-based therapeutic agent in the nanoparticles.

The nanoparticles may comprise polymers of **Formula I** wherein the or each oligopeptide has a net negative charge at pH 7 and a virus-based therapeutic agent that has a net positive charge at pH7. The negatively charged oligopeptides interact with a positively charged virus-based therapeutic agent during the process of nanoparticle formation and facilitate encapsulation of the virus-based therapeutic agent in the nanoparticles.

The nanoparticles may optionally comprise a mixture of different polymers of **Formula I**. For example, nanoparticles may comprise

(a) a polymer according to **Formula I** wherein the or each oligopeptide has a net positive charge at pH 7; and
(b) a polymer according to **Formula I** wherein the or each oligopeptide has a net negative charge at pH 7.

Thus, the invention provides nanoparticles with net surface charge that may be varied by modifying the proportions of polymers (a) and (b) above. The ratio of (a) to (b) may be 1:99, 5:95, 10:90, 25:75, 50:50, 75:25, 90:10, 95:5, or 99:1 by weight.

Such nanoparticles are suitable for virus-based therapeutic agent encapsulation and show improved pharmacological properties.

In some embodiments, polymers according to **Formula I** wherein the or each oligopeptide has a net positive charge at pH 7 may be used in combination with polymers according to **Formula I** wherein the or each oligopeptide has a net negative charge at pH 7.

Further, the inclusion of polymers modified with oligopeptides that have a net negative charge at pH 7 may facilitate delivery of the nanoparticles through complex body barriers, such as intestinal and pulmonary mucosa, as the net surface charge changes may vary during the interaction with those barriers.

The nanoparticles may comprise a mixture of two or more different polymers of **Formula I** in combination with the virus-based therapeutic agent. The nanoparticles may comprise a combination of a first polymer of **Formula I** which is PAG-ylated or PEG-ylated, and a second polymer of **Formula I** which is not PAG-ylated or PEG-ylated. For example the nanoparticles may comprise (i) a polymer as defined in the previous aspects of the invention which feature at least one $R_3$ group which is a polyalkylene glycol, in combination with (ii) a second polymer which does not feature an $R_3$ group which is a polyalkylene glycol. The ratio of the two different polymers (i) and (ii) may be 1:99, 5:95, 10:90, 25:75, 35:65, 50:50, 65:35, 75:25, 90:10, 95:5, or 99:1 by weight or by volume. In one embodiment the ratio of polymers (i) and (ii) is from 25:75 to 45:55, preferably 35:65 (v/v).

Nanoparticles of the present invention may be formed with high active agent content and high encapsulation efficiency.

Herein, the active agent encapsulation efficiency refers to the virus-based therapeutic agent incorporated into the nanoparticles as a weight percentage of the total active agent used in the method of preparation of the virus-based therapeutic agent-containing nanoparticles. It is typically up to and including 95%, more typically from 70% to 95%.

Herein, virus-based therapeutic agent entrapment refers to the weight percentage of the viral agent in the viral agent-loaded nanoparticles. Virus-based therapeutic agent entrapment is preferably at least 2 wt%, more preferably at least 5 wt%, more preferably at least 10 wt% and typically in the range of from 2 wt% to 20 wt%, more preferably from 5 wt% to 20 wt%, more preferably from 10 wt% to 20 wt%.

When the composition comprises nanoparticles, preferably, the nanoparticles constitute from about 1% to about 90% by weight of the composition. More preferably, the nanoparticles constitute about 5% to about 50% by weight of the composition, more preferably, about 10% to about 30%. The composition may further comprise a vehicle. The vehicle may be any pharmaceutically acceptable diluent or excipient, as known in the art. The vehicle is typically pharmacologically inactive. Preferably, the vehicle is a polar liquid. Particularly preferred vehicles include water and physiologically

acceptable aqueous solutions containing salts and/or buffers, for example, saline or phosphate-buffered saline. Optionally, the vehicle is a biological fluid. A liquid vehicle may be removed by, for example, lyophilization, evaporation or centrifugation for storage or to provide a powder for pulmonary or nasal administration, a powder for suspension for infusion, or tablets or capsules for oral administration.

Administration of the compositions described herein can be via any of the accepted modes of administration for such compositions including, but not limited to, orally, sublingually, subcutaneously, intravenously, intratumorally, intranasally, topically, transdermally, intraperitoneally, intramuscularly, intrapulmonarilly, vaginally, rectally, or intraocularly. In some embodiments, oral or parenteral administration is used. In some embodiments the compositions are administered intravenously or intratumorally.

The nanoparticles are biocompatible and sufficiently resistant to their environment of use that a sufficient amount of the nanoparticles remain substantially intact after entry into the mammalian body so as to be able to reach the desired target and achieve the desired physiological effect. The polymers described herein are biocompatible and preferably biodegradable.

Herein, the term 'biocompatible' describes as substance which may be inserted or injected into a living subject without causing an adverse response. For example, it does not cause inflammation or acute rejection by the immune system that cannot be adequately controlled. It will be recognized that "biocompatible" is a relative term, and some degree of immune response is to be expected even for substances that are highly compatible with living tissue. An *in vitro* test to assess the biocompatibility of a substance is to expose it to cells; biocompatible substances will typically not result in significant cell death (for example, >20%) at moderate concentrations (for example, 29 $\mu$g/$10^4$ cells).

Herein, the term 'biodegradable' describes a polymer which degrades in a physiological environment to form monomers and/or other non-polymeric moieties that can be reused by cells or disposed of without significant toxic effect. Degradation may be biological, for example, by enzymatic activity or cellular machinery, or may be chemical, typically a chemical process that takes place under physiological conditions. Degradation of a polymer may occur at varying rates, with a half-life in the order of days, weeks, months, or years, depending on the polymer or copolymer used. The components preferably do not induce inflammation or other adverse effects *in vivo*. In certain preferred embodiments, the chemical reactions relied upon to break down the biodegradable compounds are uncatalysed.

Herein, the term 'nanoparticles' refers to a solid particle with a diameter of from about 1nm to less than 1000nm. Herein, the term 'microparticles' refers to a solid particle with a diameter of from 1$\mu$m to about 100$\mu$m. The mean diameter of the nanoparticles of the present invention may be determined by methods known in the art, preferably by dynamic light scattering. In particular, the invention relates to nanoparticles that are solid particles with a diameter of from about 1nm to less than 1000nm when analysed by dynamic light scattering at a scattering angle of 90° and at a temperature of 25°C, using a sample appropriately diluted with filtered water and a suitable instrument such as the Zetasizer™ instruments from Malvern Instruments (UK) according to the standard test method ISO 22412:2008 (cumulants method A. 1.3.2). Where a particle is said to have a diameter of *x* nm, there will generally be a distribution of particles about this mean, but at least 50% by number (e.g. >60%, >70%, >80%, >90%, or more) of the particles will have a diameter within the range $x \pm 20\%$. The diameter of the nanoparticles of the present invention may also be determined by scanning electron microscopy.

Preferably, the diameter of the nanoparticle is from about 10 to less than 1000nm, more preferably from about 5 to about 500nm, more preferably from about 50 to about 400nm, more preferably from about 50 to about 150nm. Alternatively, the diameter of the nanoparticle is from about 1 to about 100nm. In one embodiment, the nanoparticles exhibit a degree of agglomeration of less than 10%, preferably less than 5 %, preferably less than 1%, and preferably the nanoparticles are substantially non-agglomerated, as determined by transmission electron microscopy.

The present invention further provides a method of encapsulating a virus-based therapeutic agent in a matrix of polymer of **Formula I** to form nanoparticles, the method comprising steps of: providing a virus-based therapeutic agent; providing the polymer; and contacting the virus-based therapeutic agent and the polymer under suitable conditions to form nanoparticles. In particular, the virus-based therapeutic agent and polymer may be mixed in solution at concentrations appropriate to obtain the desired ratio, mixed slowly and then incubated in at room temperature for about 30 minutes to enable the electrostatic interaction between the negative surface charge of the virus and the positive charge of the polymer to form. The polymer-virus complex is then ready to be used.

### *Synthetic methods*

A method of synthesizing a polymer of **Formula I** comprises the steps of reacting a compound of **Formula II**, wherein $L_3$ is as defined above, with a compound of formula $L_4H_2$, wherein $L_4$ is as defined above, to produce a polymer of **Formula II** as shown below.

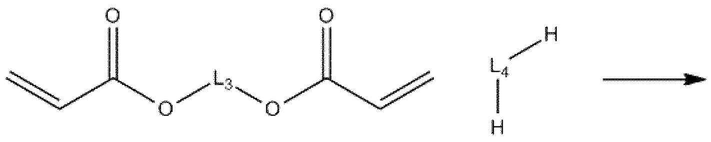

**Formula II**

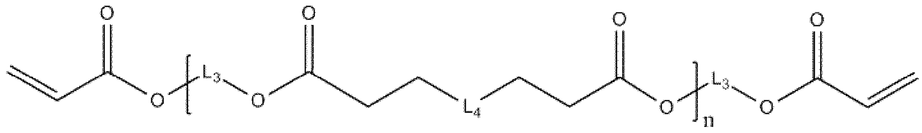

**Formula III.**

The compound of **Formula III** is further reacted with compounds of **Formula IV** to form a compound of **Formula V**:

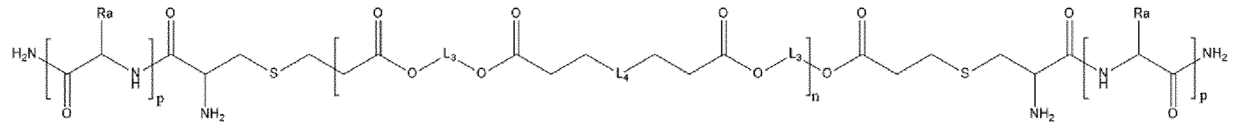

**Formula V,**

wherein p and $R_a$ independently at each occurrence are selected from the lists defined above. In some cases, each occurrence of p is the same and the $R_a$ groups are selected such that the sequence of $R_a$ groups starting from the sulfur linkage is the same at each end of the compound, that is, p and $R_a$ are selected such that the polymer has two-fold symmetry about $L_4$.

In an alternative to the above step, the compound of **Formula III** is further reacted with compounds of formula $H_2NR_y$, wherein $R_y$ is as defined above, and compounds of **Formula IV** and the resulting mixture is separated to obtain a compound of **Formula VI**:

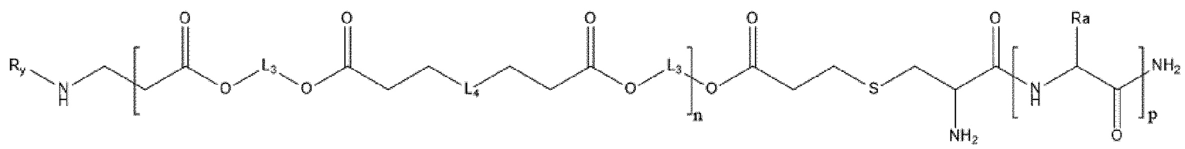

**Formula VI,**

wherein $R_a$ is independently selected at each occurrence from the lists defined above and p is as defined above.

It will be recognized that further methods of attaching an oligopeptide to the compound of **Formula III** would be available to the skilled person, who would be aware of appropriate nucleophiles for reaction at the terminal acrylate groups of **Formula III**.

According to a further aspect of the invention, there is provided a complex or composition as defined herein for use in medicine.

According to a further aspect of the invention, there is provided a complex or composition as defined herein for use in systemic viral gene therapy.

According to a further aspect of the invention, there is provided a complex or composition as defined herein for use in the treatment of cancer. In some embodiments the cancer is liver cancer. In some embodiments the cancer is pancreatic cancer.

BRIEF DESCRIPTION OF THE FIGURES

Figure 1 shows the results of coagulation assays.

Figure 2 shows the results of assays to determine platelet activation. The polymer assays are shown as the left hand bar in each of the "polymer" and "polymer + ADP" results of Figure 2. The DMSO assays are shown as the right hand bar in each of the "polymer" and "polymer + ADP" results of Figure 2.

Figure 3 depicts a schematic representation of the oncolytic adenovirus AdNuPARmE1A.

Figure 4 depicts schematic representations of the adenoviruses AduPARmE1A and AdNuPARmE1A.

Figure 5 shows a virus (1) without the polymer coating according to the invention, and the same virus attacked by a binding antibody (2)

Figure 6 shows a coated virus (1) with a polymer coating (3) according to the invention. The antibody (4) is unable to bind to the virus due to the presence of the polymer coating.

Figure 7 shows the results of assays to determine the masking capacity of the PBAE polymers against neutralizing antibodies.

Figure 8 shows the results of assays to determine the activation of the adaptive immune response, specifically the determination of the ND50 discussed hereinbelow.

Figure 9 shows the results of assays to determine the blood circulation kinetics.

Figure 10 shows the results of assays to determine liver tropism where RLU refers to Relative Light Units.

Figure 11 shows the results of assays to determine tumour tropism where RLU refers to Relative Light Units.

Figure 12 shows the protocol of the anti-tumoral activity study set out in example 16.

Figure 13 shows the results of the study performed in example 16.

Figure 14 shows the results of the study performed in example 16.

Figure 15 shows the results of the study performed in example 17.

Figure 16 shows the results of the study performed in example 17.

Figure 17 shows the results of the study performed in example 17.

Figure 18 shows the results of the study performed in example 17.

Figure 19 shows a transmission electron microscopy micrograph of SAG-101.

Figure 20 shows the results of the study performed in example 19.

Figure 21 shows a scanning electron microscopy micrograph of the nanoparticles in example 20.

The invention is further illustrated by the following examples. It will be appreciated that the examples are for illustrative purposes only and are not intended to limit the invention as described above. Modification of detail may be made without departing from the scope of the invention.

EXAMPLES

*Example 1:* Synthesis of PBAE polymers

Poly($\beta$-aminoester)s were synthesized following a two-step procedure, described in the literature (e.g. in Montserrat, N. et al. J. Biol. Chem. 286, 12417-12428 (2011)). First, an acrylate-terminated polymer was synthesized by addition reaction of primary amines with diacrylates (at 1:1.2 molar ratio of amine:diacrylate). Finally, PBAEs were obtained by end-capping modification of the resulting acrylate-terminated polymer with different kinds of amine- and

thiol-bearing moieties. Synthesized structures were confirmed by $^1$H-NMR and FT-IR analysis. NMR spectra were recorded in a 400 MHz Varian (Varian NMR Instruments, Claredon Hills, IL) and methanol-$d_4$ was used as solvent. IR spectra were obtained using a Nicolet Magna 560 (Thermo Fisher Scientific, Waltham, MA) with a KBr beamsplitter, using methanol as solvent in evaporated film. Molecular weight determination was conducted on a Hewlett-Packard 1050 Series HPLC system equipped with two GPC Ultrastyragel columns, $10^3$ and $10^4$ Å (5 μm mixed, 300 mm x 19 mm, Waters Millipore Corporation, Milford, MA, USA) and THF as mobile phase. The molecular weight was calculated by comparison with the retention times of polystyrene standards.

*Example 2:* Synthesis of acrylate terminated intermediate (C32)

1,4-butanediol diacrylate (8.96g, $4.07\times10^{-2}$ mol) and 5-amino-1-pentanol (3.5g, $3.39\times10^{-2}$ mol) were mixed in a vial. The mixture was stirred at 90°C for 24h, and then cooled to room temperature to form a slightly yellow viscous solid, the acrylate terminated intermediate (designated C32). Intermediate C32 was stored at 4°C before being used in subsequent steps.

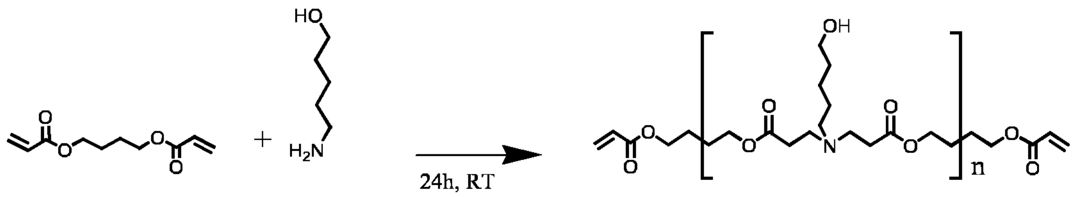


C 32 C32

*Example 2A:* Synthesis of acrylate terminated intermediate (C6)

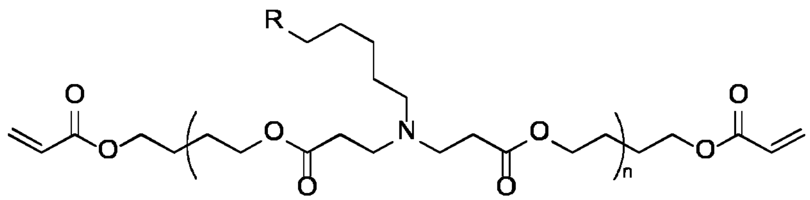

R= $^-CH_3$ or $^-OH$

(C6)

In a round-bottomed flask were mixed 5-amino-1-pentanol (3.9 g, 38 mmol), hexylamine (3.8 g, 38 mmol) and 1,4-butanediol diacrylate (18 g, 82 mmol) and the reaction was stirred at 90 °C under nitrogen for 18 h. After cooling down to room temperature, the product (designated C6) was collected as a yellow oil (25 g, n = 8, Mw = 2300). The product was analysed by NMR and GPC.

$^1$H-NMR ($CDCl_3$): 6.40 (dd, 2H, *J* 17.3, 1.5 Hz), 6.11 (dd, 2H, *J* 17.3, 10.4 Hz), 5.82 (dd, 2H, *J* 10.4, 1.5 Hz), 4.18 (m, 4H), 4.08 (m, 32 H), 3.61 (m, 16H), 2.76 (m, 32H, *J* 7.2 Hz), 2.41 (m, 48H), 1.69 (m, 32H), 1.56 (m, 8H), 1.49-1.20 (m, 40H) and 0.87 (t, 12H, *J* 6.9 Hz) ppm.

*Example 2B:* Synthesis of acrylate terminated intermediate featuring disulfide bond

4-amino-1-butanol or 5-amino-1-pentanol was polymerized with an equal molar mixture of hexane-1,6-diyl diacrylate and disulfanediylbis(ethane-2,1-diyl)diacrylate to form acrylate terminated intermediates featuring a disulfide bond.

*Example 3:* Synthesis of PBAEs end-modified with oligopeptides

In general, oligopeptide-modified PBAEs were obtained as follows: acrylate-terminated polymer C32 or C32SS and either amine- or thiol-terminated oligopeptide (for example, HS-Cys-Arg-Arg-Arg (CR3), $H_2N$-Arg-Arg-Arg (R3) or HS-Cys-Glu-Glu-Glu (CE3) - other oligopeptides are indicated by similar abbreviations using the standard one-letter code) were mixed at 1:2 molar ratio in DMSO. The mixture was stirred overnight at room temperature and the resulting

polymer was obtained by precipitation in diethyl ether:acetone (3:1).

(a) The following synthetic procedure to obtain tri-arginine end-modified PBAEs is shown as an example: Intermediate C32 was prepared as described in Example 2 above. A solution of intermediate C32 (0.15 g, 0.075 mmol) in DMSO (2 ml) was mixed with the corresponding solution of oligopeptide (Cys-Arg-Arg-Arg (CR3; 0.11 g, 0.15 mmol)) in DMSO (1 mL) in an appropriate molar ratio, 1:2 respectively. The mixture was stirred overnight at room temperature, then was precipitated in diethyl ether/acetone (3:1).

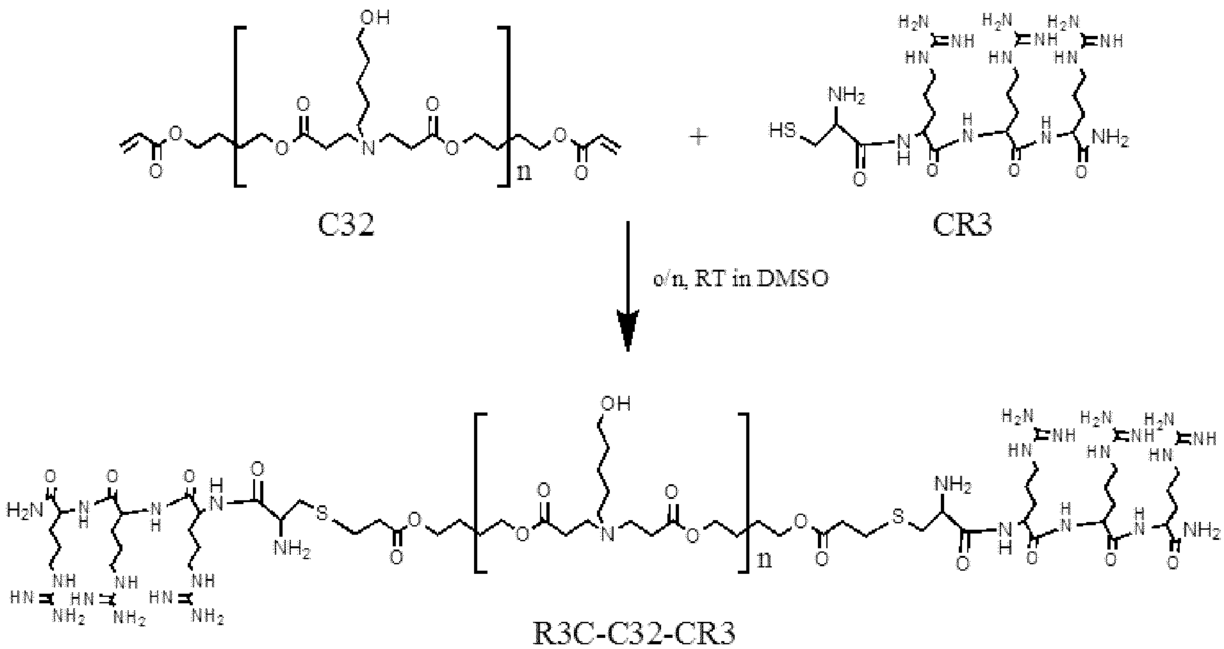

C32 + CR3

R3C-C32-CR3

**IR** (evaporated film): ν = 721, 801, 834, 951, 1029, 1133 (C-O), 1201, 1421, 1466, 1542, 1672 (C=O, from peptide amide), 1731 (C=O, from ester), 2858, 2941, 3182, 3343 (N-H, O-H) $cm^{-1}$ **$^1$H-NMR** (400 MHz, $CD_3OD$, TMS) (ppm): δ = 4.41-4.33 (br, $NH_2$-C(=O)-CH-NH-C(=O)-CH-NH-C(=O)-CH-NH-C(=O)-CH-$CH_2$-, 4.11 (t, $CH_2$-$CH_2$-O), 3.55 (t, $CH_2$-$CH_2$-OH), 3.22 (br, $NH_2$-C(=NH)-NH-$CH_2$-, OH-$(CH_2)_4$-$CH_2$-N-), 3.04 (t, $CH_2$-$CH_2$-N-), 2.82 (dd, -$CH_2$-S-$CH_2$), 2.48 (br, -N-$CH_2$-$CH_2$-C(=O)-O), 1.90 (m, $NH_2$-C(=NH)-NH-$(CH_2)_2$-$CH_2$-CH-), 1.73 (br, -O-$CH_2$-$CH_2$-$CH_2$-$CH_2$-O), 1.69 (m, $NH_2$-C(=NH)-NH-$CH_2$-$CH_2$-$CH_2$-), 1.56 (br, -$CH_2$-$CH_2$-$CH_2$-$CH_2$-OH), 1.39 (br, -N-$(CH_2)_2$-$CH_2$-$(CH_2)_2$-OH).

(b) Tri-lysine modified oligopeptides (K3C-C32-CK3) were prepared according to the same protocol and characterized as follows:

**IR** (evaporated film): ν = 721, 799, 834, 1040, 1132, 1179 (C-O), 1201, 1397, 1459, 1541, 1675 (C=O, from peptide amide), 1732 (C=O, from ester), 2861, 2940, 3348 (N-H, O-H) $cm^{-1}$ **$^1$H-NMR** (400 MHz, $CD_3OD$, TMS) (ppm): δ = 4.38-4.29 (br, $NH_2$-$(CH_2)_4$-CH-), 4.13 (t, $CH_2$-$CH_2$-O-),3.73 (br,$NH_2$-CH-$CH_2$-S-), 3.55 (t, $CH_2$-$CH_2$-OH), 2.94 (br, $CH_2$-$CH_2$-N-, $NH_2$-$CH_2$-$(CH_2)_3$-CH-), 2.81 (dd, -$CH_2$-S-$CH_2$), 2.57 (br, -N-$CH_2$-$CH_2$-C(=O)-O), 1.85 (m, $NH_2$-$(CH_2)_3$-$CH_2$-CH-), 1.74 (br, -O-$CH_2$-CH2-CH2-$CH_2$-O), 1.68 (m, $NH_2$-$CH_2$-$CH_2$-$(CH_2)_2$-CH-), 1.54 (br, -$CH_2$-$CH_2$-$CH_2$-$CH_2$-OH), 1.37 (br, -N-$(CH_2)_2$-$CH_2$-$(CH_2)_2$-OH).

(c) Tri-histidine modified oligopeptides (H3C-C32-CH3) were prepared according to the same protocol and characterized as follows:

**IR** (evaporated film): ν = 720, 799, 832, 1040, 1132, 1201, 1335, 1403, 1467, 1539, 1674 (C=O, from peptide amide), 1731 (C=O, from ester), 2865, 2941, 3336 (N-H, O-H) $cm^{-1}$ **$^1$H-NMR** (400 MHz, $CD_3OD$, TMS) (ppm): δ = 8.0-7.0 (br -N(=CH)-NH-C(=CH)-) 4.61-4.36 (br, -CH2-CH-), 4.16 (t, $CH_2$-$CH_2$-O-), 3.55 (t, $CH_2$-$CH_2$-OH), 3.18 (t, $CH_2$-$CH_2$-N-, 3.06 (dd, -$CH_2$-CH-), 2.88 (br, OH-$(CH_2)_4$-$CH_2$-N-), 2.82 (dd, -$CH_2$-S-$CH_2$-), 2.72 (br, -N-$CH_2$-$CH_2$-C(=O)-O), 1.75 (br, -O-$CH_2$-CH2-CH2-$CH_2$-O), 1.65 (m, $NH_2$-$CH_2$-$CH_2$-$(CH_2)_2$-CH-), 1.58 (br, -$CH_2$-$CH_2$-$CH_2$-$CH_2$-OH), 1.40 (br, -N-$(CH_2)_2$-$CH_2$-$(CH_2)_2$-OH).

*Example 3A:* Synthesis of PBAEs end-modified with oligopeptides (R3C-C6-CR3)

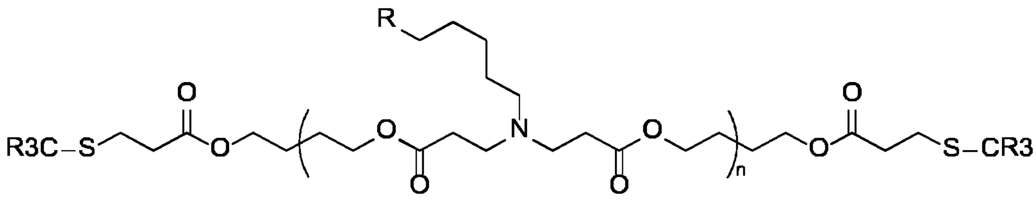

R= $^-CH_3$ or $^-OH$

To obtain the chlorhydrate of the peptide, 20 mL of 0.1 M HCl were added to peptide CRRR (200 mg) and the solution was freeze-dried.

In a round-bottomed flask were mixed a solution of the PBAE C6 (113 mg, 0.054 mmol) in dimethyl sulfoxide (1.1 mL) and a solution of peptide CRRR chlorhydrate (99 mg, 0.13 mmol) in dimethyl sulfoxide (1 mL). The reaction was stirred at room temperature under nitrogen for 24 h. The reaction mixture was added over diethyl ether-acetone (7:3) and a white precipitate was obtained. The suspension was centrifuged at 4000 rpm for 10 min and the solvent was take off. The solid was washed two times with diethyl ether-acetone (7:3) and dried under vacuum to obtain a white solid (233 mg). The product was analysed by NMR (MeOD) and the structure was in concordance.

*Example 3B:* Synthesis of other PBAEs end-modified with oligopeptides

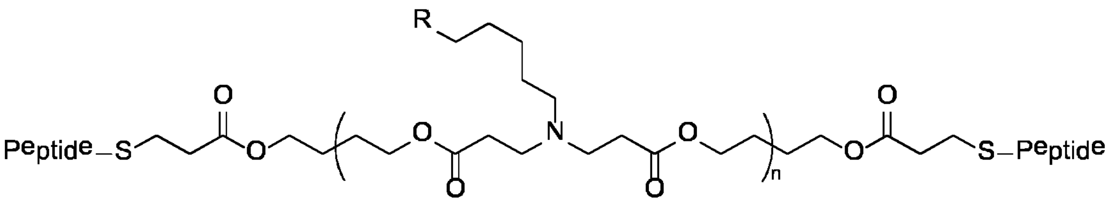

R= $^-CH_3$ or $^-OH$

The same procedure described for the synthesis of PBAE R3C-C6-CR3 was used with PBAE C6 for the syntheses of:

- PBAE H3C-C6-CH3 with peptide CHHH.
- PBAE K3C-C6-CK3 with peptide CKKK.
- PBAE D3C-C6-CD3 with peptide CDDD.
- PBAE E3C-C6-CE3 with peptide CEEE.

*Example 3C: Synthesis of other PBARs end-modified with oligopeptides*

The procedure described above for the synthesis of PBAE R3C-C6-CR3 can be used with PBAE C32 for the synthesis of:

- PBAE R3C-C32-CR3 with peptide CRRR.
- PBAE H3C-C32-CH3 with peptide CHHH.
- PBAE K3C-C32-CK3 with peptide CKKK.
- PBAE D3C-C32-CD3 with peptide CDDD.
- PBAE E3C-C32-CE3 with peptide CEEE.

*Example 4:* Synthesis of PBAEs with asymmetric end modifications

In general, asymmetric oligopeptide-modified PBAEs were obtained as follows: Acrylate-terminated polymer C32 (or C32SS) and either amine- or thiol-terminated oligopeptide (for example, CR3, R3 or CE3) were mixed at 1:1 molar ratio in DMSO. The mixture was stirred overnight at room temperature. Equimolar amount of a second amine- or thiol-terminated oligopeptide, or of a primary amine, was added and the mixture was stirred overnight at room temperature.

The resulting asymmetric PBAE polymers were obtained by precipitation in diethyl ether/acetone (3:1).

The following synthetic procedure to obtain asymmetric end-modified B3-C32-CR3 PBAEs is shown as an example: a solution of intermediate C32 (0.15 g, 0.075 mmol) in DMSO (2 mL) was mixed with the corresponding solution of oligopeptide Cys-Arg-Arg-Arg (CR3; 0.055 g, 0.075 mmol) in DMSO (1 ml) and was stirred overnight at room temperature. Subsequently, 2-methyl-1,5-pentanediamine (0.017 g, 0.02 ml, 0.15 mmol) was added in the mixture for 4h at room temperature in DMSO. A mixture of asymmetric end-modified polymer B3-C32-CR3 with B3-C32-B3 and R3C-C32-CR3 was obtained by precipitation overnight in diethyl ether/acetone (3:1). The asymmetric end-modified polymer B3-C32-CR3 may be separated from the mixture by standard methods.

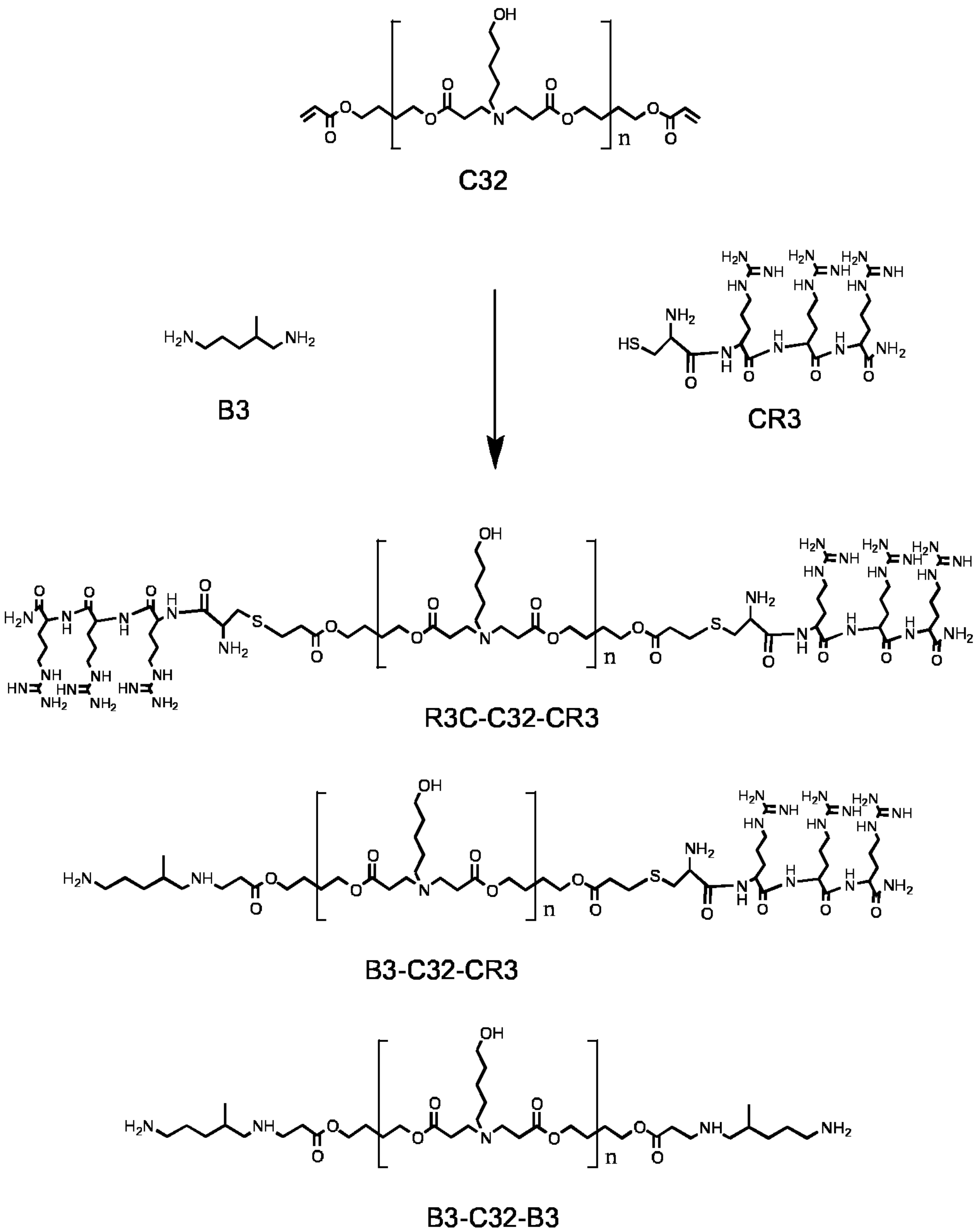

C32

B3

CR3

R3C-C32-CR3

B3-C32-CR3

B3-C32-B3

*Example 5A:* Synthesis of PEG modified PBAE

Step 1: Synthesis of MeO-PEG-COOH

To a solution of MeO-PEG (5 g, Mw = 2000, 2.5 mmol) and succinic anhydride (0.275 g, 2.75 mmol) in dichloromethane (5 mL) was added triethylamine (0.174 mL, 1.25 mmol). The reaction mixture was stirred at room temperature for 4 h and washed with 1 M HCl (1 ml) twice. The organic phase was washed with brine twice and dried over $MgSO_4$. The solid was filtered off and the solvent was evaporated under vacuum to obtain a white solid (4.47 g). The product

was analysed by NMR ($CDCl_3$) and the structure was in concordance.

Step 2: Synthesis of N-boc 5-amino-1-pentanol

To a solution of 5-amino-1-pentanol (0.525 g, 5.1 mmol) and triethylamine (0.779 mL, 5.6 mmol) in dichloromethane (16 mL) was added a solution of di-tert-butyl dicarbonate (1.1 g, 5.1 mmol) in dichloromethane (5 mL). The mixture was stirred at room temperature for 1 h and then washed with 0.5 M HCl (1 ml) three times. The organic phase was dried over $MgSO_4$. The solid was filtered off and the solvent was evaporated under vacuum to obtain a white solid (1.3 g). The product was analysed by NMR ($CDCl_3$) and the structure was in concordance.

Step 3: Synthesis of MeO-PEG-NHBoc

To a solution of MeO-PEG-COOH (1 g, 0.49 mmol) in dichloromethane (14 mL) were added dicyclohexylcarbodiimide (151 mg, 0.74 mmol) and N,N'-dimethylaminopyridine (9 mg, 0.074 mmol). After 5 min, a solution of N-boc 5-amino-1-pentanol (100 mg, 0.49 mmol) in dichloromethane (1 mL) was added to the mixture. The reaction mixture was stirred at room temperature for 6 h and then the solid was filtered off. The solvent was evaporated under vacuum and the residue was washed with diethyl ether (5 mL) three times. The product was dried under vacuum to obtain a white solid (0.940 g). The compound was analysed by NMR ($CDCl_3$) and the structure was in concordance with the anticipated structure.

Step 4: Synthesis of MeO-PEG-$NH_2$

To a solution of MeO-PEG-NHBoc (464 mg, 0.21 mmol) in dichloromethane (3 mL) was added trifluoroacetic acid (1.2 mL) at 0 °C. The reaction mixture was stirred at 0 °C for 10 min and then it was stirred at room temperature for 2 h. The solvent was reduced under vacuum and the residue was washed with diethyl ether (5 mL) twice. The product was dissolved in dichloromethane (8 mL) and washed with 0.5 M NaOH (1 ml) twice. The organic phase was washed with brine and dried over $MgSO_4$. The solid was filtered off and the solvent was evaporated under vacuum to obtain a white solid (319 mg). The product was analysed by NMR ($CDCl_3$) and the structure was in concordance with the anticipated structure.

Step 5: Synthesis of PBAE C6-PEG

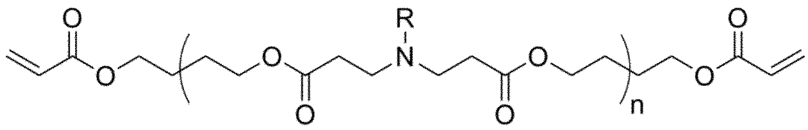

R = $-CH_2-CH_2-CH_2-CH_2-CH_2-CH_3$
$-CH_2-CH_2-CH_2-CH_2-CH_2-OH$
-PEG-OMe

5-Amino-1-pentanol (42 mg, 0.41 mmol), hexylamine (41 mg, 0.41 mmol) and MeO-PEG-$NH_2$ (314 mg, 0.14 mmol) were mixed in dichloromethane (2 mL) and the solvent was reduced under vacuum. To the residue was added 1,4-butanediol diacrylate (198 mg, 1 mmol) and the reaction mixture was stirred at 90 °C under nitrogen for 18 h. After cooling down to room temperature, the product was collected as a yellow solid (527 mg, n = 7). The product was analysed by NMR ($CDCl_3$) and the structure was in concordance.
$^1$H-NMR ($CDCl_3$): 6.40 (dd, 2H, *J* 17.3, 1.5 Hz), 6.11 (dd, 2H, *J* 17.3, 10.4 Hz), 5.82 (dd, 2H, *J* 10.4, 1.5 Hz), 4.18 (m, 4H), 4.08 (m, 28 H), 3.63 (m, $-OCH_2-CH_2O-$, PEG), 3.37 (s, CH3O-, PEG), 2.76 (m, 28H), 2.43 (m, 42H), 1.80-1.20 (m) and 0.87 (t, 12H, *J* 6.9 Hz) ppm.

Step 6: Synthesis of PBAE R3C-C6-CR3-PEG

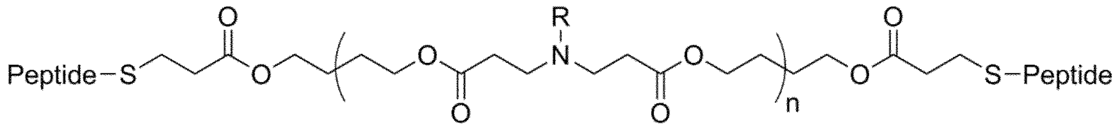

R = $-CH_2-CH_2-CH_2-CH_2-CH_2-CH_3$
$-CH_2-CH_2-CH_2-CH_2-CH_2-OH$
-PEG-OMe

To obtain the chlorhydrate of the peptide, 15 mL of 0.1 M HCl were added to peptide CRRR (150 mg) and the solution was freeze-dried.

In a round-bottomed flask were mixed a solution of PBAE C6-PEG (92 mg, 0.022 mmol) in dimethyl sulfoxide (1.2 mL) and a solution of peptide CRRR chlorhydrate (40 mg, 0.054 mmol) in dimethyl sulfoxide (1.1 mL). The reaction was stirred at room temperature under nitrogen for 20 h. The reaction mixture was added over diethyl ether-acetone (7:3) and a white precipitate was obtained. The suspension was centrifuged at 4000 rpm for 10 min and the solvent was taken off. The solid was washed two times with diethyl ether-acetone (7:3) and dried under vacuum to obtain a white solid (133 mg). The product was analyzed by NMR (MeOD) and the structure was in concordance.

*Example 5B:* Synthesis of PEG modified PBAEs where the PEG is bound to the PBAE through a linker moiety

Step1: Synthesis of methoxy-PEG Acid

1. Add methoxy-PEG (5 g, 2.5 mmol) into a round-bottom flask.
2. Add dichloromethane (5 mL) to the flask.
3. Add succinic anhydride (0.275 g, 2.75 mmol) to the solution.
4. Add trietylamine (0.174 mL, 1.25 mmol) to the mixture.
5. Then, stir the mixture at room temperature for 4 h.
6. Wash the mixture reaction with 1M HCl (1 ml) twice.
7. Wash the solution with brine twice.
8. Dry the organic phase over $MgSO_4$.
9. Filter off the solid and evaporate the solvent under vacuum.

Step 2: Reaction of esterification

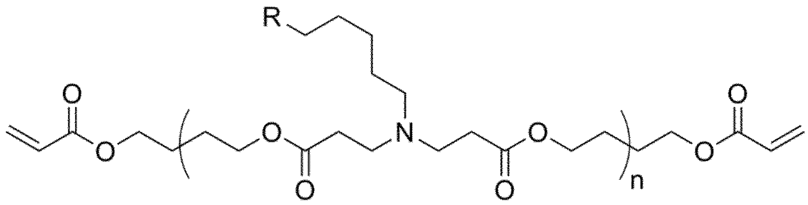

R = $-CH_3$, -OH, or -PEG-OMe

1. Add methoxy-PEG acid (230 mg, 0.11 mmol) in a screw tap tube.
2. Add dichloromethane (1.5 mL) to the tube.
3. Add dicyclohexylcarbodiimide (34 mg, 0.17 mmol) to the solution.
4. Stir the solution for 20 min at room temperature.
5. Add a solution of C6 PBAE (200 mg, 0.099 mmol) in dichloromethane (1 mL).
6. Then, stir the mixture at room temperature for 20 h.
7. Filter off the solid and evaporate the solvent under vacuum.

Step 3: Reaction with peptides

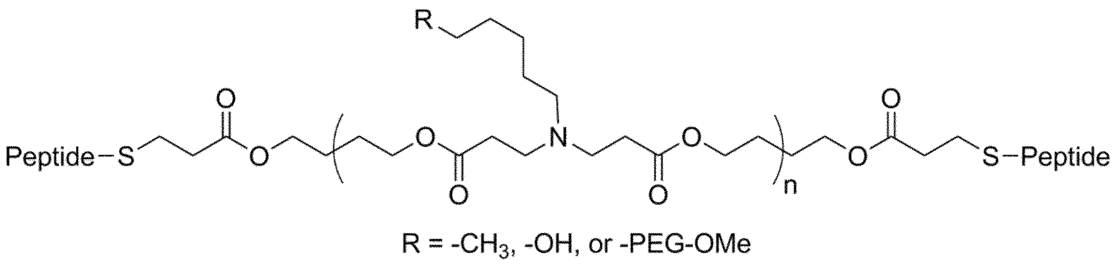

1. Add 0.1 M HCl (20 mL) to peptide Cys-Arg-Arg-Arg (200 mg).
2. Freeze the solution at - 80 °C and freeze-dried the peptide.
3. Make a solution of C6-linkPEG PBAE (114 mg, 0.027 mmol) in dimethylsulfoxide (0.8 mL).
4. Make a solution of Cys-Arg-Arg-Arg (50 mg, 0.068 mmol) in dimethylsulfoxide (0.8 mL).
5. Mix the two solutions in a screw tap tube.
6. Stir the mixture solution at room temperature for 20 h.
7. Add dropwise the mixture to 7:3 diethyl ether/acetone (8 mL).
8. Centrifuge the suspension at 4000 rpm for 10 min and remove the solvent.
9. Wash the solid with 7:3 diethyl ether/acetone (4 mL) twice.
10. Dry the product under vacuum.
11. Make a solution of 100 mg/mL of the product in dimethylsulfoxide.

*Example 6:* Library of compounds

A library of different oligopeptide end-modified PBAEs was synthesized by adding primary amines to diacrylates followed by end-modification. According to **Formula I**, the oligopeptide end-modified PBAEs shown in Table 1 were synthesized.

*Table 1:* Library of oligopeptide end-modified PBAEs wherein at least one of $R_1$ and $R_2$ is an oligopeptide

| Polymer | $L_3$ | $L_4$ | $HL_1-R_1$ | $HL_2-R_2$ |
|---|---|---|---|---|
| R3-C32-R3 | $-CH_2-(CH_2)_2-CH_2-$ | $>N-(CH_2)_5-OH$ | $H_2N$-Arg-Arg-Arg | $H_2N$-Arg-Arg-Arg |
| K3-C32-K3 | $-CH_2-(CH_2)_2-CH_2-$ | $>N-(CH_2)_5-OH$ | $NH_2$-Lys-Lys-Lys | $H_2N$-Lys-Lys-Lys |
| H3-C32-H3 | $-CH_2-(CH_2)_2-CH_2-$ | $>N-(CH_2)_5-OH$ | $NH_2$-His-His-His | $NH_2$-His-His-His |
| R3C-C32-CR3 | $-CH_2-(CH_2)_2-CH_2-$ | $>N-(CH_2)_5-OH$ | HS-Cys-Arg-Arg-Arg | HS-Cys-Arg-Arg-Arg |
| K3C-C32-CK3 | $-CH_2-(CH_2)_2-CH_2-$ | $>N-(CH_2)_5-OH$ | HS-Cys-Lys-Lys-Lys | HS-Cys-Lys-Lys-Lys |
| H3C-C32-CH3 | $-CH_2-(CH_2)_2-CH_2-$ | $>N-(CH_2)_5-OH$ | HS-Cys-His-His-His | HS-Cys-His-His-His |
| B3-C32-R3 | $-CH_2-(CH_2)_2-CH_2-$ | $>N-(CH_2)_5-OH$ | $H_2N-CH_2-(CH_2)_2-CH(CH_3)-CH_2-NH_2$ | $H_2N$-Arg-Arg-Arg |
| B3-C32-CR3 | $-CH_2-(CH_2)_2-CH_2-$ | $>N-(CH_2)_5-OH$ | $H_2N-CH_2-(CH_2)_2-CH(CH_3)-CH_2-NH_2$ | HS-Cys-Arg-Arg-Arg |
| B3-C32-CK3 | $-CH_2-(CH_2)_2-CH_2-$ | $>N-(CH_2)_5-OH$ | $H_2N-CH_2-(CH_2)_2-CH(CH_3)-CH_2-NH_2$ | HS-Cys-Lys-Lys-Lys |
| B3-C32-CH3 | $-CH_2-(CH_2)_2-CH_2-$ | $>N-(CH_2)_5-OH$ | $H_2N-CH_2-(CH_2)_2-CH(CH_3)-CH_2-NH_2$ | HS-Cys-His-His-His |

(continued)

| Polymer | $L_3$ | $L_4$ | $HL_1$-$R_1$ | $HL_2$-$R_2$ |
|---|---|---|---|---|
| R3C-C32-CK3 | $-CH_2-(CH_2)_2-CH_2-$ | $>N-(CH_2)_5-OH$ | HS-Cys-Arg-Arg-Arg | HS-Cys-Lys-Lys-Lys |
| R3C-C32-CH3 | $-CH_2-(CH_2)_2-CH_2-$ | $>N-(CH_2)_5-OH$ | HS-Cys-Arg-Arg-Arg | HS-Cys-His-His-His |
| K3C-C32-CH3 | $-CH_2-(CH_2)_2-CH_2-$ | $>N-(CH_2)_5-OH$ | HS-Cys-Lys-Lys-Lys | HS-Cys-His-His-His |
| R3C-C32SS-CR3 | $-CH_2-CH_2-S-S-CH_2-CH_2-$ | $>N-(CH_2)_5-OH$ | HS-Cys-Arg-Arg-Arg | HS-Cys-Arg-Arg-Arg |
| K3C-C32SS-CK3 | $-CH_2-CH_2-S-S-CH_2-CH_2-$ | $>N-(CH_2)_5-OH$ | HS-Cys-Lys-Lys-Lys | HS-Cys-Lys-Lys-Lys |
| H3C-C32SS-CH3 | $-CH_2-CH_2-S-S-CH_2-CH_2-$ | $>N-(CH_2)_5-OH$ | HS-Cys-His-His-His | HS-Cys-His-His-His |
| B3-C32SS-CR3 | $-CH_2-CH_2-S-S-CH_2-CH_2-$ | $>N-(CH_2)_5-OH$ | $H_2N-CH_2-(CH_2)_2-CH(CH_3)-CH_2-N H_2$ | HS-Cys-Arg-Arg-Arg |
| B3-C32SS-CK3 | $-CH_2-CH_2-S-S-CH_2-CH_2-$ | $>N-(CH_2)_5-OH$ | $H_2N-CH_2-(CH_2)_2-CH(CH_3)-CH_2-N H_2$ | HS-Cys-Lys-Lys-Lys |
| B3-C32SS-CH3 | $-CH_2-CH_2-S-S-CH_2-CH_2-$ | $>N-(CH_2)_5-OH$ | $H_2N-CH_2-(CH_2)_2-CH(CH_3)-CH_2-NH_2$ | HS-Cys-His-His-His |
| R3C-C32SS-CK3 | $-CH_2-CH_2-S-S-CH_2-CH_2-$ | $>N-(CH_2)_5-OH$ | HS-Cys-Arg-Arg-Arg | HS-Cys-Lys-Lys-Lys |
| R3C-C32SS-CH3 | $-CH_2-CH_2-S-S-CH_2-CH_2-$ | $>N-(CH_2)_5-OH$ | HS-Cys-Arg-Arg-Arg | HS-Cys-His-His-His |
| K3C-C32SS-CH3 | $-CH_2-CH_2-S-S-CH_2-CH_2-$ | $>N-(CH_2)_5-OH$ | HS-Cys-Lys-Lys-Lys | HS-Cys-His-His-His |
| D3C-C32-CD3 | $-CH_2-(CH_2)_2-CH_2-$ | $>N-(CH_2)_5-OH$ | HS-Cys-Asp-Asp-Asp | HS-Cys-Asp-Asp-Asp |
| E3C-C32-CE3 | $-CH_2-(CH_2)_2-CH_2-$ | $>N-(CH_2)_5-OH$ | HS-Cys-Glu-Glu-Glu | HS-Cys-Glu-Glu-Glu |
| D3C-C32-CE3 | $-CH_2-(CH_2)_2-CH_2-$ | $>N-(CH_2)_5-OH$ | HS-Cys-Asp-Asp-Asp | HS-Cys-Glu-Glu-Glu |
| E3C-C32SS-CD3 | $-CH_2-CH_2-S-S-CH_2-CH_2-$ | $>N-(CH_2)_5-OH$ | HS-Cys-Glu-Glu-Glu | HS-Cys-Asp-Asp-Asp |
| E3C-C32SS-CE3 | $-CH_2-CH_2-S-S-CH_2-CH_2-$ | $>N-(CH_2)_5-OH$ | HS-Cys-Glu-Glu-Glu | HS-Cys-Glu-Glu-Glu |
| D3C-C32SS-CE3 | $-CH_2-CH_2-S-S-CH_2-CH_2-$ | $>N-(CH_2)_5-OH$ | HS-Cys-Asp-Asp-Asp | HS-Cys-Glu-Glu-Glu |
| R3C-C6-CR3 | $-CH_2-(CH_2)_2-CH_2-$ | $>N-(CH_2)_5-OH$<br>>N-(CH2)5-CH3 | HS-Cys-Arg-Arg-Arg | HS-Cys-Arg-Arg-Arg |
| H3RC-C6-CRH3 | $-CH_2-(CH_2)_2-CH_2-$ | $>N-(CH_2)_5-OH$<br>$>N-(CH_2)_5-CH_3$ | HS-Cys-Arg-His-His-His | HS-Cys-Arg-His-His-His |
| R3C-C6-CR3-linkPEG | $-CH_2-(CH_2)_2-CH_2-$ | $>N-(CH_2)_5-OH$<br>$>N-(CH_2)_5-CH_3$<br>$>N-(CH_2)_5$-PEG-OMe | HS-Cys-Arg-His-His-His | HS-Cys-Arg-His-H is-H is |

(continued)

| Polymer | $L_3$ | $L_4$ | $HL_1$-$R_1$ | $HL_2$-$R_2$ |
|---|---|---|---|---|
| R3C-C6-CR3-PEG | $-CH_2-(CH_2)_2-CH_2-$ | $>N-(CH_2)_5-OH$ | HS-Cys-Arg-Arg-Arg | HS-Cys-Arg-Arg-Arg |
| | | $>N-(CH_2)_5-CH_3$ | | |
| | | >N-PEG-OMe | | |

*Table 2:* Library of end-modified PBAEs (reference compounds)

| Polymer | $L_3$ | $L_4$ | $HL_1$-$R_1$ | $HL_2$-$R_2$ |
|---|---|---|---|---|
| B3 | $-CH_2-(CH_2)_2-CH_2-$ | $>N-(CH_2)_5-OH$ | $NH_2-CH_2-(CH_2)_2-CH(CH_3)-CH_2-NH_2$ | $H_2N-CH_2-(CH_2)_2-CH(CH_3)-CH_2-NH_2$ |
| B3-C32SS-B3 | $-CH_2-CH_2-S-S-CH_2-CH_2-$ | $>N-(CH_2)_5-OH$ | $H_2N-CH_2-(CH_2)_2-CH(CH_3)-CH_2-N\ H_2$ | $H_2N-CH_2-(CH_2)_2-CH(CH_3)-CH_2-NH_2$ |

*Example 7: Coagulation assay*

The coagulation cascade is generally divided into three pathways. The effect of the polymers described in the present application on the three coagulation pathways was evaluated by measuring three representative parameters. Specifically, the intrinsic pathway was measured by the activated partial thromboplastin time, the extrinsic pathway was measured by the prothrombin time and the final common pathway was evaluated by measuring the thrombin time. The possible changes induced in the coagulation cascade due to binding or depletion of the coagulation factors with the polymers described in the present invention were evaluated measuring the time necessary to form a clot formation.

The polymer used contains 65% of R3C-C6-CR3 and 35% of R3C-C6-CR3-PEG. Three different concentrations were studied: 355 μg/ml, 213 μg/ml and 106.5 μg/ml. Briefly, the polymers were incubated with human pool plasma from at least three donors. Clot formation was detected by a viscosity-based detection system, using a hemostasis analyzer, which measures in seconds. This system avoids interference due to physicochemical attributes of the sample. The reference values for each pathway are as follows: partial thromboplastin time (APTT) ≤ 34.1 sec, prothrombin time (PT) ≤ 13.4 sec and thrombin time (TT) ≤ 21 sec. There is no guidance on the degree of prolongation, but generally prolongation ≥ 2-fold versus normal control is considered physiologically significant.

As shown in Figure 1, there were no alterations in the coagulation times of the pooled human plasma (negative control) with respect to the normal control. Normal and pathological (abnormal) controls were used as internal controls of the technique. Since the polymer stocks were dissolved in DMSO, a control of the dissolution was also included in the assays. DMSO alone did not show changes in coagulation times.

As shown in Figure 1, the polymers did not induce significant alterations on coagulation times at any of the concentrations tested. The coagulation times were within the normal limits after exposure of the polymer at 355 μg/mL, 213 μg/mL and 106.5 μg/mL. The polymer decreases the prothrombin time (PT) at the highest concentrations tested and it was statistically significant with respect to the negative control.

*Example 8: Platelet activation assay*

Platelet activation comes with degranulation and activation of endothelial cells, leukocytes and other platelets, which ultimately cause formation of a thrombus. The platelets are small anucleate discoid cells involved in primary hemostasis. Their internal structure and membrane play a central role in platelet activation. One of the most reliable markers for platelet activation is CD62P. This is a platelet-specific selectin protein, which is expressed on the internal α-granule membrane of resting platelets. This receptor mediates tethering and rolling of platelets on the surface of activated endothelial cells. Upon platelet activation and granule secretion, the α-granule membrane fuses with the external plasma membrane and the CD62P antigen is expressed on the surface of the activated platelet.

The effect of the polymers described in the present application to induce or inhibit platelet activation was measured by the expression of CD62P on the surface of the activated platelet by flow cytometry. The polymer used contained 65% of R3C-C6-CR3 and 35% of R3C-C6-CR3-PEG. Three different concentrations were studied: 355 μg/ml, 213 μg/ml and 106.5 μg/ml. The results were normalized with respect to basal level (negative control). A result was considered positive if the relative fluorescent intensity was > 2.0 with respect to the negative control.

As shown in Figure 2, the polymers did not induce platelet activation at any of the concentrations tested when it was incubated with a pool of platelet-rich plasma (PRP). Since the polymer stock was dissolved in DMSO, a control of the dissolution was also included in the assays. As shown in Figure 2, DMSO did not induce platelet activation.

As a control of the potential inhibitory effect of the polymer on platelet activation, the assay was also performed with ADP (adenosine diphosphate). The polymer did not inhibit the platelet activation in presence of ADP. The results suggest that polymers described in the present application do not induce or inhibit platelet activation under the conditions tested.

*Example 9:* AdNuPARmE1A virus

**Structure**

Notch-responsive genes are characterized by a DNA-binding domain, recognizing the CSL transcription factor, in the promoter region. The presence of dual "sequence-paired" CSL-binding sites (SPS) orientated head-to-head and separated by 16nt promotes the dimerization of the Notch transcriptional complex, leading to transcriptional activation of Notch target genes, such as Hes1 (Nam Y, Sliz P, Pear WS, Aster JC, Blacklow SC. Cooperative assembly of higher-order Notch complexes functions as a switch to induce transcription. Proc Natl Acad Sci USA. 2007; 104:2103-2108).

The AdNuPARmE1A contains a synthetic promoter, engineered with three sequences that respond to Notch signalling activation (SPS) and a minimal uPAR promoter, controlling E1A expression. Moreover, the 214 bp short interspersed nuclear element B2 from the growth hormone boundary region (SINEB2) is inserted upstream the uPAR promoter to act as an insulator sequence to avoid any possible unspecific transcriptional activation of E1A by the ITR viral promoter, which would lead to a decrease in tumour selectivity. Expression of the E1A adenoviral gene is controlled by the 3xSPSuPARm promoter. SINEB2 insulator sequence was cloned upstream the promoter sequence (see Figure 3).

**Production and analysis**

The oncolytic adenovirus AdNuPARmE1A is generated by first cloning the 3xSPSuPARm promoter into a pShuttle vector and inserting the SINEB2 insulator upstream the promoter to generate pShSINE3xSPSuPARmE1A. Homologous recombination of pShSINE3xSPSuPARmE1A vector with the adenoviral genome, is performed following the standard protocol to generate pAdNuPARmE1A. Recombinant genomes are then transfected in HEK293 cells and amplified in A549 cells and purified by standard caesium chloride banding (Mato-Berciano A1, Raimondi G, Maliandi MV, Alemany R, Montoliu L, Fillat C. A NOTCH-sensitive uPAR-regulated oncolytic adenovirus effectively suppresses pancreatic tumor growth and triggers synergistic anticancer effects with gemcitabine and nab-paclitaxel. Oncotarget. 2017; 8(14) 22700-22715).

Adenoviral concentration is determined by two different methods:

a) the physical particle concentration (vp/ml) is determined by optical density reading (OD260)
b) the plaque forming units (pfu/ml) are determined on HEK293 cells by the anti-hexon staining-based method.

*Example 10:* AduPARmE1A and AdNuPARmE1A viruses

An oncolytic AduPARmE1A virus in which the E1A gene expression was regulated by the uPAR promoter was generated. A Kozak sequence was engineered upstream of the E1A gene to increase its replication potency (this sequence on an mRNA molecule is recognized by the ribosome as the translational start site, from which a protein is coded by that mRNA molecule). A DNA fragment from the myotonic dystrophy locus (DM-1), with enhancer-blocking insulator activity, was introduced upstream the uPAR promoter to isolate it from enhancer and transcriptional units from the adenovirus genome (see Figure 4).

The AdNuPARmE1A has the same structure, but a shorter version of the uPAR promoter and three responsive elements capable of binding to the Notch intracellular domain (NICD) (see Figure 2).

*Example 11:* Formation and characterization of polymer-virus complexes

**Considerations before preparing the complex**

The virus stock must be titrated by vp/ml and by pfu/ml and the ratio between vp/pfu must be under 100. If this quality acceptance criteria is not reached, the virus production needs to be repeated. It is necessary to have the physical Vp/ml titer before proceeding with this procedure.

The main formulation used is R3C-C6-CR3/R3C-C6-CR3-PEG with a ratio of 65/35 but this protocol can be

adapted to use other formulations. The only must is to maintain the ratio 4e6 molecules PBAE/ vp.

**Procedure for *in vitro* scale**

1. Take a 2μl virus aliquot from the -80°C freezer (*The freeze-thawing process of adenoviral vectors is not recommended, since they lose infection efficiency. For this reason, when producing the virus, it is strongly recommended to aliquot it in small aliquots, in order to thaw only the fraction intended for use*). The virus should be thawed very slowly on ice.
2. Thaw both polymers and prepare a mixture at 65/35 v/v. The polymer mixture can be stored at -20°C.
3. When preparing coated virus to be used *in vitro*, an entire 2μl aliquot is coated. Prepare a 1/50 dilution of virus stock in PBS (2 μl of virus + 98 μl PBS). The resulting solution is labelled as **VS**.
4. Calculate the amount of polymer needed (μl PBAE stock) to coat all viral particles in VS following equation 1.

$$mPBAEs = \left(\frac{VPt}{1000} * Vstock\ ul\right) * 4e6$$

$$X\ ul\ PBAE\ stock = \frac{mPBAEs * 3287{,}52\frac{g}{mol} * 1e4}{6{,}023e23}$$

Equation 1. VPt refers to the physical titer in VP/ml

5. Prepare polymer solution (**PS**) by mixing PBS with the calculated volume of PBAE stock to a final volume of 100μl.
6. Mix **PS** and **VS** by adding **PS** over **VS** and pipetting up & down slowly at least 10 times.
7. Incubate the sample for 30 minutes at room temperature to enable the electrostatic interaction between the negative surface charge of the virus and the positive charge of the polymer.
8. Now the sample is ready to be used taking into account that the resulting titer is 100 fold diluted. It can be diluted with complete medium to reach desired working concentration.

**Procedure for *in vivo* scale**

1. Calculate the Total **VPs** needed taking into account n° of animals (with an excess of 1 animal every 4 animals) and dose (typically $4\times10^{10}$ vp/animal injected I.V. in 100 μl bolus injection through the tail vein).
2. Calculate the volume of virus stock needed (**Vstock**) to prepare an injectable solution of 4e11 VPs/ml in final volume of 1 ml physiologic saline solution 0,9% sodium chloride. (The concentration of the injectable solution depends on the working dose, in this case $4\times10^{10}$ VP/animal)
3. Calculate the total amount of PBAE stock solution needed to coat all viral particles.

$$mPBAEs = (4e11\ total\ VPs) * 4e6$$

$$X\ ul\ PBAE\ stock = \frac{mPBAEs * 3287{,}52\frac{g}{mol} * 1e4}{6{,}023e23}$$

4. Prepare polymer solution (**PS**) by mixing 0,9% saline with the calculated volume of PBAE stock to a final volume of 200 μl.
5. Prepare virus solution (**VS**) by mixing 0,9% saline with the calculated volume of virus stock to a final volume of 200μl.
6. Mix **PS** and **VS** by adding **PS** over **VS** and pipetting up & down slowly at least 10 times.
7. Incubate the sample for 30 minutes at room temperature to enable the electrostatic interaction between the negative surface charge of the virus and the positive charge of the polymer.
8. Add 600 μl of 0,9% saline and mix pipetting slowly 5 times.
9. The sample is ready to be injected (e.g. the sample can treat 8 animals).

**Examples 12-16**

All non-clinical data, except for the anti-tumoral activity results (example 16), have been obtained with a recombinant serotype 5 Adenovirus, called AdTrackluc (AdTL), which expresses two reporter genes, GFP and luciferase. Furthermore, in the *in vivo* studies, this virus has been combined with two different polymeric coatings: **C6Ad**, which corresponds to a 100% R3C-C6-CR3 coating and **CPEGAd**, which stands for a combination of 65% R3C-C6-CR3 and 35% R3C-C6-CR3-PEG.

*Example 12:* Masking capacity against neutralizing antibodies

In order to determine which polymer combination was the best one to protect adenoviruses from anti-Ad5 neutralizing antibodies (Nabs), viral particles were coated with different combinations of R3C-C6-CR3 with H3C-C6-CH3 and R3C-C6-CR3-PEG polymers, and the naked and coated samples were then incubated with Nabs during 30 minutes. Naked adenoviruses were used as sample control. Then, viral preparations (MOI 50) were added to 96 well plates containing $1{,}5x10^4$ PANC-1 cells. After 2 h, the media was changed and cells were incubated 48h. Finally, GPF positive cells were quantified by flow cytometry analysis.

The polymer combinations tested are as follows:

| Designation | R3C-C6-CR3 | R3C-C6-CR3-PEG | H3C-C6-CH3 |
|---|---|---|---|
| C6CR3-25% | 75% | 25% | 0% |
| C6CR3-35% | 65% | 35% | 0% |
| C6CR3-45% | 55% | 45% | 0% |
| C6CRH3 | 60% | 0% | 40% |
| C6CRH3-25% | 35% | 25% | 40% |

As Figure 7 shows, when naked adenoviruses are incubated in the presence of neutralizing antibodies (+ Naked), their transduction capacity is significantly reduced from 80% to 55%. Among the different coating combinations, it was observed that one coating containing 65% of R3C-C6-CR3 and 35% of R3C-C6-CR3-PEG (named C6CR3-35% in the graph) conferred an outstanding protection against the anti-Ad5 neutralizing antibodies (see the single result highlighted in the rectangle). Furthermore, the coating not only did not decrease the infectivity of the virus, but induced an increase in its potency (see the multiple results highlighted in the larger rectangle).

*Example 13:* Activation of the adaptive immune response

The method by which naked and coated viral particles activated the adaptive immune response after two intravenous administrations was also studied. C57BL/6J mice (n=6) were divided into three groups (Naked Ad, R3C-C6-CR3 Ad, and R3C-C6-CR3-35%PEG-Ad). $1\times10^{10}$ vp/animal were injected, at days 0 and 14, in the tail vein of C57BL/6J mice (n=5) and one week later, at day 21, animals were sacrificed and blood was collected by intracardiac puncture. Next, sera were extracted and heat inactivated and they were used to perform neutralization assays in the presence of naked Adenoviruses.

In order to compare the antibody concentration of each sample, the neutralizing dilution 50 (ND50) for each anti-serum was calculated. The ND50, defined as the dilution of the serum needed to neutralize half of the viral transduction, was determined as follows. Naked Ads (MOI 0.25) were incubated for 1 hour in 96-well plates with serial dilutions of sera from mice immunized with naked or coated Ads. Then, $1x10^5$ HEK293 cells were added to each well. After 24 h, luciferase activity was quantified and ND50 was calculated.

As shown in Figure 8, animals injected with CPEGAd produced a three times less neutralizing sera than those injected with naked Ads, indicating that the CPEG-coating strategy (65% R3C-C6-CR3 + 35%R3C-C6-CR3-PEG) did reduce the activation of the adaptive immune response. In the case of C6Ad, this difference was less striking.

*Example 14:* Increase in blood circulation time

In order to compare the blood circulation kinetics of naked and coated viral particles, $1\times10^{10}$ vp/animal were injected in the tail vein of CC57BL/6J mice (n=5). Three groups were established, naked-Ad, C6Ad and CPEGAd, and blood samples were extracted from the saphenous vein at two time points post injection: 2 minutes and 10 minutes. Next, genomic DNA extraction was performed from each blood sample and quantified viral genomes/$\mu$l using hexon

specific primers by qPCR.

The blood circulation kinetics were determined as follows. Genomic DNA was extracted from each blood sample and viral genomes copies were quantified using hexon specific primers by qPCR. The 100% condition (equivalent to the injected dose) was analyzed by diluting the administered dose in 2 ml of whole mice blood before DNA extraction. The area under the curve was calculated from 2 minutes to 10 minutes and fold-change transformed.

As Figure 9 shows, coated viral particles had an improved circulation time, especially those coated with the CPEG combination. In particular, their area under the curve, from 2 minutes to 10 minutes, was 3-fold bigger when compared to naked viral adenoviruses.

*Example 15A:* Liver tropism

One of the main problems associated with adenoviruses is their high tropism towards the liver, which is responsible for their significant hepatotoxicity. In order to determine if the polymeric coating of the invention could decrease this natural behaviour, $1\times10^{10}$ vp of naked and coated (C6Ad and CPEGAd) adenoviruses were administered in the tail vein of C57BL/6J mice (n=5) and 5 days later, whole body bioluminescent images were taken and luciferase activity was quantified from liver homogenates of mice treated with naked Ads or coated with two different polymers (C6Ad, CPEGAd).

As Figure 10 shows, both types of coated viral particles significantly reduced hepatocytes transducing capacity, indicating that the polymeric coating could indeed modify the natural adenoviral tropism.

*Example 15B:* Tumour tropism

To determine if the decreased liver tropism observed with coated Ads also takes place in tumour bearing mice the following study was performed. $1\times10^{6}$ PANC-1 cells (derived from human pancreatic adenocarcinoma) were administered subcutaneously in inmunodeficient Balb/C nu/nu mice and when tumours reached a volume around 150mm$^3$, $1\times10^{10}$ vp of naked and coated (C6Ad and CPEGAd) adenoviruses were administered in the tail vein (n=6). Five days post injection, animals were sacrificed and luciferase activity (used as reporter gene) was quantified from tumours and liver homogenates of mice treated with naked Ads or Ads coated with one of two different polymers (C6Ad, CPEGAd).

As can be seen in Figure 11, apart from the previously observed significant decreased transduction in the liver, when Ads were coated with the polymeric coating named CPEG a marked increased infection in the tumours was detected. This trend was not observed with the C6Ad, probably due to the lack of PEG in the formulation. Altogether, these results led to a significantly increased tumour-liver ratio when CPEGAd were administered in comparison to naked Ads.

*Example* 16: Increasing antitumoral activity

According to the data obtained with the two different coated recombinant Adenovirus (AdTL), C6Ad and CPEGAd, the latter coating consisting of 65% of R3C-C6-CR3 + 35% R3C-C6-CR3-PEG was chosen to be combined with the oncolytic adenovirus AdNuPARmE1A in order to form SAG-101.

In order to study the effect of the polymeric coating on the therapeutic effect of the virus, an efficacy study in tumour bearing mice was performed. In particular, the efficacy of the coated AdNuPARmE1A (SAG-101) after systemic administration was compared with that of naked AdNuPARmE1A in naive or pre-immune mice. To generate a pre-immune status in nude mice, the mice were passively immunized by an intraperitoneal injection of anti-Ad5 neutralizing serum from C57BL6 mice (nude mice bearing subsutaneous PANC-1 tumours were injected intraperitoneally with either PBS (naïve groups) or anti-Ad5 neutralizing mice serum (pre-immune groups)). The next day, naïve or passively immunized nude mice bearing PANC-1 tumours were injected intravenously with PBS, or $4\times10^{10}$ vp of AdNuPARmE1A naked or coated (SAG-101) (n=8) and the tumour volume was monitored. Figure 12 summarizes the protocol of this experiment performed to determine the effect of the polymeric coating on the therapeutic effect of the AdNuPARmE1A. Each group had n=8.

As shown in Figure 13, a significant reduction in tumour growth was observed in three of the treated groups (naked and coated naive AdNuPARmE1A and pre-immunized coated AdNuPARmE1A) compared with the saline control and the pre-immunized naked virus groups (*Results are expressed as mean +/- SEM (*p* < 0.05; ***p < 0.01; ***p < 0.001)*). Importantly, the pre-immunized coated group showed a very similar effect when compared to the naïve naked virus, indicating that the polymeric coating protected the virus from the pre-existing neutralizing antibodies. Moreover, the naïve coated group was significantly more efficacious than the naïve naked group. An overview of the median survival results and change in the fraction survival over time for each group tested is set out in Figure 14.

The experimental data demonstrate that the coated viral particles unexpectedly exhibit the following properties:

1. a reduced tendency to be neutralized by antibodies;
2. a reduced *de novo* adaptive immune response generation capacity;
3. improved bloodstream kinetics; and
***4.*** a decreased liver tropism, to the benefit of tumor transduction.

*Example 17:*

In order to study the toxicity of the coated AdNuPARmE1A (SAG-101), a toxicity study in mice was performed. The coating consisted of **CPEGAd**, which stands for a combination of 65% R3C-C6-CR3 and 35% R3C-C6-CR3-PEG. Doses of SAG-101 after intravenous administration were compared with that of naked AdNuPARmE1A (Ad) in immunocompetent BALB/c mice. The immunocompetent mice were injected intravenously with PBS, or $4\times10^{10}$ vp of AdNuPARmE1A naked or coated (i.e. "low-dose"), or $7.5\times10^{10}$ vp of AdNuPARmE1A naked or coated (i.e. "high-dose").

*Example 17A:* Body weight

As shown in Figure 15, no significant ($p > 0.05$) changes in body weight were observed between groups, although mice receiving high-dose naked AdNuPARmE1 (Ad) did show an important body weight decrease at day five post-injection. The group which was administered high-dose SAG-101 showed a decrease in body weight, indicating that less toxicity was associated with the coated viral particles.

*Example 17B:* Transaminases levels

Serum enzymatic transaminases activity was determined at day 7 post virus IV injection. Specifically, aspartate transaminase (AST) and alanine transaminase (ALT) levels were measured from blood taken from an intracardiac puncture.

Aminotransferases (AST, ALT) are commonly analyzed in serum to assess and monitor liver damage and possible viral infections of the liver. These enzymes are elevated in many forms of liver disease, presumably as a result of leakage from damaged cells. ALT is mainly found in the liver, but also in smaller amounts in the kidneys, heart, muscles, and pancreas. AST is present in the liver but also in considerable amounts in other tissues including the muscles.

As shown in Figure 16, transaminases levels were significantly (*$p < 0.05$) higher when a high-dose of naked virus (AdNu) was administered, indicating liver damage. This effect was not observed in mice that receive the same dose of SAG-101. Thus, the experimental data demonstrate that the coated viral particles provide a decrease in transaminase levels.

*Example 17C:* Hemogram and platelet count

A hemogram analysis and platelet count was conducted. The platelet count was based on blood extraction from the tail vein of the mice every other day from day 1 until day 7. As shown in Figure 17B, no significant changes in the hemogram were detected at day 7 between mice receiving naked AdNuPARmE1A (Ad) or coated AdNuPARmE1A (CPEGAd).

Furthermore, no thrombocytopenia was observed, as shown in Figure 17A. There were no significant differences between the number of platelets in mice receiving naked AdNuPARmE1A (Ad) or coated AdNuPARmE1A (SAG101).

*Example 17D:* Cytokine quantification

Levels of cytokines were measured. At six hours and three days post-injection, blood aliquots were collected and cytokine concentration was evaluated using the Luminex xMAP® technology platform. As shown in Figure 18, no significant differences in the levels of cytokines were observed after the administration of naked AdNuPARmE1A and SAG-101, indicating that the SAG-101 polymers did not increase the toxicity of the virus.

*Example 18:* TEM micrograph

Figure 19A provides a TEM micrograph showing SAG-101 at a low magnification (scale bar 20μm) and Figure 19B provides a TEM micrograph showing SAG-101 at a high magnification (scale bar 200nm). As observed in Figures 19A and 19B, no big aggregates were observed when analyzing the samples at low magnification.

*Example 19:* Surface charge change

Adeno-associated viral (AAV) particles were coated with 65% of R3C-C6-CR3 + 35% R3C-C6-CR3-PEG polymers. The polymeric coating formation was tracked by assessing the surface charge change in order to determine suitable coating concentrations i.e. suitable ratios of AAV to polymer.

The surface of AAVs are negatively charged (as can be seen in Figures 20A and 20B), whereas the polymer used offered a net positive charge. Thus, once the polymer effectively covered the virus, the surface charge of the complex was positive. This surface charge change was therefore assessed to track polymeric coating formation. Different ratios were tested in order to follow their surface charge change. Figure 20B shows a plateau in the surface charge measurement. Thus, adding additional polymer was illogical. In Figure 20A, closer spaced polymer/virus ratios were tested in order to see a progressive change of the surface charge.

As can be seen in Figure 20, when ratios of 1e-9 $\mu$g PBAE/ AAV viral particle (VP) or higher were used, positive surfaces were observed, as indicated by the positive Z potential value measurements, which indicates that the AAV particles were coated. As can be seen in Figure 20B, the variability in Z potential value measurements for the free polymer is very high compared to the values when forming a coating of AAV.

*Example 20:* Scanning electron microscopy

Adeno-associated viral (AAV) particles ("naked AAV"), AAV particles coated **C6Ad**, which corresponds to a 100% R3C-C6-CR3 coating and AAB particles coated with **CPEGAd**, which stands for a combination of 65% R3C-C6-CR3 and 35% R3C-C6-CR3-PEG were characterized by scanning electron microscopy, as shown in Figure 21. Briefly, 200$\mu$l of each sample (naked and coated virus) were prepared (as described in other examples) at a final concentration of $10^{11}$ vp/ml (which was the required concentration to obtain an optimal visualization). Samples were deposited on the grid and incubated there for one minute. Next, they were irradiated with gold for 40 seconds to create a thin layer over them. Finally, they were analysed with a Field Emission Scanning Electron Microscope.

Furthermore, scanning electron microscopy was used to determine the nanoparticle diameter (in nm):

| *(all in nm)* | **Naked AAV** | **C6-AAV** | **CPEG-AAV** | **Aggregates of C6-AAV** |
|---|---|---|---|---|
| **Mean** | 24.81 | 50.32 | 68.12 | 150.40 |
| **Desvest** | 4.52 | 16.79 | 13.24 | 53.59 |
| **Max** | 39.05 | 119.65 | 118.46 | 372.55 |
| **Min** | 10.98 | 17.21 | 27.75 | 49.48 |
| **Counts** | 1000 | 1000 | 1000 | 1000 |

A broader distribution appears for the C6-AAV nanoparticles due to the presence of aggregates. As shown by the data above, all aggregates were smaller than 500nm and thus this sample could also be used for intravenous administration.

**EMBODIMENTS**

1. A complex of a virus-based therapeutic agent with a polymer of formula I:

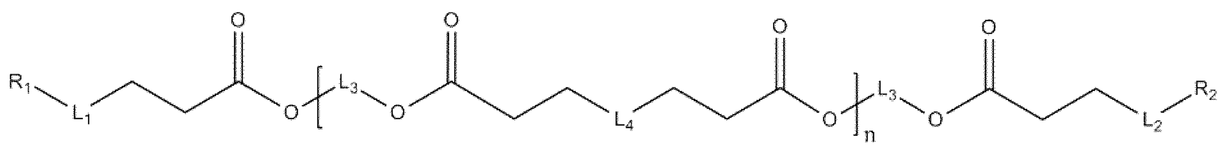

**Formula I**

wherein

each $L_1$ and $L_2$ is independently selected from the group consisting of:

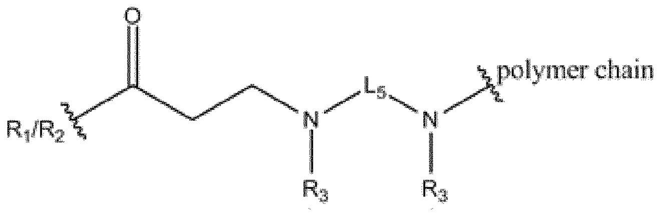

,

O, S, $NR_x$ and a bond; wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl;

$L_3$ is independently selected from the group consisting of alkylene, alkenylene, heteroalkylene, heteroalkenylene, arylene or heteroarylene; or

at least one occurrence of $L_3$ is

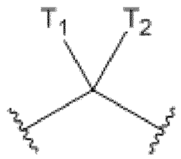

,

wherein $T_1$ is

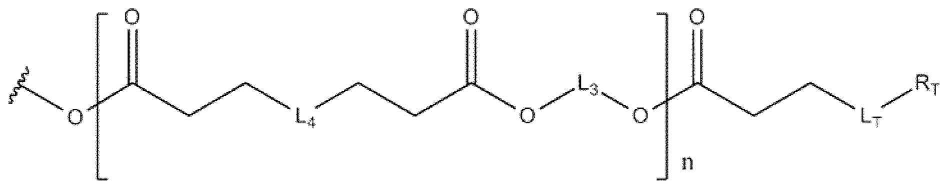

and $T_2$ is selected from H, alkyl or

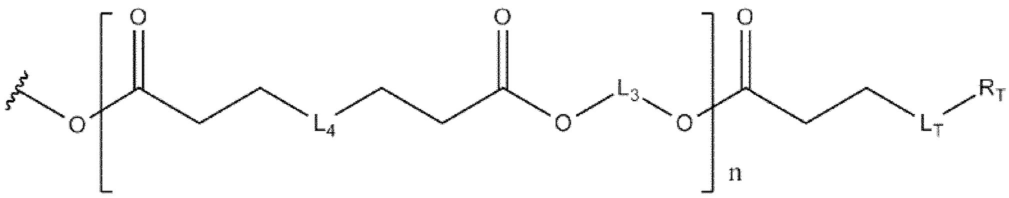

;

wherein $L_T$ is independently selected from the group consisting of:

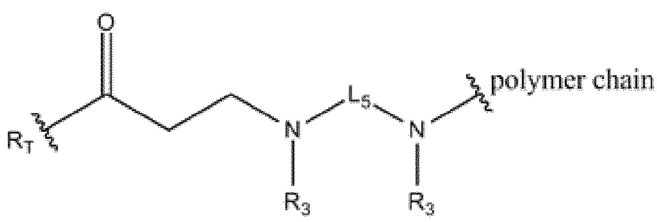

,

O, S, $NR_x$ and a bond, wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl, and the remaining $L_3$ groups are independently selected at each occurrence from the group consisting of alkylene, alkenylene, heteroalkylene, heteroalkenylene, arylene or heteroarylene; $L_4$ is selected from the group consisting of

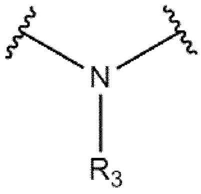 and 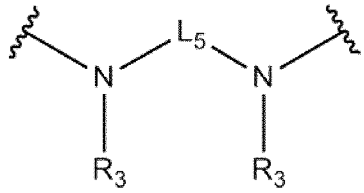 ;

L5 is independently selected from the group consisting of alkylene, alkenylene, heteroalkylene, heteroalkenylene, arylene or heteroarylene;
$R_1$ and $R_2$ and $R_T$ (if present) are independently selected from an oligopeptide and $R_y$;
wherein at least one of $R_1$ and $R_2$ and $R_T$ (if present) is an oligopeptide;
and wherein $R_y$ is selected from the group consisting of hydrogen, halogen, alkyl, cycloalkyl, alkenyl, cycloalkenyl, heteroalkyl, heterocycloalkyl, acyl, aryl or heteroaryl;
each $R_3$ is independently selected from the group consisting of hydrogen, halogen, alkyl, cycloalkyl, alkenyl, cycloalkenyl, heteroalkyl, heterocycloalkyl, acyl, aryl, heteroaryl and polyalkylene glycols, wherein said polyalkylene glycol is either bound directly to the nitrogen atom to which $R_3$ is attached or bound to the nitrogen atom to which $R_3$ is attached via a linker moiety, wherein said linker moiety is an alkylene, cycloalkylene, alkenylene, cycloalkenylene, heteroalkylene, heterocycloalkylene, arylene or heteroarylene group; and
n is an integer from 5 to 1,000;

or a pharmaceutically acceptable salt thereof.

2. The complex according to embodiment 1, wherein $L_3$ is independently selected from the group consisting of alkylene, alkenylene, heteroalkylene, heteroalkenylene, arylene or heteroarylene.

3. The complex according to embodiment 1, wherein at least one occurrence of $L_3$ is

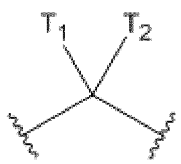 ,

wherein $T_1$ is

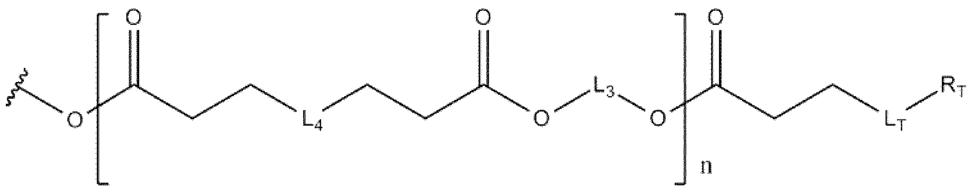

and $T_2$ is selected from H, alkyl or

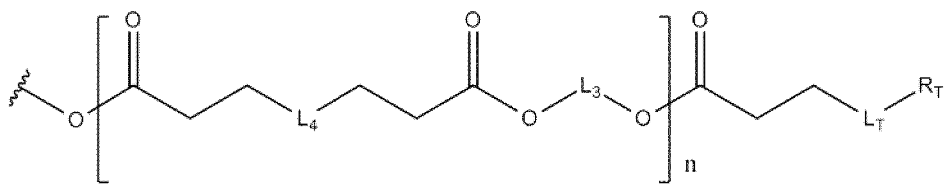 ;

wherein $L_T$ is independently selected from the group consisting of:

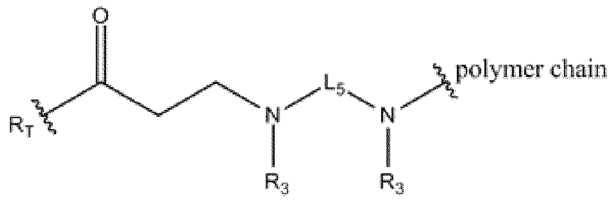

,

O, S, $NR_x$ and a bond; wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl, and the remaining $L_3$ groups are independently selected at each occurrence from the group consisting of alkylene, alkenylene, heteroalkylene, heteroalkenylene, arylene or heteroarylene.

4. The complex according to any one of embodiments 1 to 3, wherein at least one $R_3$ group is a polyalkylene glycol, preferably a polyethylene glycol.

5. The complex according to embodiment 4, wherein the at least one $R_3$ group which is a polyalkylene glycol is bound to the nitrogen atom of an $L_4$ group either directly or through a linker moiety.

6. The complex according to embodiment 4 or embodiment 5, wherein the at least one $R_3$ group which is a polyalkylene glycol is bound to the nitrogen atom to which it is attached through a linker moiety which is an alkylene, alkenylene or heteroalkylene group.

7. The complex according to embodiment 4 or embodiment 5, wherein the at least one $R_3$ group which is a polyalkylene glycol is bound directly to the nitrogen atom to which it is attached.

8. The complex of any one of the preceding embodiments, wherein the or each oligopeptide comprises from 3 to 20 amino acid residues.

9. The complex of any one of the preceding embodiments, wherein the or each oligopeptide has a net positive charge at pH 7.

10. The complex of embodiment 9, wherein the or each oligopeptide comprises amino acid residues selected from the group consisting of lysine, arginine and histidine.

11. The complex of any one of the preceding embodiments, wherein the or each oligopeptide is a compound of Formula VII:

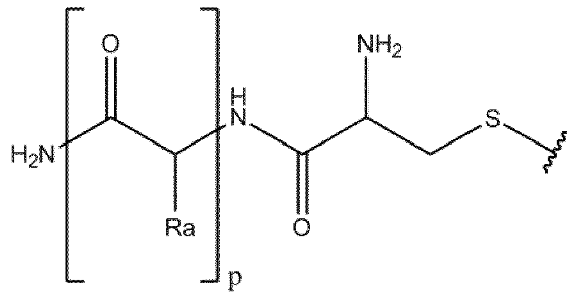

**Formula VII**

wherein p is an integer from 2 to 19 and wherein $R_a$ is selected at each occurrence from the group consisting of $H_2NC(=NH)-NH(CH_2)_3-$, $H_2N(CH_2)_4-$ or (1*H*-imidazol-4-yl)-$CH_2$-.

12. The complex of any one of the preceding embodiments, wherein $R_1$ and $R_2$ are both oligopeptides.

13. The complex of embodiment 12, wherein $R_1$ and $R_2$ are different oligopeptides.

14. The complex of any one of embodiments 1-11, wherein one of $R_1$ and $R_2$ is an oligopeptide and one of $R_1$ and $R_2$ is $R_y$.

15. The complex of any one of the preceding embodiments, wherein n is from 10 to 700, or from 20 to 500.

16. The complex of any one of the preceding embodiments, wherein $R_y$ is selected from a group consisting of hydrogen, $-(CH_2)_mNH_2$, $-(CH_2)_mNHMe$, $-(CH_2)_mOH$, $-(CH_2)_mCH_3$, $-(CH_2)_2(OCH_2CH_2)_mNH_2$, $-(CH_2)_2(OCH_2CH_2)_mOH$ or $-(CH_2)_2(OCH_2CH_2)_mCH_3$ wherein m is an integer from 1 to 20.

17. The complex of any one of the preceding embodiments, wherein each $L_3$ is independently selected from the group consisting of $-C_{1-10}$ alkylene- $(S-S)_q-C_{1-10}$ alkylene-, wherein q is 0 or 1.

18. The complex of any one of the preceding embodiments, wherein each $R_3$ is independently selected from hydrogen, $C_{1-6}$ alkyl, $C_{1-6}$ alkenyl, $C_{1-6}$ alkynyl, $C_{1-6}$ hydroxyalkyl, hydroxyl, $C_{1-6}$ alkoxy, halogen, aryl, heterocyclic, heteroaryl, cyano, $-O_2C-C_{1-6}$alkyl, carbamoyl, $-CO_2H$, $-CO_2-C_{1-6}$alkyl, $C_{1-6}$ alkylthioether, thiol, ureido, and polyalkylene glycols, wherein said polyalkylene glycol is either bound directly to the nitrogen atom to which $R_3$ is attached or bound to the nitrogen atom to which $R_3$ is attached via a linker moiety, wherein said linker moiety is an alkylene, cycloalkylene, alkenylene, cycloalkenylene, heteroalkylene, heterocycloalkylene, arylene or heteroarylene group.

19. The complex of any one of the preceding embodiments, wherein $L_4$ is selected from $-N(R_3)-$ and/or wherein $L_3$ is selected from $C_{1-6}$ alkylene groups.

20. The complex of any one of the preceding embodiments, wherein at least one $R_3$ group is polyethylene glycol.

21. A composition comprising a virus-based therapeutic agent coated with polymeric material comprising or consisting of polymer(s) of **Formula I** as defined in any one of embodiments 1 to 20.

22. The composition of embodiment 21, wherein the composition comprises nanoparticles containing the virus-based therapeutic agent coated with polymeric material comprising or consisting of polymer(s) of **Formula I** as defined in any one of embodiments 1 to 20.

23. The complex of any one of embodiments 1 to 20 or the composition of embodiments 21 or 22 wherein the virus-based therapeutic agent and the polymer(s) are non-covalently linked.

24. The complex of any one of embodiments 1 to 20 or 23 or the composition of any of embodiments 21 to 23, wherein the surface of said virus-based therapeutic agent comprises binding sites suitable for binding to a polymer of Formula I wherein the or each oligopeptide has a net positive charge at pH 7 .

25. The complex of any one of embodiments 1 to 20 or 23 to 24 or the composition of any of embodiments 21 to 24, wherein the virus-based therapeutic agent is selected from an adenoviral vector, an adeno-associated viral vector, a retroviral vector, a lentiviral vector, a herpex simplex viral vector, a vaccinia viral vector, a vesicular stomatitis viral vector, a reoviral vector, or a Semliki forest viral vector.

26. The complex or composition of embodiment 25, wherein the virus-based therapeutic agent is selected from an adenoviral vector, an adeno-associated viral vector, a retroviral vector, and a lentiviral vector, and preferably wherein the virus-based therapeutic agent is an adenoviral vector or adeno-associated viral vector.

27. A complex according to any one of embodiments 1 to 20 or 23 to 26 or a composition according to any one of embodiments 21 to 26 for use in medicine.

28. A complex according to any one of embodiments 1 to 20 or 23 to 26 or a composition according to any one of embodiments 21 to 26 for use in systemic viral gene therapy, particularly in the treatment of cancer, particularly liver cancer or pancreatic cancer.

29. A method of encapsulating a complex of a virus-based therapeutic agent and one or more polymers of Formula I according to any one of embodiments 1 to 20 to form nanoparticles, the method comprising the steps of: providing a virus-based therapeutic agent; providing the polymer(s) of Formula (I); and contacting the virus-based therapeutic agent and the polymer(s) under suitable conditions to form nanoparticles.

## Claims

1. A complex of a virus-based therapeutic agent with a polymer of formula I:

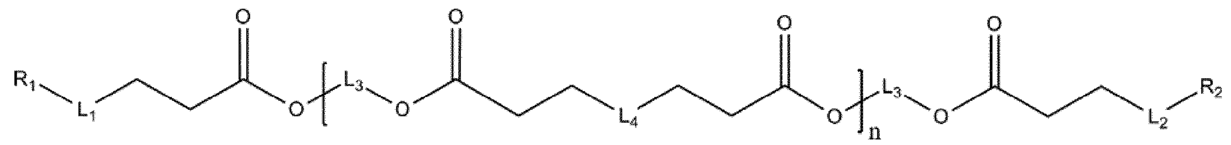

**Formula I**

wherein

each $L_1$ and $L_2$ is independently selected from the group consisting of:

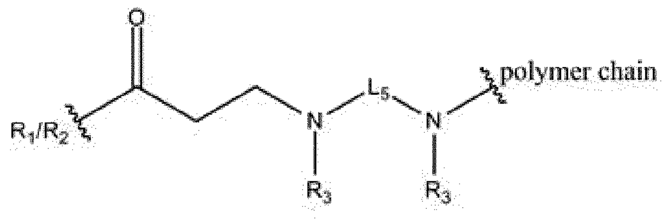

,

O, S, $NR_x$ and a bond; wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl; $L_3$ is independently selected from the group consisting of alkylene, alkenylene, heteroalkylene, heteroalkenylene, arylene or heteroarylene; or at least one occurrence of $L_3$ is

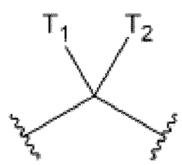

,

wherein $T_1$ is

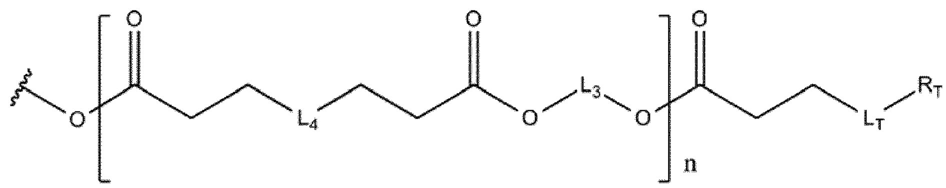

and $T_2$ is selected from H, alkyl or

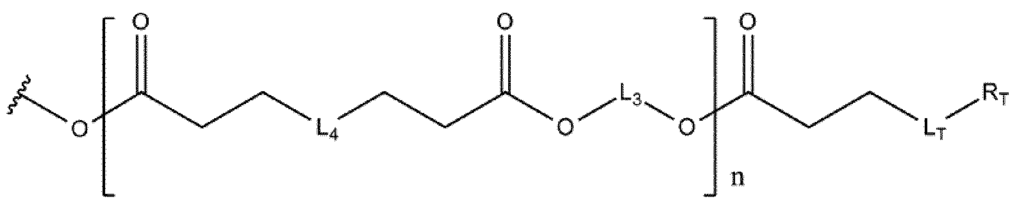

;

wherein $L_T$ is independently selected from the group consisting of:

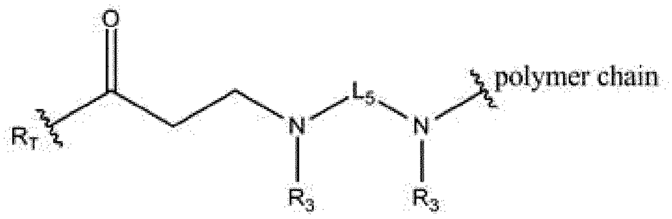

,

O, S, $NR_x$ and a bond, wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl, and the remaining $L_3$ groups are independently selected at each occurrence from the group consisting of alkylene, alkenylene, heteroalkylene, heteroalkenylene, arylene or heteroarylene;

$L_4$ is selected from the group consisting of

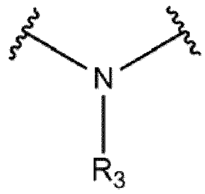 and 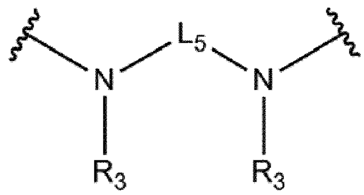 ;

L5 is independently selected from the group consisting of alkylene, alkenylene, heteroalkylene, heteroalkenylene, arylene or heteroarylene;

$R_1$ and $R_2$ and $R_T$ (if present) are independently selected from an oligopeptide and $R_y$; wherein at least one of $R_1$ and $R_2$ and $R_T$ (if present) is an oligopeptide;

and wherein $R_y$ is selected from the group consisting of hydrogen, halogen, alkyl, cycloalkyl, alkenyl, cycloalkenyl, heteroalkyl, heterocycloalkyl, acyl, aryl or heteroaryl; each $R_3$ is independently selected from the group consisting of hydrogen, halogen, alkyl, cycloalkyl, alkenyl, cycloalkenyl, heteroalkyl, heterocycloalkyl, acyl, aryl, heteroaryl and polyalkylene glycols, wherein said polyalkylene glycol is either bound directly to the nitrogen atom to which $R_3$ is attached or bound to the nitrogen atom to which $R_3$ is attached via a linker moiety, wherein said linker moiety is an alkylene, cycloalkylene, alkenylene, cycloalkenylene, heteroalkylene, heterocycloalkylene, arylene or heteroarylene group; and

n is an integer from 5 to 1,000;

or a pharmaceutically acceptable salt thereof.

**2.** The complex according to claim 1, wherein (i) $L_3$ is independently selected from the group consisting of alkylene, alkenylene, heteroalkylene, heteroalkenylene, arylene or heteroarylene; or (ii) at least one occurrence of $L_3$ is

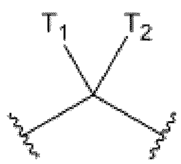

,

wherein $T_1$ is

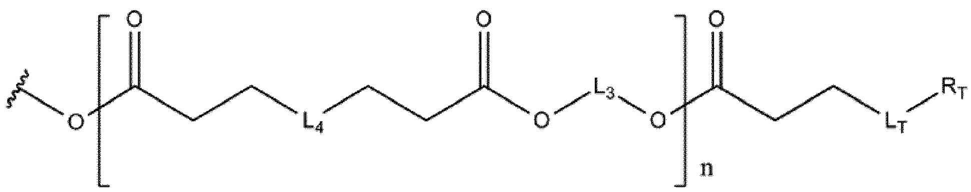

and $T_2$ is selected from H, alkyl or

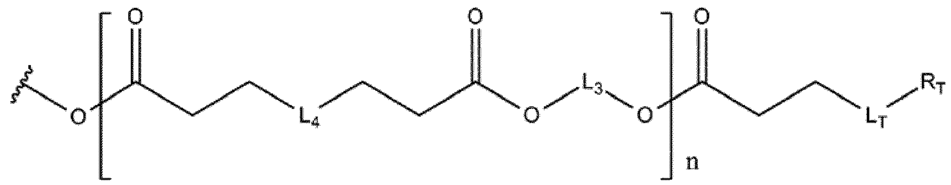

;

wherein $L_T$ is independently selected from the group consisting of:

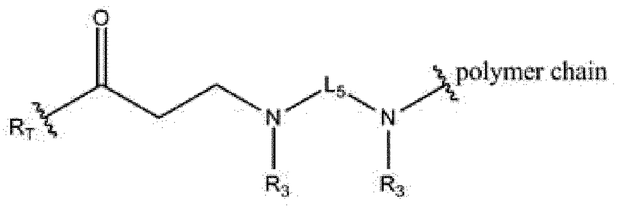

,

O, S, $NR_x$ and a bond; wherein $R_x$ is independently selected from the group consisting of hydrogen, halogen, alkyl, cycloalkyl, alkenyl, cycloalkenyl, heteroalkyl, heterocycloalkyl, acyl, aryl or heteroaryl, and the remaining $L_3$ groups are independently selected at each occurrence from the group consisting of alkylene, alkenylene, heteroalkylene, heteroalkenylene, arylene or heteroarylene.

**3.** The complex according to any one of claims 1 to 2, wherein at least one $R_3$ group is a polyalkylene glycol, preferably a polyethylene glycol; for example, wherein the at least one $R_3$ group which is a polyalkylene glycol is bound to the nitrogen atom of an $L_4$ group either directly or through a linker moiety.

**4.** The complex according to claim 3, wherein (i) the at least one $R_3$ group which is a polyalkylene glycol is bound to the nitrogen atom to which it is attached through a linker moiety which is an alkylene, alkenylene or heteroalkylene group; or (ii) the at least one $R_3$ group which is a polyalkylene glycol is bound directly to the nitrogen atom to which it is attached.

**5.** The complex of any one of the preceding claims, wherein the or each oligopeptide comprises from 3 to 20 amino acid residues; and/or wherein the or each oligopeptide has a net positive charge at pH 7, for example, wherein the or each oligopeptide comprises amino acid residues selected from the group consisting of lysine, arginine and histidine.

**6.** The complex of any one of the preceding claims, wherein the or each oligopeptide is a compound of Formula VII:

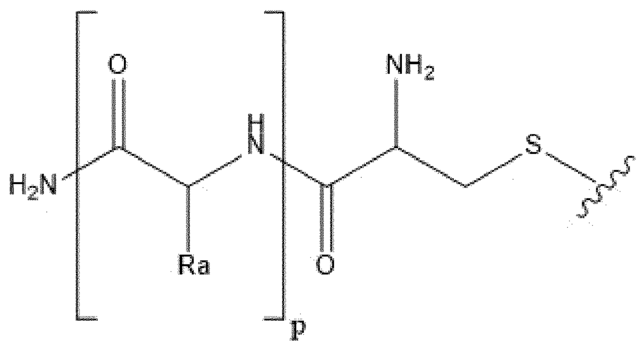

**Formula VII**

wherein p is an integer from 2 to 19 and wherein $R_a$ is selected at each occurrence from the group consisting of $H_2NC(=NH)-NH(CH_2)_3-$, $H_2N(CH_2)_4-$ or (1*H*-imidazol-4-yl)-$CH_2$-.

**7.** The complex of any one of the preceding claims, wherein $R_1$ and $R_2$ are both oligopeptides; for example, wherein $R_1$ and $R_2$ are different oligopeptides.

**8.** The complex of any one of claims 1-6, wherein one of $R_1$ and $R_2$ is an oligopeptide and one of $R_1$ and $R_2$ is $R_y$.

**9.** The complex of any one of the preceding claims, wherein n is from 10 to 700, or from 20 to 500; and/or wherein $R_y$ is selected from a group consisting of hydrogen, - $(CH_2)_mNH_2$, $-(CH_2)_mNHMe$, $-(CH_2)_mOH$, $-(CH_2)_mCH_3$, $-(CH_2)_2(OCH_2CH_2)_mNH_2$, - $(CH_2)_2(OCH_2CH_2)_mOH$ or $-(CH_2)_2(OCH_2CH_2)_mCH_3$ wherein m is an integer from 1 to 20; and/or wherein each $L_3$ is independently selected from the group consisting of $-C_1$-$_{10}$ alkylene- $(S\text{-}S)_q$-$C_{1\text{-}10}$ alkylene-, wherein q is 0 or 1.

**10.** The complex of any one of the preceding claims, wherein each $R_3$ is independently selected from hydrogen, $C_{1\text{-}6}$ alkyl, $C_{1\text{-}6}$ alkenyl, $C_{1\text{-}6}$ alkynyl, $C_{1\text{-}6}$ hydroxyalkyl, hydroxyl, $C_{1\text{-}6}$ alkoxy, halogen, aryl, heterocyclic, heteroaryl, cyano, $-O_2C\text{-}C_{1\text{-}6}$alkyl, carbamoyl, $-CO_2H$, $-CO_2\text{-}C_{1\text{-}6}$alkyl, $C_{1\text{-}6}$ alkylthioether, thiol, ureido, and polyalkylene glycols, wherein said polyalkylene glycol is either bound directly to the nitrogen atom to which $R_3$ is attached or bound to the nitrogen atom to which $R_3$ is attached via a linker moiety, wherein said linker moiety is an alkylene, cycloalkylene, alkenylene, cycloalkenylene, heteroalkylene, heterocycloalkylene, arylene or heteroarylene group; and/or wherein $L_4$ is selected from $-N(R_3)$- and/or wherein $L_3$ is selected from $C_{1\text{-}6}$ alkylene groups; and/or wherein at least one $R_3$ group is polyethylene glycol.

**11.** A composition comprising a virus-based therapeutic agent coated with polymeric material comprising or consisting of polymer(s) of **Formula I** as defined in any one of claims 1 to 10.

**12.** The composition of claim 11, wherein the composition comprises nanoparticles containing the virus-based therapeutic agent coated with polymeric material comprising or consisting of polymer(s) of **Formula I** as defined in any one of claims 1 to 10.

**13.** The complex of any one of claims 1 to 10 or the composition of claims 11 or 12 wherein the virus-based therapeutic agent and the polymer(s) are non-covalently linked.

**14.** The complex of any one of claims 1 to 10 or 13 or the composition of any of claims 11 to 13, wherein the surface of said virus-based therapeutic agent comprises binding sites suitable for binding to a polymer of Formula I wherein the or each oligopeptide has a net positive charge at pH 7 .

**15.** The complex of any one of claims 1 to 10 or 13 to 14 or the composition of any of claims 11 to 14, wherein the virus-based therapeutic agent is selected from an adenoviral vector, an adeno-associated viral vector, a retroviral vector, a lentiviral vector, a herpex simplex viral vector, a vaccinia viral vector, a vesicular stomatitis viral vector, a reoviral vector, or a Semliki forest viral vector; for example, wherein the virus-based therapeutic agent is selected from an adenoviral vector, an adeno-associated viral vector, a retroviral vector, and a lentiviral vector, and preferably wherein the virus-based therapeutic agent is an adenoviral vector or adeno-associated viral vector.

**16.** A complex according to any one of claims 1 to 10 or 13 to 15 or a composition according to any one of claims 11 to 15 for use in medicine; or a complex according to any one of claims 1 to 10 or 13 to 15 or a composition according to any one of claims 11 to 15 for use in systemic viral gene therapy, particularly in the treatment of cancer, particularly liver cancer or pancreatic cancer.

**17.** A method of encapsulating a complex of a virus-based therapeutic agent and one or more polymers of Formula I according to any one of claims 1 to 10 to form nanoparticles, the method comprising the steps of: providing a virus-based therapeutic agent; providing the polymer(s) of Formula (I); and contacting the virus-based therapeutic agent and the polymer(s) under suitable conditions to form nanoparticles.

**Patentansprüche**

**1.** Komplex eines virusbasierten therapeutischen Mittels mit einem Polymer der Formel I:

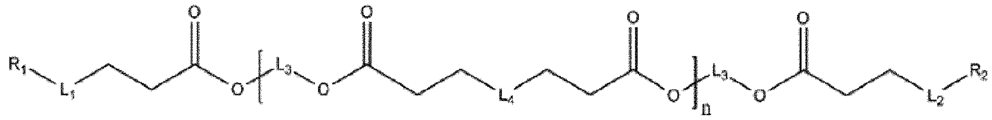

**Formel I**

wobei

$L_1$ und $L_2$ jeweils unabhängig aus der Gruppe ausgewählt sind, bestehend aus:

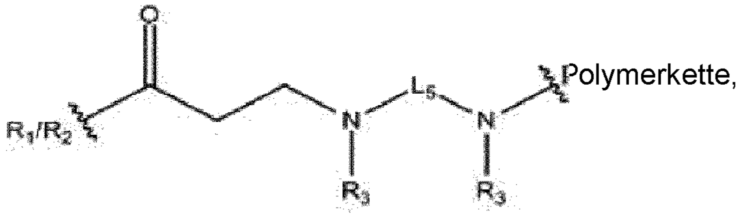

O, S, $NR_x$ und einer Bindung, wobei

$R_x$ unabhängig aus der Gruppe ausgewählt ist, die aus Wasserstoff, Halogen, Alkyl, Cycloalkyl, Alkenyl, Cycloalkenyl, Heteroalkyl, Heterocycloalkyl, Acyl, Aryl oder Heteroaryl besteht,

$L_3$ unabhängig aus der Gruppe ausgewählt ist, die aus Alkylen, Alkenylen, Heteroalkylen, Heteroalkenylen, Arylen oder Heteroarylen besteht, oder mindestens ein Vorkommen von $L_3$

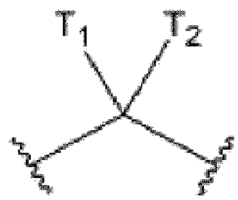

ist,

wobei $T_1$

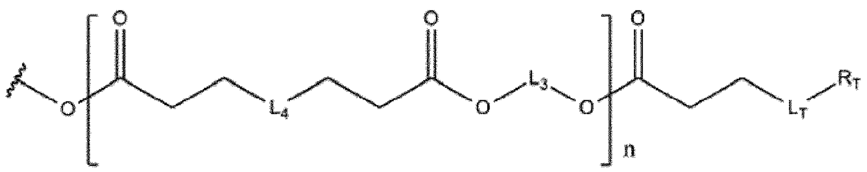

ist

und $T_2$ aus H, Alkyl oder

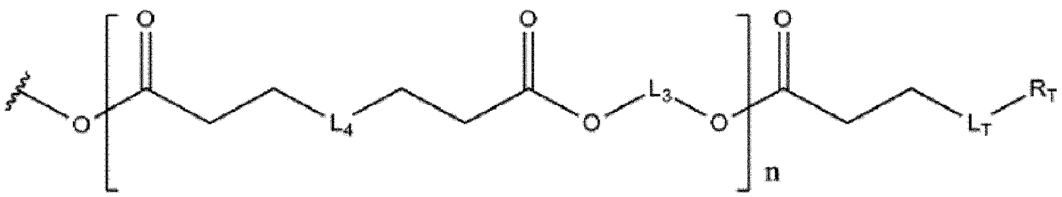

ausgewählt ist,

wobei $L_T$ unabhängig aus der Gruppe ausgewählt ist, bestehend aus:

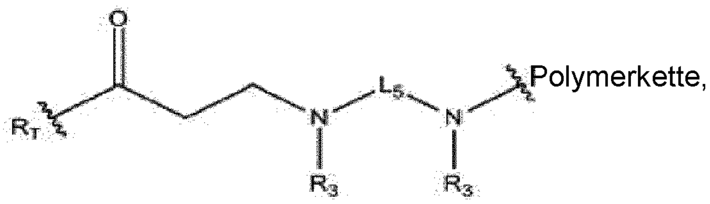

O, S, $NR_x$ und einer Bindung, wobei $R_x$ unabhängig aus der Gruppe ausgewählt ist, die aus Wasserstoff, Halogen, Alkyl, Cycloalkyl, Alkenyl, Cycloalkenyl, Heteroalkyl, Heterocycloalkyl, Acyl, Aryl oder Heteroaryl besteht, und die übrigen $L_3$-Gruppen bei jedem Vorkommen unabhängig aus der Gruppe ausgewählt sind, die aus Alkylen, Alkenylen, Heteroalkylen, Heteroalkenylen, Arylen oder Heteroarylen besteht,
$L_4$ aus der Gruppe ausgewählt ist, bestehend aus

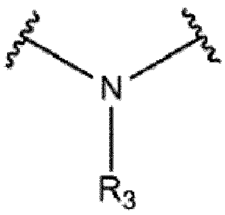 und 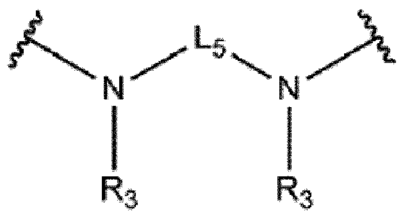 ,

$L_5$ unabhängig aus der Gruppe ausgewählt ist, die aus Alkylen, Alkenylen, Heteroalkylen, Heteroalkenylen, Arylen oder Heteroarylen besteht,
$R_1$ und $R_2$ und $R_T$ (sofern vorliegend) unabhängig aus einem Oligopeptid und $R_y$ ausgewählt sind,
wobei mindestens eines von $R_1$ und $R_2$ und $R_T$ (sofern vorliegend) ein Oligopeptid ist, und wobei $R_y$ aus der Gruppe ausgewählt ist, die aus Wasserstoff, Halogen, Alkyl, Cycloalkyl, Alkenyl, Cycloalkenyl, Heteroalkyl, Heterocycloalkyl, Acyl, Aryl oder Heteroaryl besteht,
jedes $R_3$ unabhängig aus der Gruppe ausgewählt ist, die aus Wasserstoff, Halogen, Alkyl, Cycloalkyl, Alkenyl, Cycloalkenyl, Heteroalkyl, Heterocycloalkyl, Acyl, Aryl, Heteroaryl und Polyalkylenglykolen besteht, wobei das Polyalkylenglykol entweder direkt an das Stickstoffatom gebunden ist, an welches $R_3$ angehängt ist, oder an das Stickstoffatom, an welches $R_3$ angehängt ist, über eine Linkereinheit gebunden ist, wobei die Linkereinheit eine Alkylen-, Cycloalkylen-, Alkenylen-, Cycloalkenylen-, Heteroalkylen-, Heterocycloalkylen-, Arylen- oder Heteroarylen-Gruppe ist, und
n eine ganze Zahl von 5 bis 1.000 ist,

oder ein pharmazeutisch akzeptables Salz davon.

**2.** Komplex nach Anspruch 1, wobei (i) $L_3$ unabhängig aus der Gruppe ausgewählt ist, die aus Alkylen, Alkenylen, Heteroalkylen, Heteroalkenylen, Arylen oder Heteroarylen besteht, oder (ii) mindestens ein Vorkommen von $L_3$

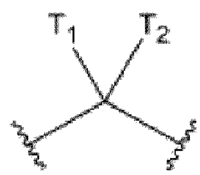 ist,

wobei $T_1$

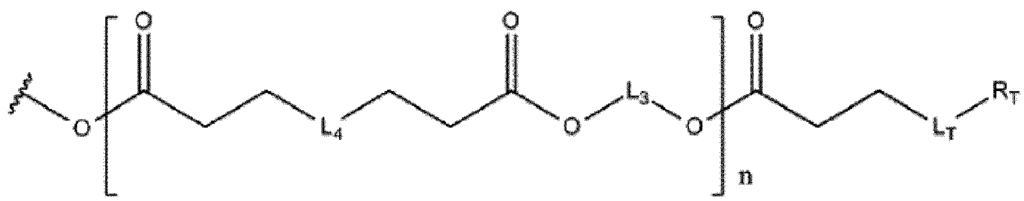

ist
und $T_2$ aus H, Alkyl oder

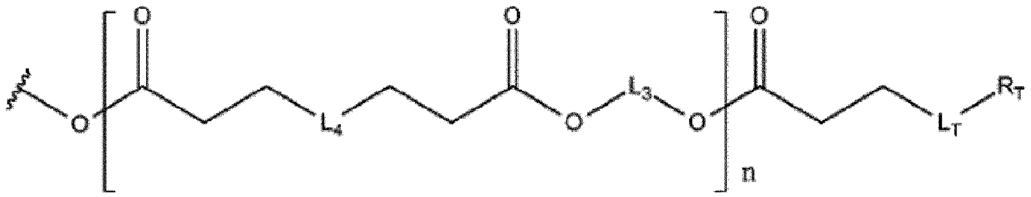

ausgewählt ist,
wobei $L_T$ unabhängig aus der Gruppe ausgewählt ist, bestehend aus:

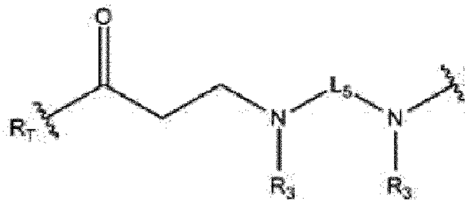 Polymerkette,

O, S, $NR_x$ und einer Bindung, wobei $R_x$ unabhängig aus der Gruppe ausgewählt ist, die aus Wasserstoff, Halogen, Alkyl, Cycloalkyl, Alkenyl, Cycloalkenyl, Heteroalkyl, Heterocycloalkyl, Acyl, Aryl oder Heteroaryl besteht, und die übrigen $L_3$-Gruppen bei jedem Vorkommen unabhängig aus der Gruppe ausgewählt sind, die aus Alkylen, Alkenylen, Heteroalkylen, Heteroalkenylen, Arylen oder Heteroarylen besteht.

**3.** Komplex nach einem der Ansprüche 1 bis 2, wobei mindestens eine $R_3$-Gruppe ein Polyalkylenglykol, zum Beispiel vorzugsweise ein Polyethylenglykol, ist, wobei die mindestens eine $R_3$-Gruppe, die ein Polyalkylenglykol ist, an das Stickstoffatom einer $L_4$-Gruppe entweder direkt oder durch eine Linkereinheit gebunden ist.

**4.** Komplex nach Anspruch 3, wobei (i) die mindestens eine $R_3$-Gruppe, die ein Polyalkylenglykol ist, an das Stickstoffatom gebunden ist, an welches sie durch eine Linkereinheit angehängt ist, die eine Alkylen-, Alkenylen- oder Heteroalkylen-Gruppe ist, oder (ii) die mindestens eine $R_3$-Gruppe, die ein Polyalkylenglykol ist, direkt an das Stickstoffatom gebunden ist, an welches sie angehängt ist.

**5.** Komplex nach einem der vorhergehenden Ansprüche, wobei das oder jedes Oligopeptid 3 bis 20 Aminosäurereste umfasst, und/oder wobei das oder jedes Oligopeptid zum Beispiel eine positive Nettoladung bei einem pH von 7 aufweist, wobei das oder jedes Oligopeptid Aminosäurereste umfasst, die aus der Gruppe ausgewählt sind, die aus Lysin, Arginin und Histidin besteht.

**6.** Komplex nach einem der vorhergehenden Ansprüche, wobei das oder jedes Oligopeptid eine Verbindung der Formel VII ist:

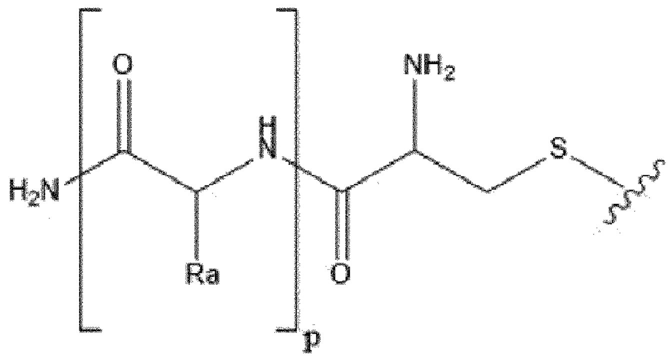

**Formel VII**

wobei p eine ganze Zahl von 2 bis 19 ist, und wobei $R_a$ bei jedem Vorkommen aus der Gruppe ausgewählt ist, die aus $H_2NC(=NH)-NH(CH_2)_3-$, $H_2N(CH_2)_4-$ oder (1H-imidazol-4-yl)-$CH_2$- besteht.

7. Komplex nach einem der vorhergehenden Ansprüche, wobei $R_1$ und $R_2$ beide Oligopeptide sind, wobei $R_1$ und $R_2$ zum Beispiel unterschiedliche Oligopeptide sind.

8. Komplex nach einem der Ansprüche 1 - 6, wobei eines von $R_1$ und $R_2$ ein Oligopeptid ist und eines von $R_1$ und $R_2$ $R_y$ ist.

9. Komplex nach einem der vorhergehenden Ansprüche, wobei n 10 bis 700, oder 20 bis 500 ist, und/oder wobei $R_y$ aus einer Gruppe ausgewählt ist, die aus Wasserstoff, $-(CH_2)_mNH_2$, $-(CH_2)_mNHMe$, $-(CH_2)_mOH$, $-(CH_2)_mCH_3$, $-(CH_2)_2(OCH_2CH_2)_mNH_2$, $-(CH_2)_2(OCH_2CH_2)_mOH$ oder $-(CH_2)_2(OCH_2CH_2)_mCH_3$ besteht, wobei m eine ganze Zahl von 1 bis 20 ist, und/oder wobei jedes $L_3$ unabhängig aus der Gruppe ausgewählt ist, die aus $-C_{1-10}$ Alkylen-(S-S)$_q$-$C_{1-10}$-Alkylen- besteht, wobei q 0 oder 1 ist.

10. Komplex nach einem der vorhergehenden Ansprüche, wobei jedes $R_3$ unabhängig ausgewählt ist aus Wasserstoff, $C_{1-6}$-Alkyl, $C_{1-6}$-Alkenyl, $C_{1-6}$-Alkynyl, $C_{1-6}$-Hydroxyalkyl, Hydroxyl, $C_{1-4}$-Alkoxy, Halogen, Aryl, Heterocycloalkyl, Heteroaryl, Cyano, $-O_2C-C_{1-6}$-Alkyl, Carbamoyl, $-CO_2H$, $-CO_2-C_{1-6}$ -Alkyl, $C_{1-6}$-Alkylthioether, Thiol, Ureido, und Polyalkylenglykolen, wobei das Polyalkylenglykol entweder direkt an das Stickstoffatom gebunden ist, an welches $R_3$ angehängt ist, oder an das Stickstoffatom, an welches $R_3$ angehängt ist, über eine Linkereinheit gebunden ist, wobei die Linkereinheit eine Alkylen-, Cycloalkylen-, Alkenylen-, Cycloalkenylen-, Heteroalkylen-, Heterocycloalkylen-, Arylen- oder Heteroarylen-Gruppe ist, und/oder wobei $L_4$ aus $-N(R_3)-$ ausgewählt ist und/oder wobei $L_3$ aus $C_{1-6}$-Alkylengruppen ausgewählt ist, und/oder wobei mindestens eine $R_3$-Gruppe Polyethylenglykol ist.

11. Zusammensetzung, umfassend ein virusbasiertes therapeutisches Mittel, das mit Polymermaterial beschichtet ist, das ein oder mehrere Polymere der Formel I umfasst oder daraus besteht, die in einem der Ansprüche 1 bis 10 definiert sind.

12. Zusammensetzung nach Anspruch 11, wobei die Zusammensetzung Nanopartikel umfasst, die das virusbasierte therapeutische Mittel enthalten, das mit Polymermaterial beschichtet ist, das ein oder mehrere Polymere der **Formel I** umfasst oder daraus besteht, die in einem der Ansprüche 1 bis 10 definiert sind.

13. Komplex nach einem der Ansprüche 1 bis 10 oder Zusammensetzung nach den Ansprüchen 11 oder 12, wobei das virusbasierte therapeutische Mittel und das oder die Polymere nicht kovalent verknüpft sind.

14. Komplex nach einem der Ansprüche 1 bis 10 oder 13 oder Zusammensetzung nach einem der Ansprüche 11 bis 13, wobei die Oberfläche des virusbasierten therapeutischen Mittels Bindungsstellen umfasst, die zur Bindung an ein Polymer der Formel I geeignet sind, wobei das oder jedes Oligopeptid eine positive Nettoladung bei einem pH von 7 aufweist.

15. Komplex nach einem der Ansprüche 1 bis 10 oder 13 bis 14 oder Zusammensetzung nach einem der Ansprüche 11 bis 14, wobei das virusbasierte therapeutische Mittel ausgewählt ist aus einem adenoviralen Vektor, einem adenoassoziierten viralen Vektor, einem retroviralen Vektor, einem lentiviralen Vektor, einem viralen Herpes simplex-Vektor, einem viralen Vaccinia-Vektor, einem viralen vesikulären Stomatitis-Vektor, einem reoviralen Vektor oder einem viralen Semliki-Forest-Vektor, wobei zum Beispiel das virusbasierte therapeutische Mittel aus einem adenoviralen Vektor, einem adenoassoziierten viralen Vektor, einem retroviralen Vektor und einem lentiviralen Vektor ausgewählt ist, und wobei das virusbasierte therapeutische Mittel vorzugsweise ein adenoviraler Vektor oder ein adenoassoziierter viraler Vektor ist.

**16.** Komplex nach einem der Ansprüche 1 bis 10 oder 13 bis 15 oder Zusammensetzung nach einem der Ansprüche 11 bis 15 zur Verwendung in der Medizin, oder Komplex nach einem der Ansprüche 1 bis 10 oder 13 bis 15 oder Zusammensetzung nach einem der Ansprüche 11 bis 15 zur Verwendung in der systemischen viralen Gentherapie, insbesondere bei der Behandlung von Krebs, insbesondere Leberkrebs oder Pankreaskrebs.

**17.** Verfahren zur Verkapselung eines Komplexes eines virusbasierten therapeutischen Mittels und eines oder mehrerer Polymere der Formel I nach einem der Ansprüche 1 bis 10 zur Bildung von Nanopartikeln, wobei das Verfahren die Schritte umfasst des: Bereitstellens eines virusbasierten therapeutischen Mittels, Bereitstellens des oder der Polymere der Formel (I), und Inkontaktbringens des virusbasierten therapeutischen Mittels und des oder der Polymere unter Bedingungen, die zur Bildung von Nanopartikeln geeignet sind.

## Revendications

**1.** Complexe d'un agent thérapeutique à base de virus avec un polymère de formule I :

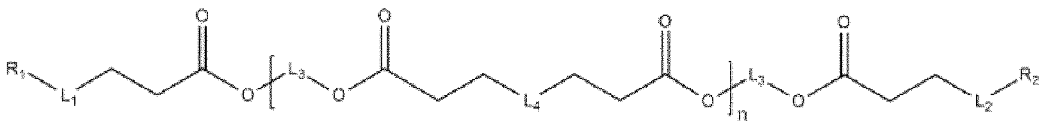

Formule I

dans lequel
$L_1$ et $L_2$ sont choisis chacun indépendamment dans le groupe consistant en

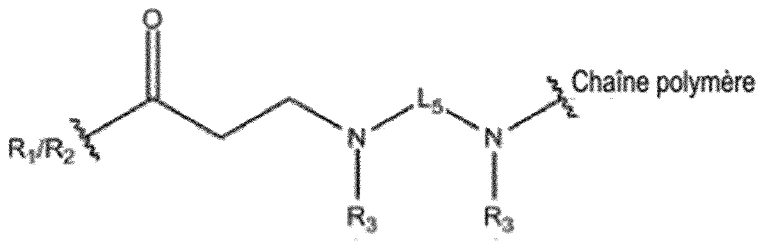

, O, S, $NR_x$ et une liaison ; dans lequel $R_x$ est indépendamment choisi dans le groupe consistant en l'hydrogène, un halogène, un alkyle, cycloalkyle, alcényle, cycloalcényle, hétéroalkyle, hétérocycloalkyle, acyle, aryle ou hétéroaryle ; $L_3$ est indépendamment choisi dans le groupe consistant en un alkylène, alcénylène, hétéroalkylène, hétéroalcénylène, arylène ou hétéroarylène ; ou
au moins une apparition de $L_3$ est

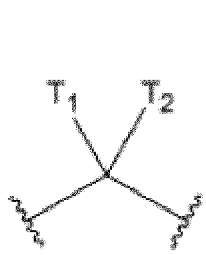

,

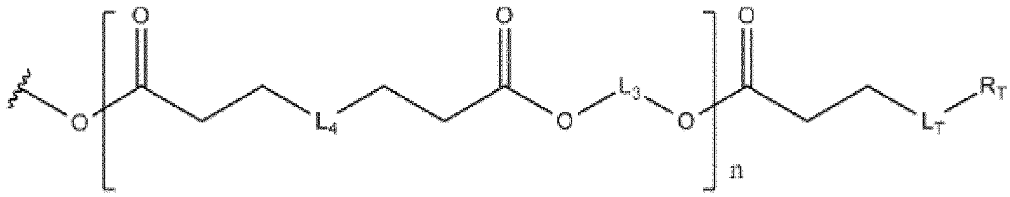

;

dans lequel $T_1$ est

et $T_2$ est choisi parmi H, un alkyle ou

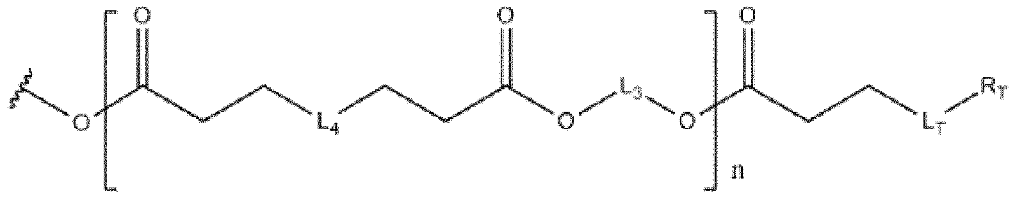

;

dans lequel $L_T$ est indépendamment choisi dans le groupe consistant en :

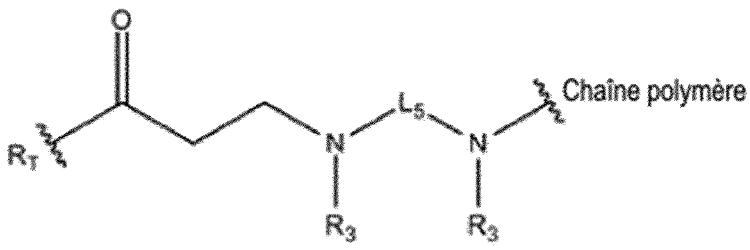

, O, S, $NR_x$ et une liaison, dans lequel $R_x$ est indépendamment choisi dans le groupe consistant en l'hydrogène, un halogène, un alkyle, cycloalkyle, alcényle, cycloalcényle, hétéroalkyle, hétérocycloalkyle, acyle, aryle ou hétéroaryle, et les groupes $L_3$ restants sont indépendamment choisis à chaque apparition dans le groupe consistant en un alkylène, alcénylène, hétéroalkylène, hétéroalcénylène, arylène ou hétéroarylène ;

$L_4$ est choisi dans le groupe consistant en

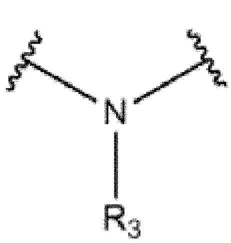 et 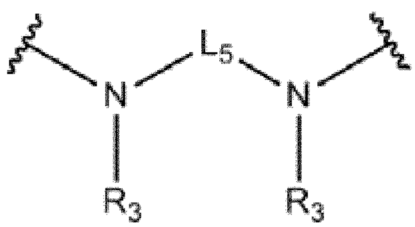 ;

$L_5$ est indépendamment choisi dans le groupe consistant en un alkylène, alcénylène, hétéroalkylène, hétéroalcénylène, arylène ou hétéroarylène ; $R_1$ et $R_2$ et $R_T$ (si présent) sont indépendamment choisis parmi un oligopeptide et $R_y$ ;

dans lequel au moins de $R_1$ et $R_2$ et $R_T$ (si présent) est un oligopeptide ;

et dans lequel $R_y$ est choisi dans le groupe consistant en l'hydrogène, un halogène, un alkyle, cycloalkyle, alcényle, cycloalcényle, hétéroalkyle, hétérocycloalkyle, acyle, aryle ou hétéroaryle ;

chaque $R_3$ est indépendamment choisi dans le groupe consistant en l'hydrogène, un halogène, un alkyle, cycloalkyle, alcényle, cycloalcényle, hétéroalkyle, hétérocycloalkyle, acyle, aryle, hétéroaryle et des polyalkylène glycols, dans lequel ledit polyalkylène glycol est soit lié directement à l'atome d'azote auquel $R_3$ est attaché soit lié à l'atome d'azote auquel $R_3$ est attaché via un fragment de liaison, dans lequel ledit fragment de liaison est un groupe alkylène, cycloalkylène, alcénylène, cycloalcénylène, hétéroalkylène, hétérocycloalkylène, arylène ou hétéroarylène ; et

n est un nombre entier de 5 à 1 000 ;

ou sel pharmaceutiquement acceptable de celui-ci.

**2.** Complexe selon la revendication 1, dans lequel (i) $L_3$ est indépendamment choisi dans le groupe consistant en un alkylène, alcénylène, hétéroalkylène, hétéroalcénylène, arylène ou hétéroarylène ; ou (ii) au moins une apparition de $L_3$ est

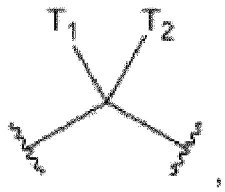

dans lequel $T_1$ est

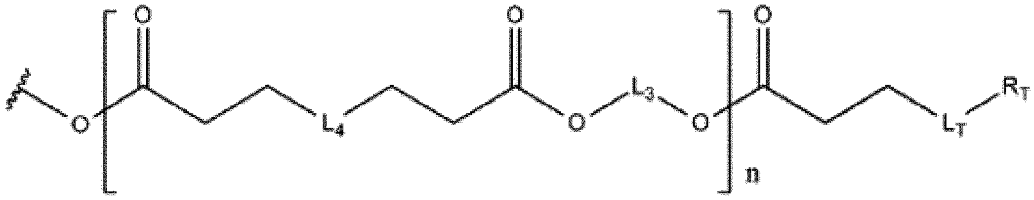

et $T_2$ est choisi parmi H, un alkyle ou

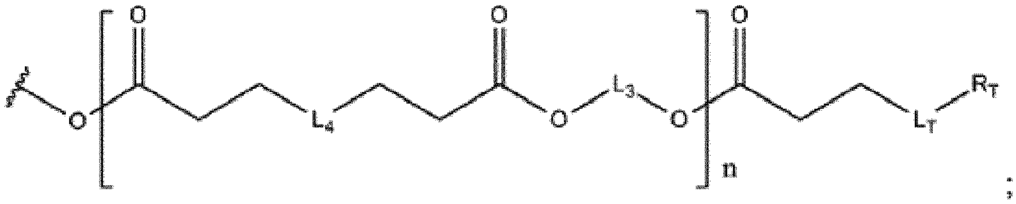

dans lequel $L_T$ est indépendamment choisi dans le groupe consistant en :

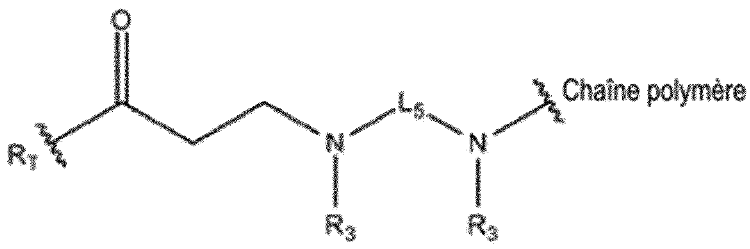

, O, S, $NR_x$ et une liaison ; dans lequel $R_x$ est indépendamment choisi dans le groupe consistant en l'hydrogène, un halogène, un alkyle, cycloalkyle, alcényle, cycloalcényle, hétéroalkyle, hétérocycloalkyle, acyle, aryle ou hétéroaryle, et les groupes $L_3$ restants sont indépendamment choisis à chaque apparition dans le groupe consistant en un alkylène, alcénylène, hétéroalkylène, hétéroalcénylène, arylène ou hétéroarylène.

**3.** Complexe selon l'une quelconque des revendications 1 à 2, dans lequel au moins un groupe $R_3$ est un polyalkylène glycol, de préférence un polyéthylène glycol ; par exemple, dans lequel le au moins un groupe $R_3$ qui est un polyalkylène glycol est lié à l'atome d'azote d'un groupe $L_4$ soit directement soit par l'intermédiaire d'un fragment de liaison.

**4.** Complexe selon la revendication 3, dans lequel (i) le au moins un groupe $R_3$ qui est un polyalkylène glycol est lié à l'atome d'azote auquel il est attaché par l'intermédiaire d'un fragment de liaison qui est un groupe alkylène, alcénylène ou hétéroalkylène ; ou (ii) le au moins un groupe $R_3$ qui est un polyalkylène glycol est lié directement à l'atome d'azote auquel il est attaché.

5. Complexe selon l'une quelconque des revendications précédentes, dans lequel le ou chaque oligopeptide comprend de 3 à 20 résidus d'acides aminés ; et/ou dans lequel le ou chaque oligopeptide présente une charge positive nette à pH 7, par exemple, dans lequel le ou chaque oligopeptide comprend des résidus d'acides aminés choisis dans le groupe consistant en lysine, arginine et histidine.

6. Complexe selon l'une quelconque des revendications précédentes, dans lequel le ou chaque oligopeptide est un composé de formule VII :

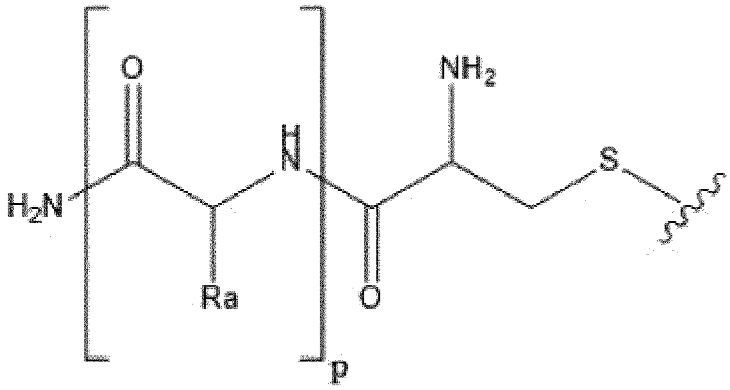

Formule VII

dans lequel p est un nombre entier de 2 à 19 et dans lequel $R_a$ est choisi à chaque apparition dans le groupe consistant en $H_2NC(=NH)-NH(CH_2)_3-$, $H_2N(CH_2)_4-$ ou (1*H*-imidazol-4-yl)-$CH_2-$.

7. Complexe selon l'une quelconque des revendications précédentes, dans lequel $R_1$ et $R_2$ sont tous les deux des oligopeptides ; par exemple, dans lequel $R_1$ et $R_2$ sont des oligopeptides différents.

8. Complexe selon l'une quelconque des revendications 1-6, dans lequel un de $R_1$ et $R_2$ est un oligopeptide et un de $R_1$ et $R_2$ est $R_y$.

9. Complexe selon l'une quelconque des revendications précédentes, dans lequel n est de 10 à 700, ou de 20 à 500 ; et/ou dans lequel $R_y$ est choisi dans un groupe consistant en l'hydrogène, $-(CH_2)_mNH_2$, $-(CH_2)_mNHMe$, $-(CH_2)_mOH$, $-(CH_2)_mCH_3$, $-(CH_2)_2(OCH_2CH_2)_mNH_2$, $-(CH_2)_2(OCH_2CH_2)_mOH$ ou $-(CH_2)_2(OCH_2CH_2)_mCH_3$ dans lequel m est un nombre entier de 1 à 20 ; et/ou dans lequel chaque $L_3$ est indépendamment choisi dans le groupe consistant en -alkylène en $C_{1-10}$-$(S-S)_q$-alkylène en $C_{1-10}$-, dans lequel q est égal à 0 ou 1.

10. Complexe selon l'une quelconque des revendications précédentes, dans lequel chaque $R_3$ est indépendamment choisi parmi l'hydrogène, un alkyle en $C_{1-6}$, alcényle en $C_{1-6}$, alcynyle en $C_{1-6}$, hydroxyalkyle en $C_{1-6}$, hydroxyle, alcoxy en $C_{1-6}$, halogène, aryle, hétérocyclique, hétéroaryle, cyano, $-O_2C$-alkyle en $C_{1-6}$, carbamoyle, $-CO_2H$, $-CO_2$-alkyle en $C_{1-6}$, alkylthioéther en $C_{1-6}$, thiol, uréido, et des polyalkylène glycols, dans lequel ledit polyalkylène glycol est soit lié directement à l'atome d'azote auquel $R_3$ est attaché soit lié à l'atome d'azote auquel $R_3$ est attaché via un fragment de liaison, dans lequel ledit fragment de liaison est un groupe alkylène, cycloalkylène, alcénylène, cycloalcénylène, hétéroalkylène, hétérocycloalkylène, arylène ou hétéroarylène ; et/ou dans lequel $L_4$ est choisi parmi $-N(R_3)-$ et/ou dans lequel $L_3$ est choisi parmi des groupes alkylène en $C_{1-6}$ ; et/ou dans lequel au moins un groupe $R_3$ est le polyéthylèneglycol.

11. Composition comprenant un agent thérapeutique à base de virus revêtu de matériau polymère comprenant ou consistant en un (des) polymère(s) de formule I comme défini dans l'une quelconque des revendications 1 à 10.

12. Composition selon la revendication 11, dans lequel la composition comprend des nanoparticules contenant l'agent thérapeutique à base de virus revêtu de matériau polymère comprenant ou consistant en un (des) polymère(s) de formule I comme défini dans l'une quelconque des revendications 1 à 10.

13. Complexe selon l'une quelconque des revendications 1 à 10 ou composition selon les revendications 11 ou 12, dans lequel l'agent thérapeutique à base de virus et le(les) polymère(s) sont liés de manière non covalente.

**14.** Complexe selon l'une quelconque des revendications 1 à 10 ou 13 ou composition selon l'une quelconque des revendications 11 à 13, dans lequel la surface dudit agent thérapeutique à base de virus comprend des sites de liaison appropriés pour une liaison à un polymère de formule I dans lequel le ou chaque oligopeptide présente une charge positive nette à pH 7.

**15.** Complexe selon l'une quelconque des revendications 1 à 10 ou 13 à 14 ou composition selon l'une quelconque des revendications 11 à 14, dans lequel l'agent thérapeutique à base de virus est choisi parmi un vecteur adénoviral, un vecteur viral adéno-associé, un vecteur rétroviral, un vecteur lentiviral, un vecteur viral de l'herpès simplex, un vecteur viral de vaccine, un vecteur viral de la stomatite vésiculeuse, un vecteur réoviral, ou un vecteur viral de la forêt de Semliki ; par exemple, dans lequel l'agent thérapeutique à base de virus est choisi parmi un vecteur adénoviral, un vecteur viral adéno-associé, un vecteur rétroviral, et un vecteur lentiviral, et de préférence dans lequel l'agent thérapeutique à base de virus est un vecteur adénoviral ou un vecteur viral adéno-associé.

**16.** Complexe selon l'une quelconque des revendications 1 à 10 ou 13 à 15 ou composition selon l'une quelconque des revendications 11 à 15 pour utilisation dans la médecine ; ou complexe selon l'une quelconque des revendications 1 à 10 ou 13 à 15 ou composition selon l'une quelconque des revendications 11 à 15 pour utilisation dans une thérapie génique virale systémique, particulièrement dans le traitement de cancer, particulièrement de cancer du foie ou de cancer pancréatique.

**17.** Procédé d'encapsulation d'un complexe d'un agent thérapeutique à base de virus et d'un ou plusieurs polymères de formule I selon l'une quelconque des revendications 1 à 10 pour former des nanoparticules, le procédé comprenant les étapes de : fourniture d'un agent thérapeutique à base de virus ; fourniture du(des) polymère(s) de formule (I) ; et mise en contact de l'agent thérapeutique à base de virus et du(des) polymère(s) dans des conditions appropriées pour former des nanoparticules.

FIGURE 1

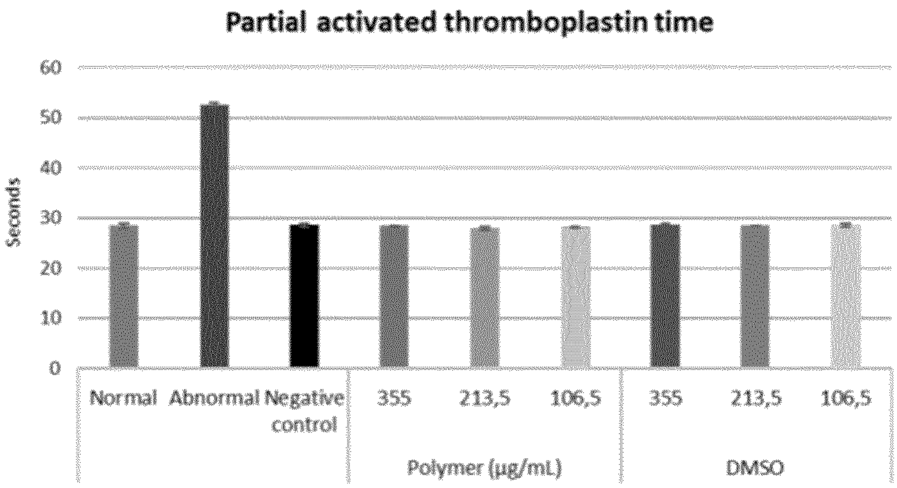
Partial activated thromboplastin time
Seconds
60
50
40
30
20
10
0
Normal
Abnormal
Negative control
355
213,5
106,5
355
213,5
106,5
Polymer (µg/mL)
DMSO

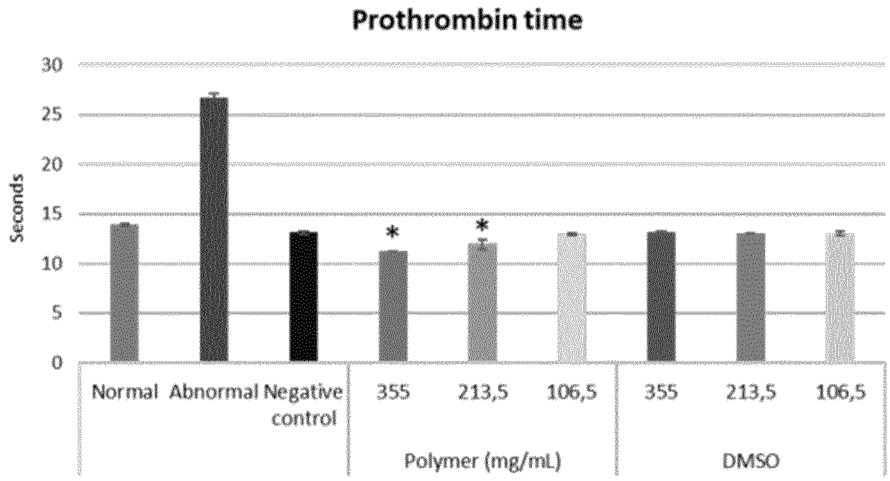
Prothrombin time
Seconds
30
25
20
15
10
5
0
*
*
Normal
Abnormal
Negative control
355
213,5
106,5
355
213,5
106,5
Polymer (mg/mL)
DMSO

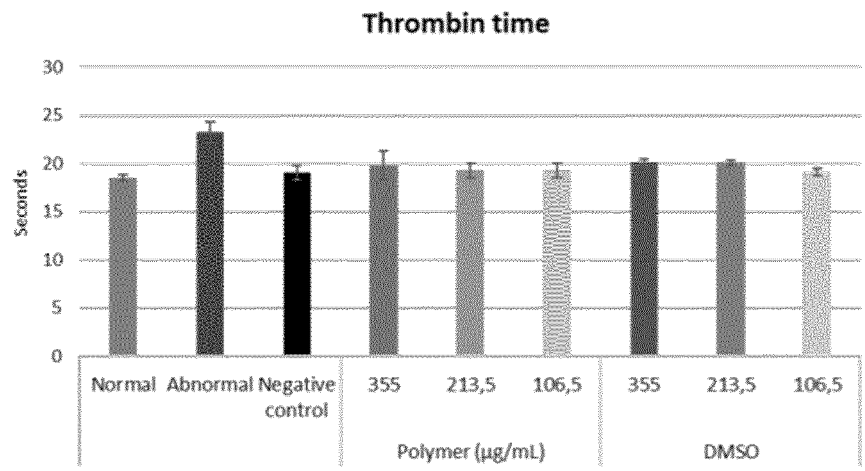
Thrombin time
Seconds
30
25
20
15
10
5
0
Normal
Abnormal
Negative control
355
213,5
106,5
355
213,5
106,5
Polymer (µg/mL)
DMSO

**FIGURE 2**

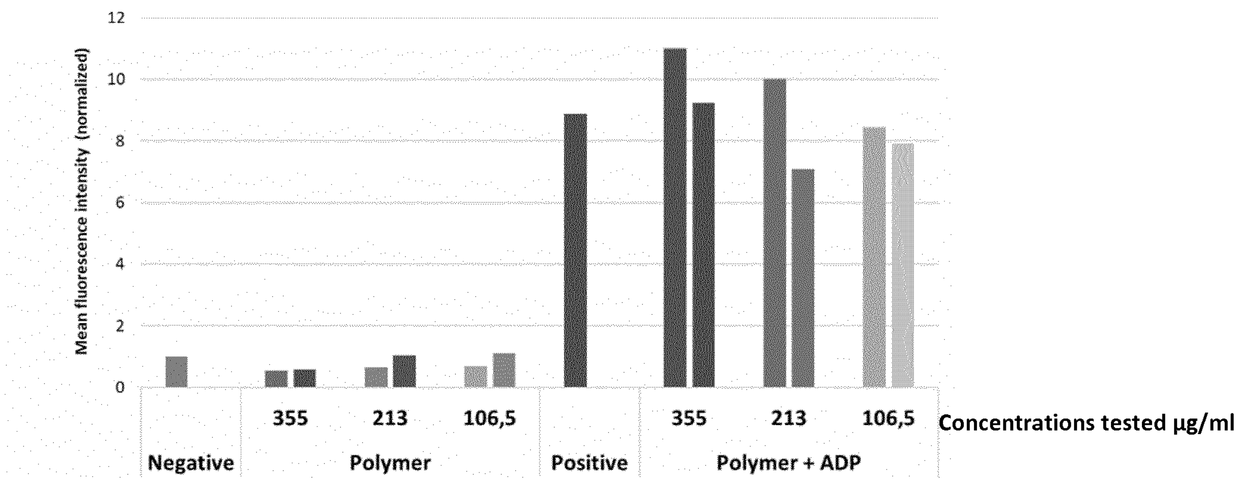
Mean fluorescence intensity (normalized)
12
10
8
6
4
2
0
355
213
106,5
355
213
106,5
Concentrations tested µg/ml
Negative
Polymer
Positive
Polymer + ADP

FIGURE 3

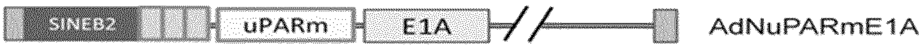


FIGURE 4

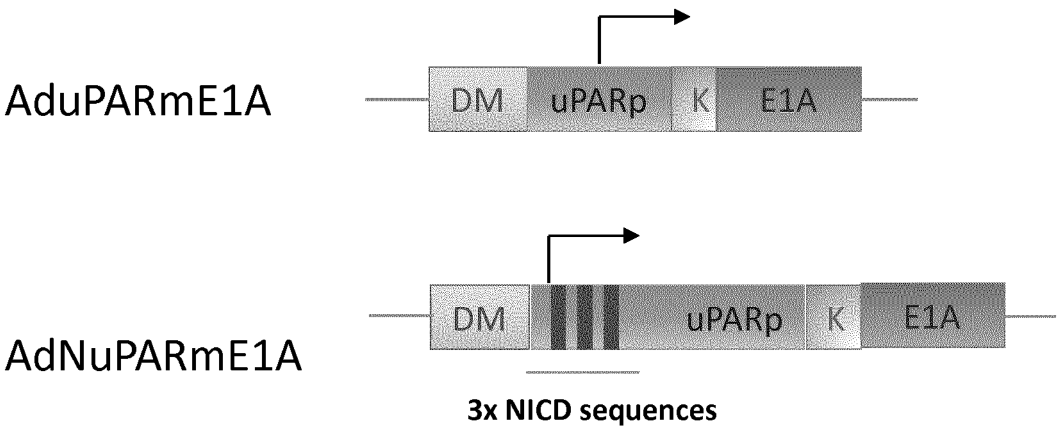

**FIGURE 5**

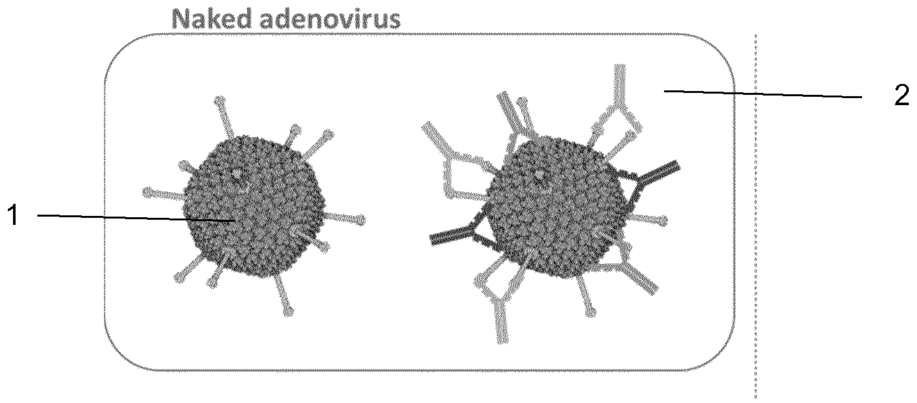


**FIGURE 6**

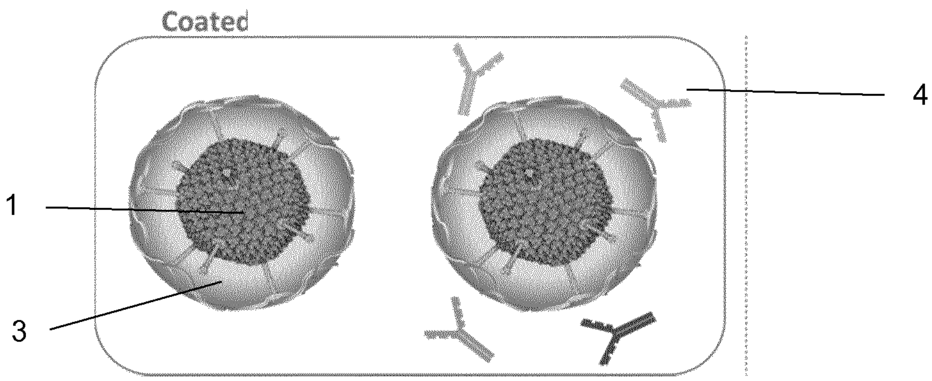

**FIGURE 7**

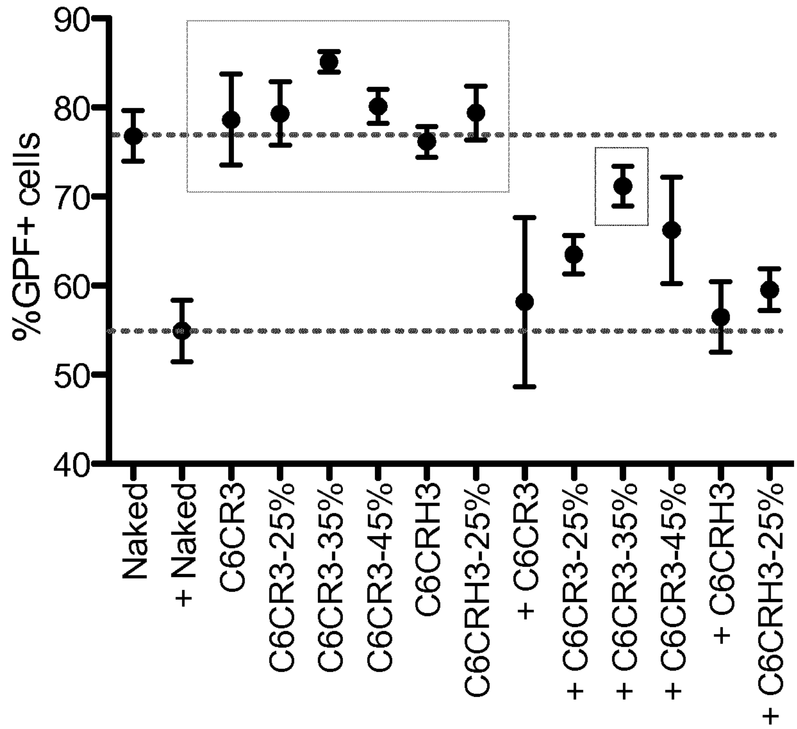
90
80
70
60
50
40
%GPF+ cells
Naked
+ Naked
C6CR3
C6CR3-25%
C6CR3-35%
C6CR3-45%
C6CRH3
C6CRH3-25%
+ C6CR3
+ C6CR3-25%
+ C6CR3-35%
+ C6CR3-45%
+ C6CRH3
+ C6CRH3-25%

## FIGURE 8

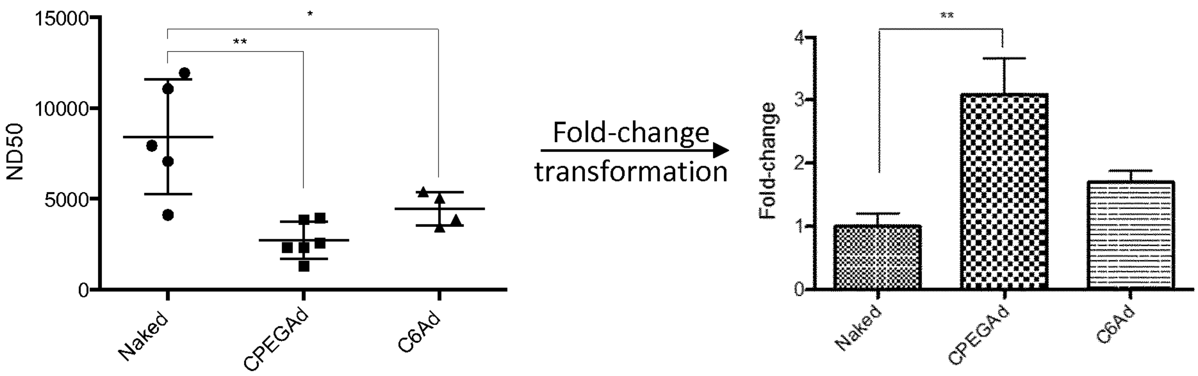


## FIGURE 9

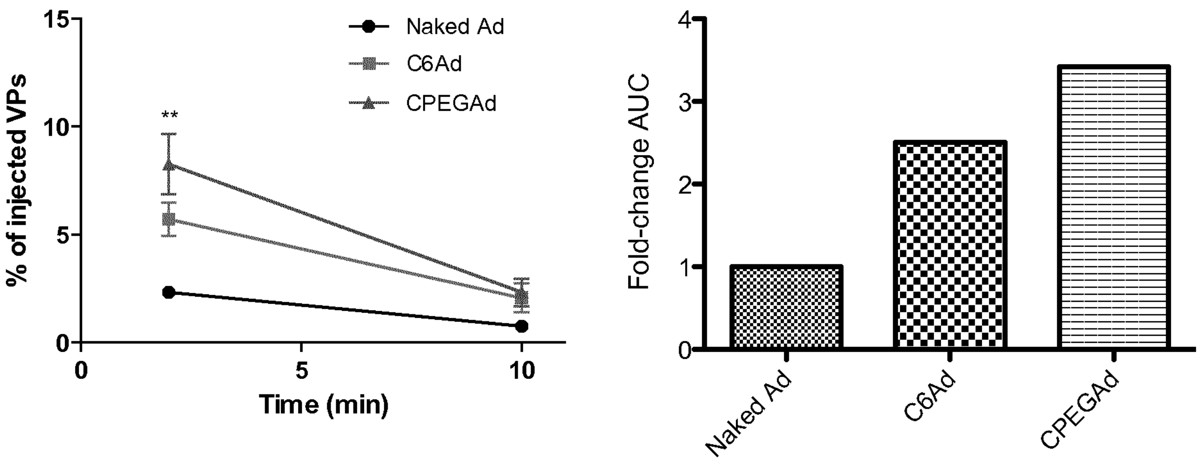

**FIGURE 10**

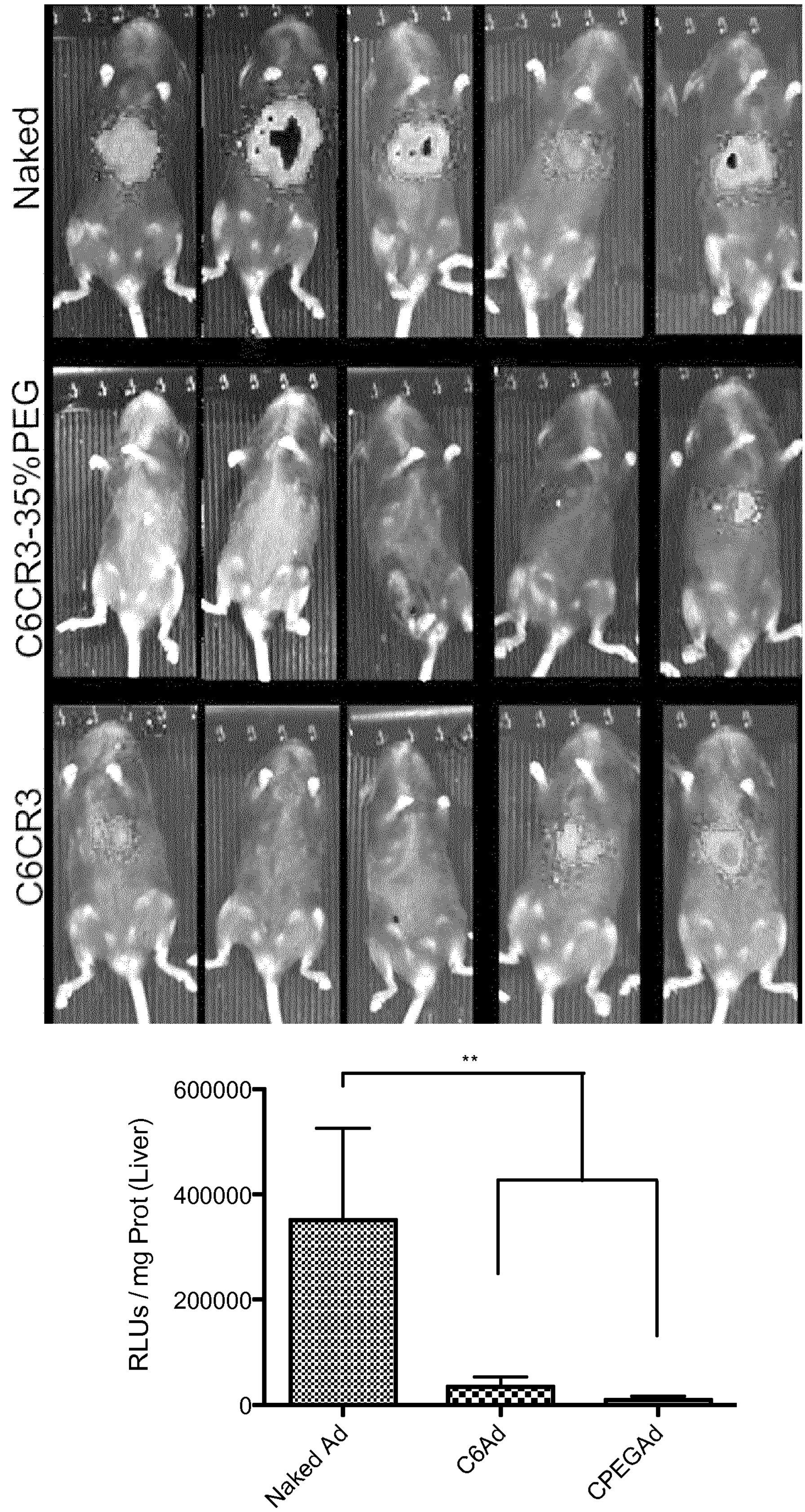
Naked
C6CR3-35%PEG
C6CR3
**
600000
400000
200000
0
RLUs / mg Prot (Liver)
Naked Ad
C6Ad
CPEGAd

**FIGURE 11**

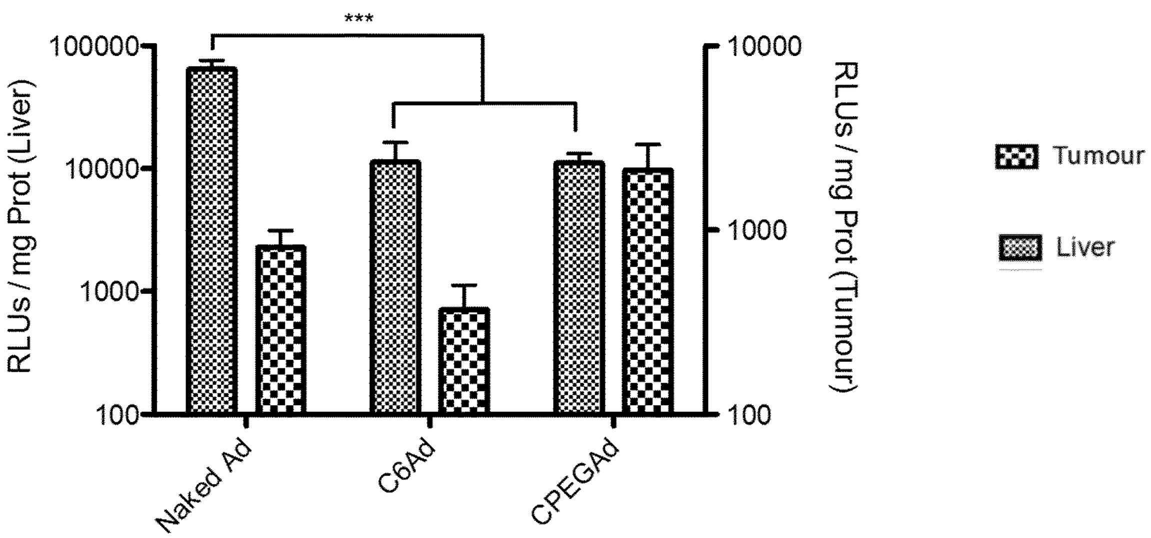
***
100000
10000
1000
100
RLUs / mg Prot (Liver)
10000
1000
100
RLUs / mg Prot (Tumour)
Tumour
Liver
Naked Ad
C6Ad
CPEGAd

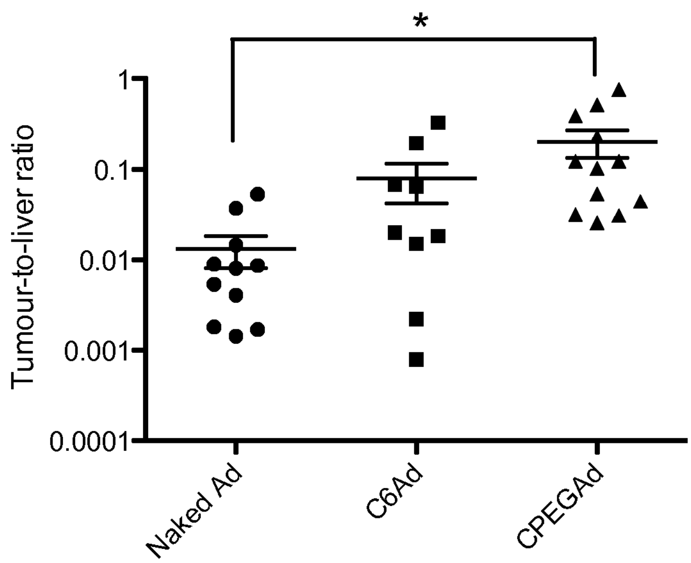
*
1
0.1
0.01
0.001
0.0001
Tumour-to-liver ratio
Naked Ad
C6Ad
CPEGAd

**FIGURE 12**

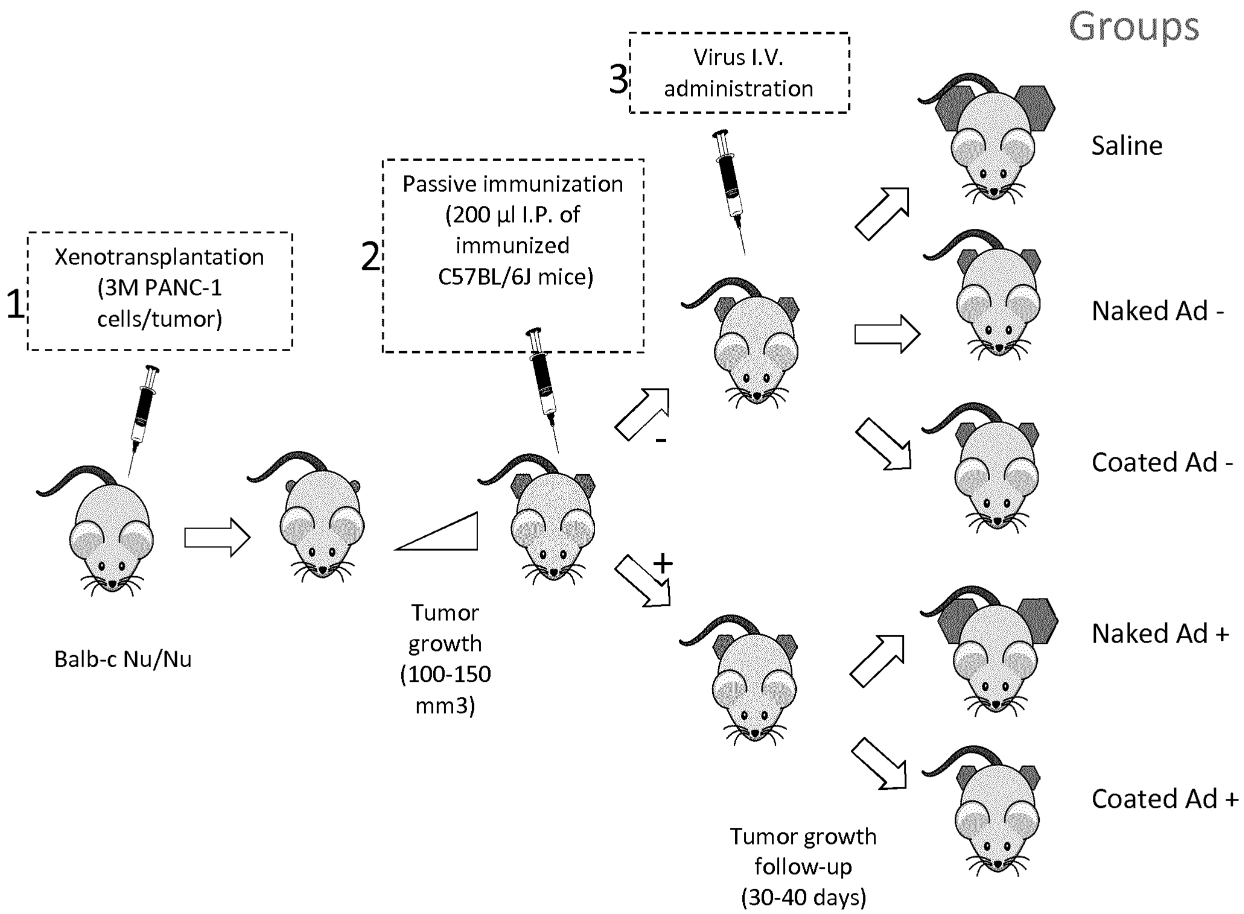

Groups
1
Xenotransplantation
(3M PANC-1
cells/tumor)
2
Passive immunization
(200 µl I.P. of
immunized
C57BL/6J mice)
3
Virus I.V.
administration
Balb-c Nu/Nu
Tumor
growth
(100-150
mm3)
-
+
Tumor growth
follow-up
(30-40 days)
Saline
Naked Ad -
Coated Ad -
Naked Ad +
Coated Ad +

**FIGURE 13**

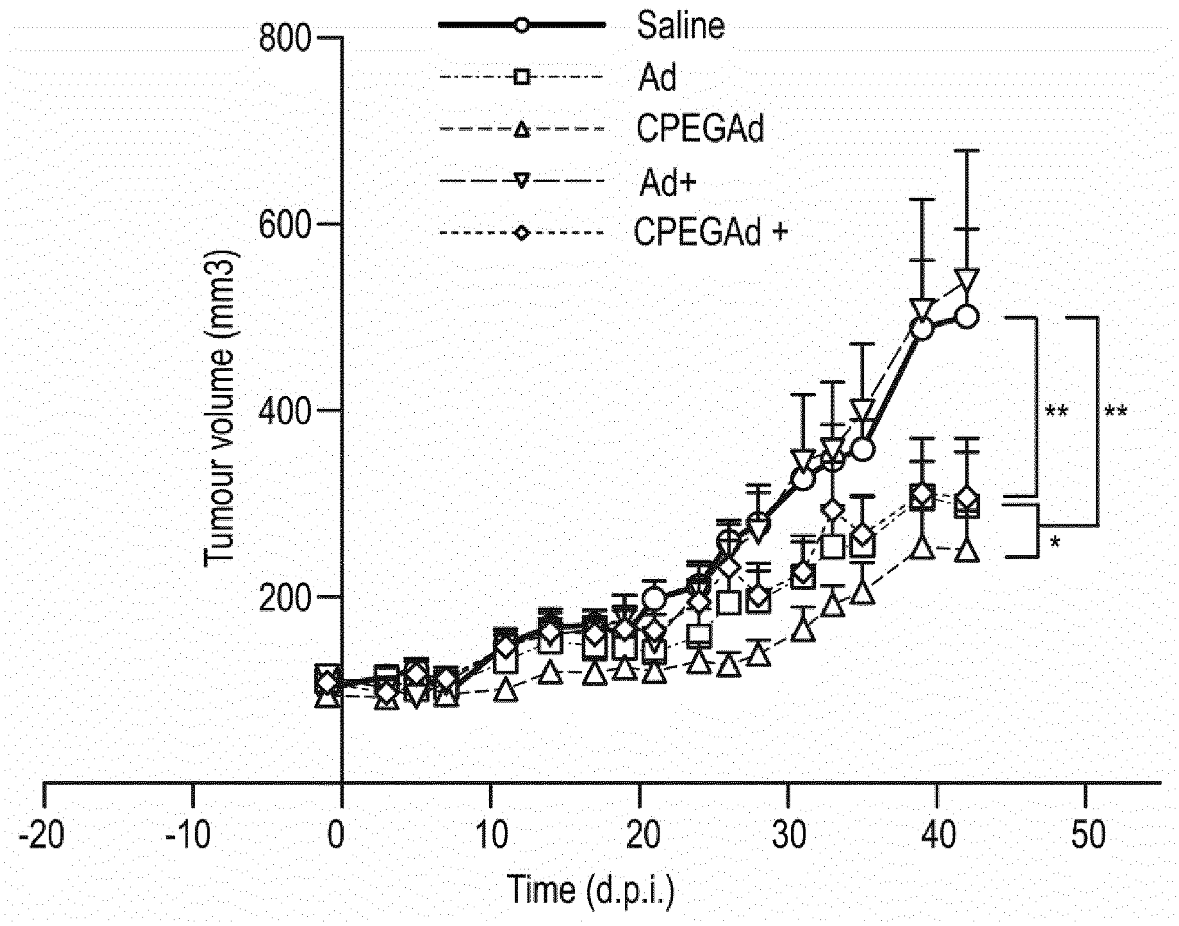
Saline
Ad
CPEGAd
Ad+
CPEGAd +
800
600
400
200
Tumour volume (mm3)
-20
-10
0
10
20
30
40
50
Time (d.p.i.)
**
**
*

**FIGURE 14**

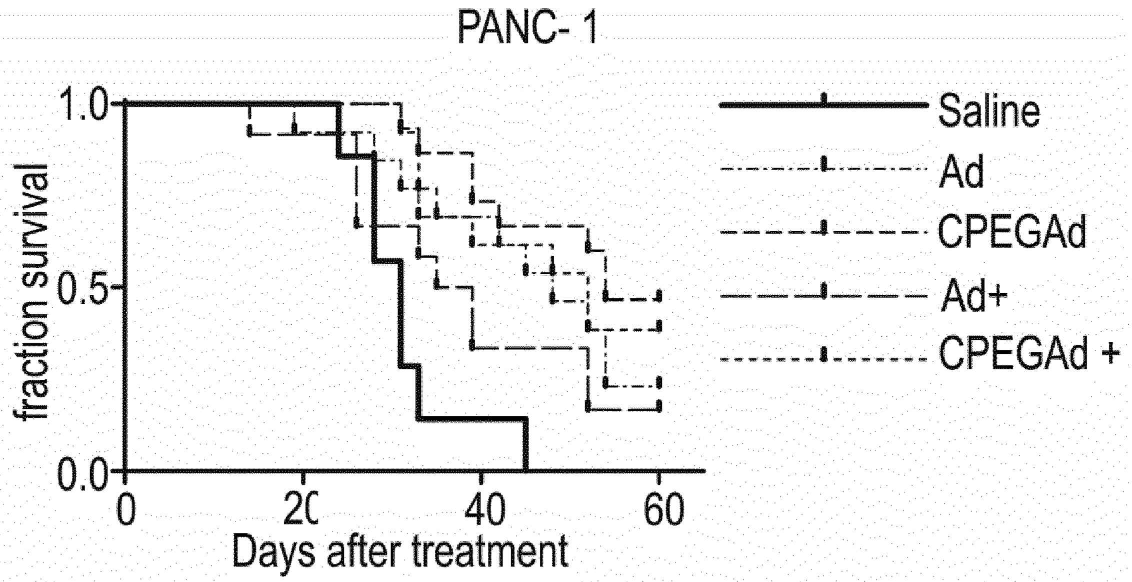


| | Saline | Ad | CPEGAd | Ad+ | CPEGAd |
|---|---|---|---|---|---|
| Median survival | 31 | 48 | 54 | 37 | 52 |

FIGURE 15

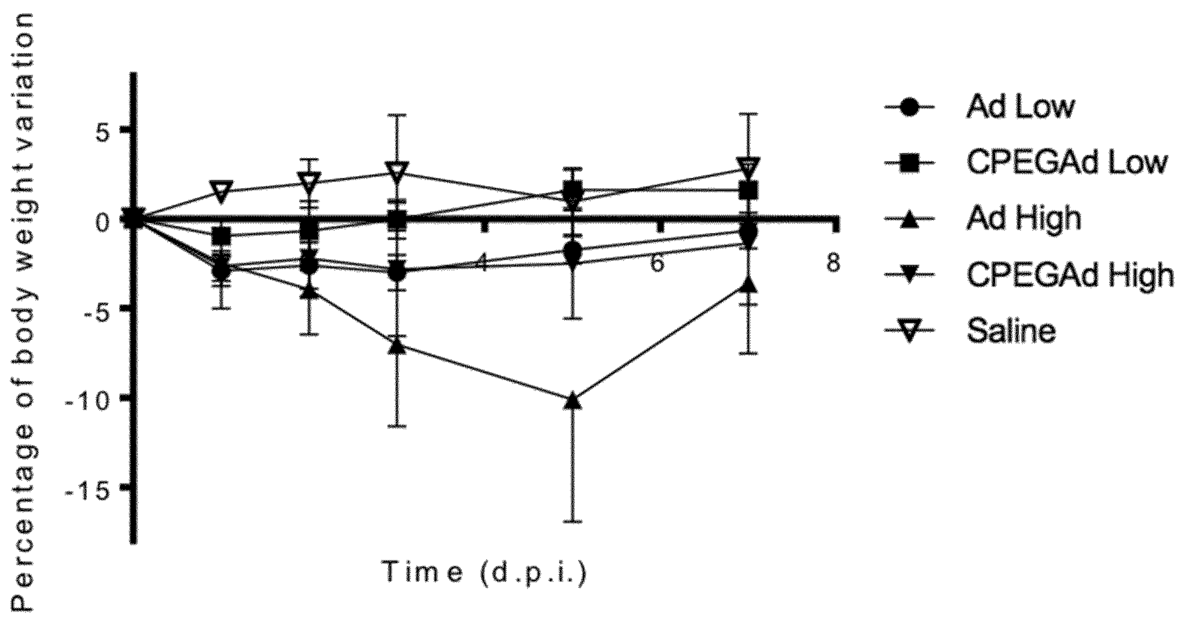


FIGURE 16

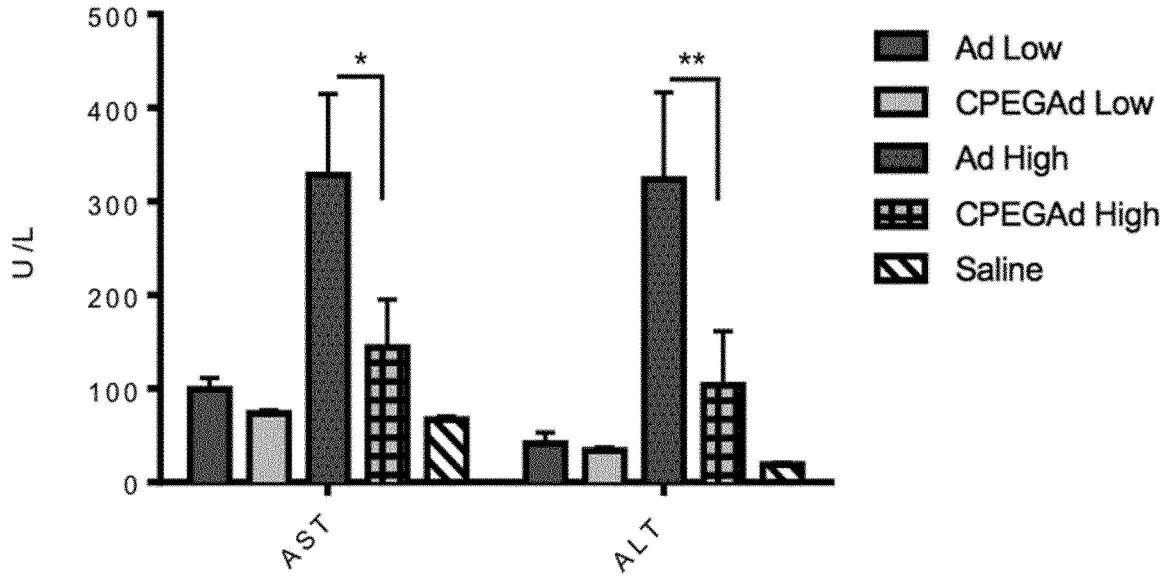

**FIGURE 17A**

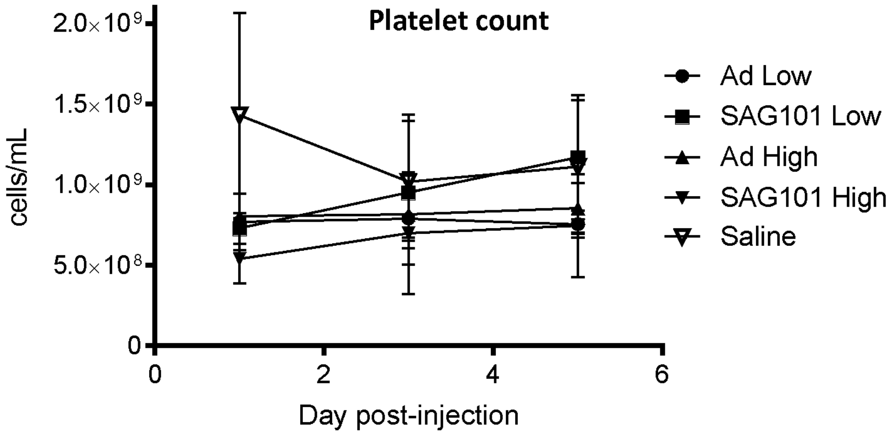


**FIGURE 17B**

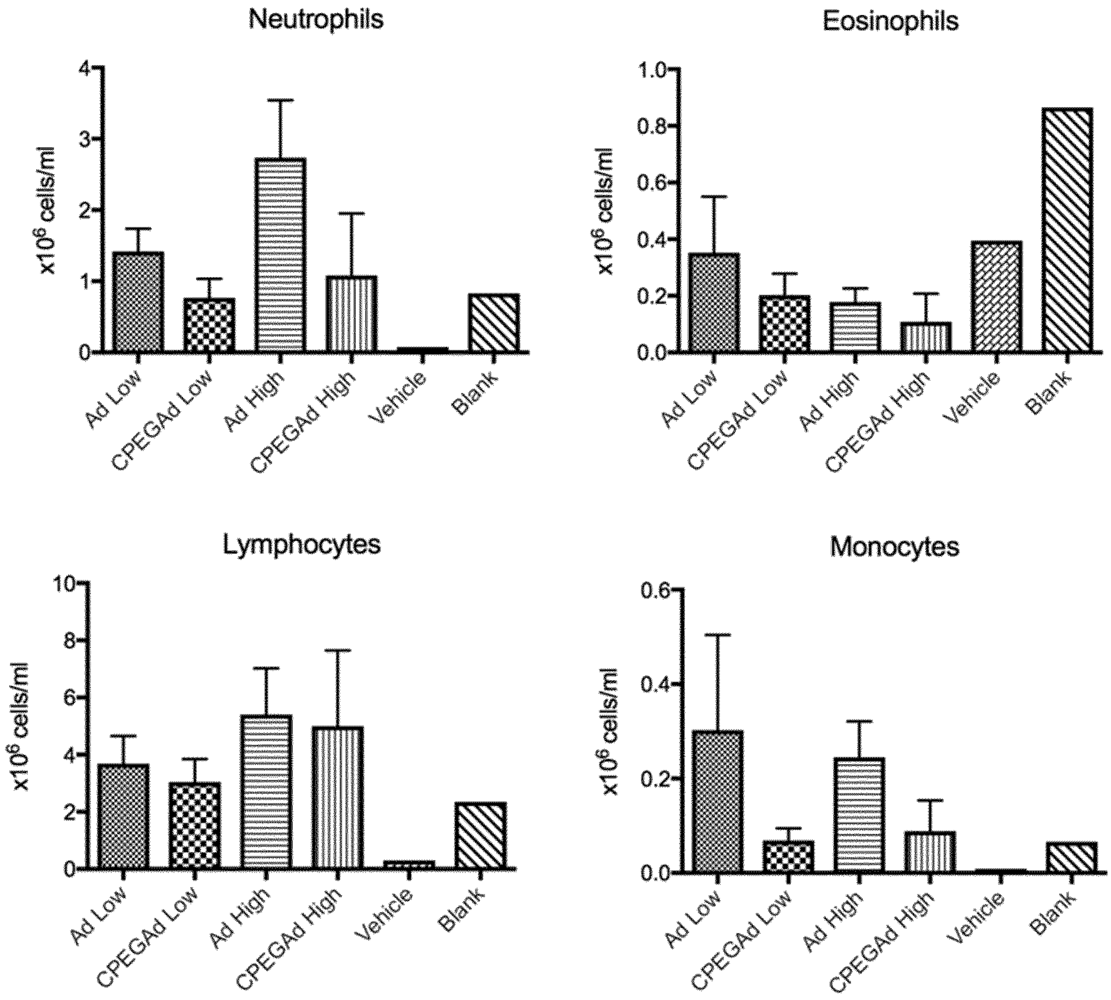

**FIGURE 18**

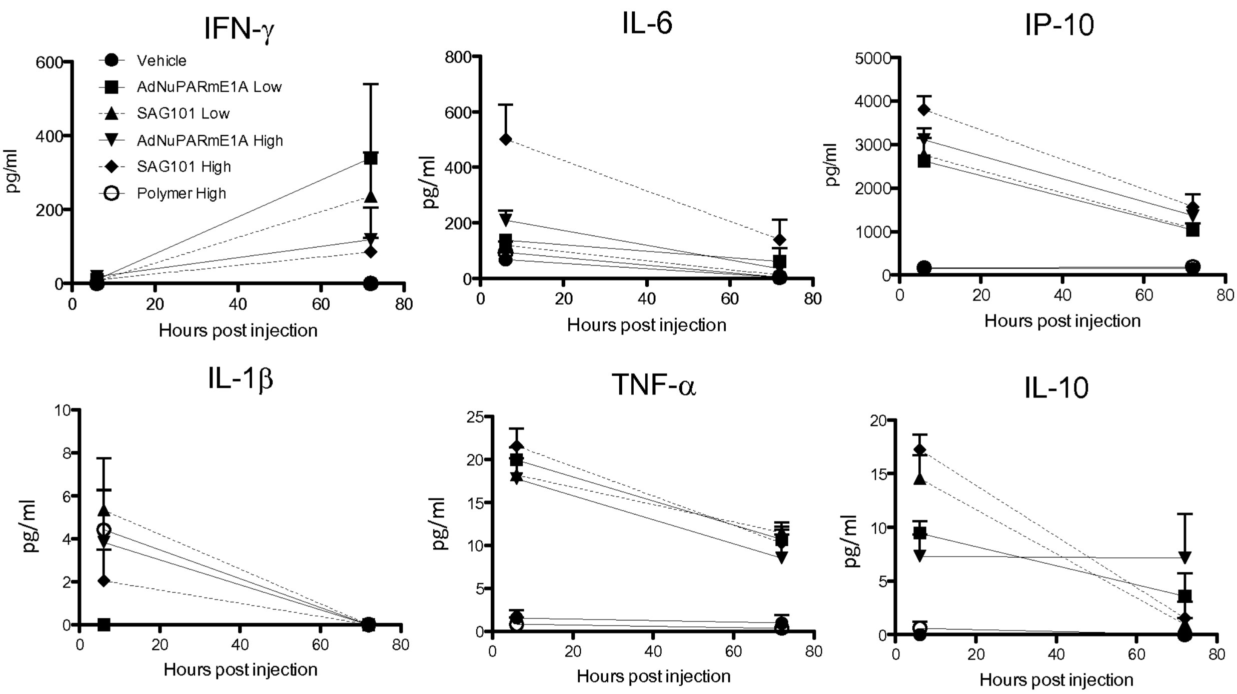
IFN-γ
Vehicle
AdNuPARmE1A Low
SAG101 Low
AdNuPARmE1A High
SAG101 High
Polymer High
pg/ml
Hours post injection
IL-6
pg/ml
Hours post injection
IP-10
pg/ml
Hours post injection
IL-1β
pg/ml
Hours post injection
TNF-α
pg/ml
Hours post injection
IL-10
pg/ml
Hours post injection

FIGURE 19A

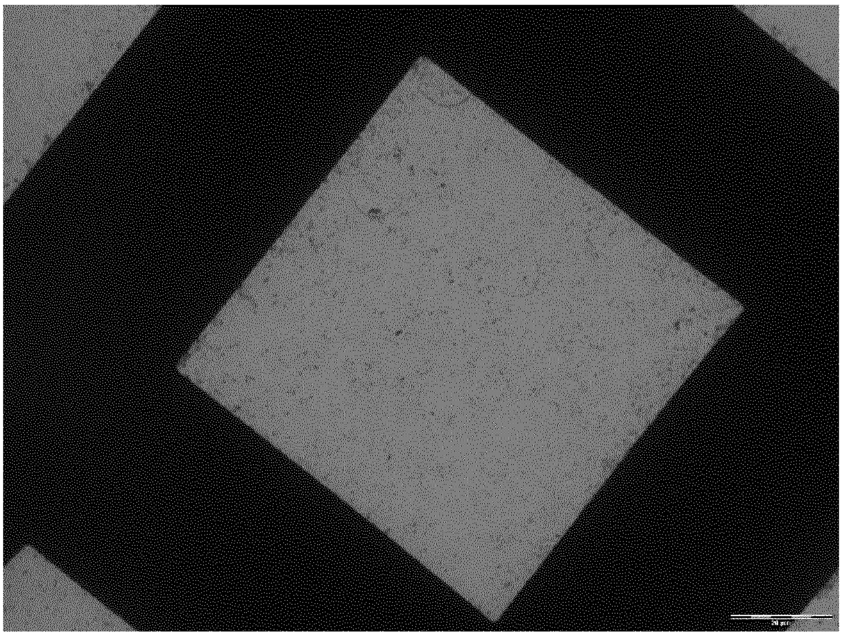

FIGURE 19B

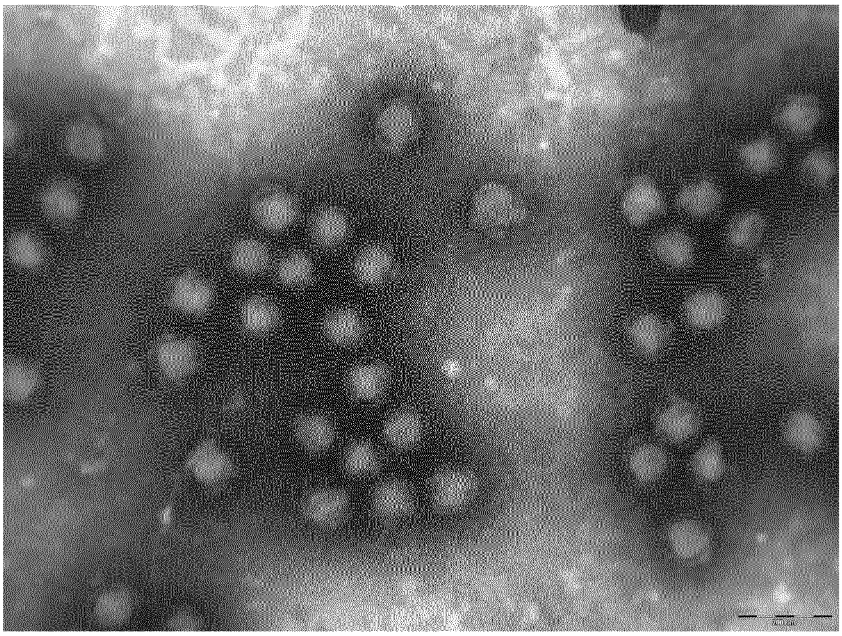

**FIGURE 20A**

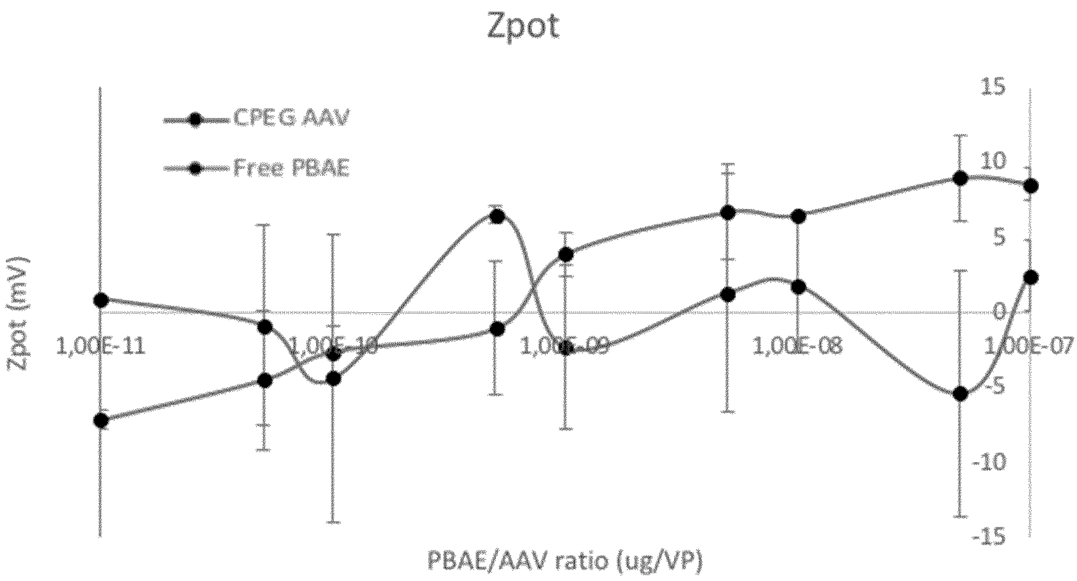


**FIGURE 20B**

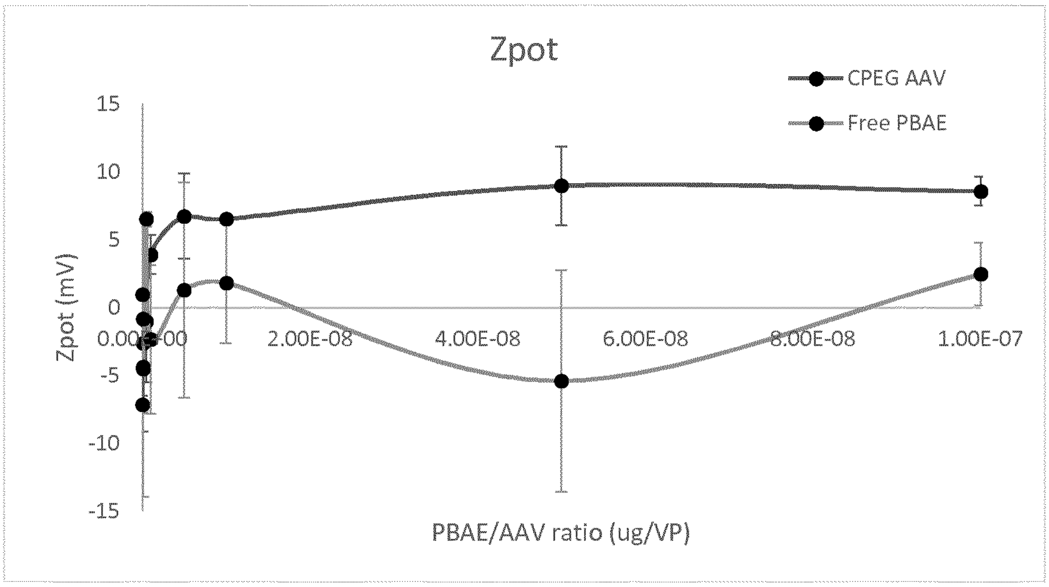

FIGURE 21

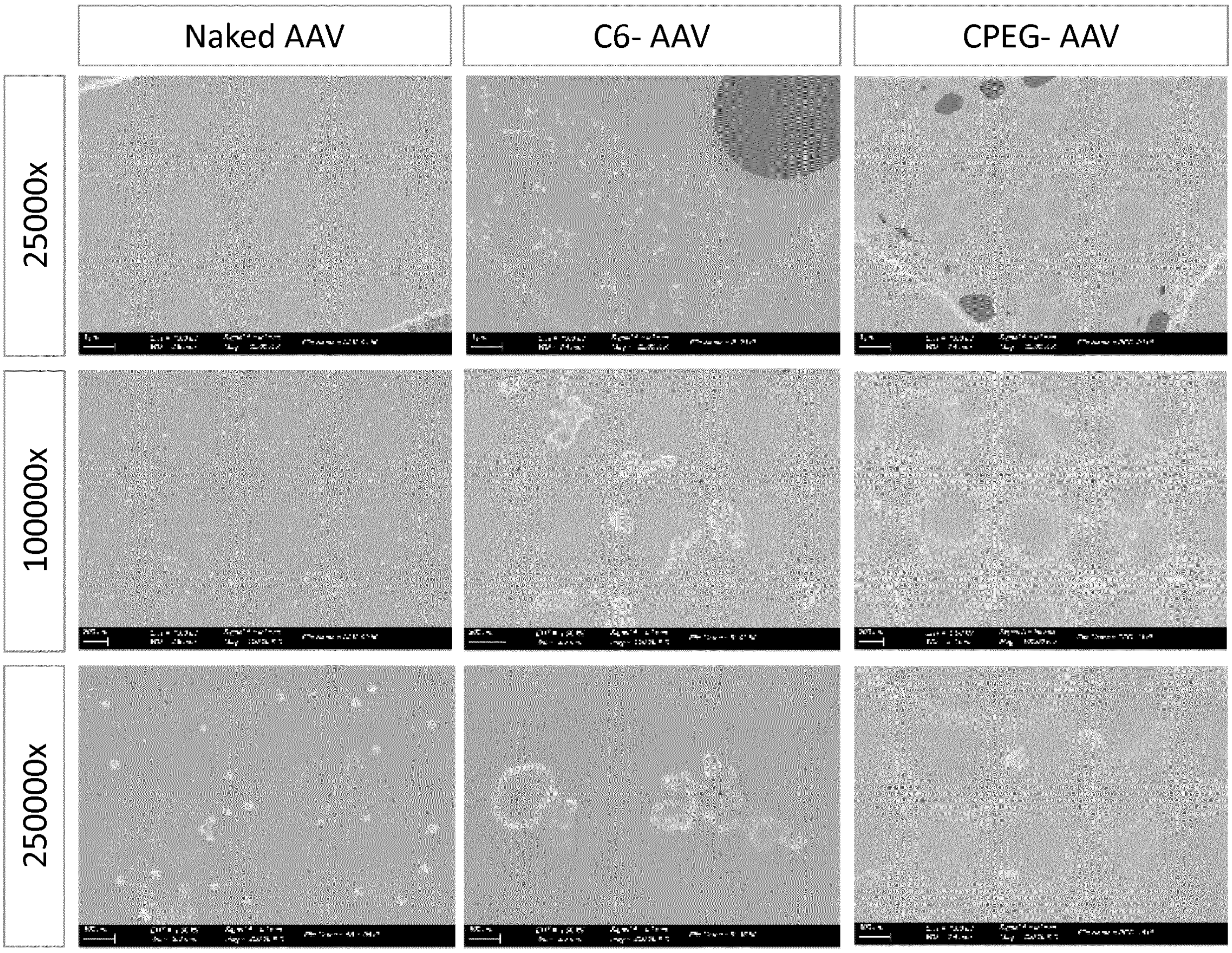

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014136100 A


### Non-patent literature cited in the description


- **ROJAS et al.** *Journal of Controlled Release,* 2016, vol. 237, 78-88
- **STAHL ; WERMUTH.** Handbook of Pharmaceutical Salts: Properties, Selection and Use. Wiley-VCH, 2002
- **MONTSERRAT, N. et al.** *J. Biol. Chem.,* 2011, vol. 286, 12417-12428
- **NAM Y ; SLIZ P ; PEAR WS ; ASTER JC ; BLACKLOW SC.** Cooperative assembly of higher-order Notch complexes functions as a switch to induce transcription. *Proc Natl Acad Sci USA.,* 2007, vol. 104, 2103-2108
- **MATO-BERCIANO A1 ; RAIMONDI G ; MALIANDI MV ; ALEMANY R ; MONTOLIU L ; FILLAT C.** A NOTCH-sensitive uPAR-regulated oncolytic adenovirus effectively suppresses pancreatic tumor growth and triggers synergistic anticancer effects with gemcitabine and nab-paclitaxel. *Oncotarget.,* 2017, vol. 8 (14), 22700-22715